# EUROPEAN PATENT APPLICATION

(11) **EP 4 353 262 A1**
(43) Date of publication of application: **17.04.2024**
(21) Application number: 22820621.5
(22) Date of filing: 10.06.2022
(51) Int. Cl.: A61K 45/00, A61P 11/00, C07D 209/12, A61K 31/496, A61K 31/341, A61K 45/06, A61P 29/00, A61P 7/10, C07D 403/06, A23L 33/10

(54) **NOVEL COMPOUND HAVING INHIBITORY ACTIVITY AGAINST PENDRIN, AND PHARMACEUTICAL USES THEREOF**

(30) Priority: 10.06.2021 KR 20210075738; 28.04.2022 KR 20220053009
(71) Applicant: UIF (University Industry Foundation), Yonsei University, Seoul 03722 (KR); Arbormed Co., Ltd., Seoul 06237 (KR)
(72) Inventor: CHOI, Jae Young, Seoul 04385 (KR); HAN, Gyoonhee, Hwaseong-si, Gyeonggi-do 18376 (KR); NAMKUNG, Wan, Incheon 21974 (KR); PARK, Moo Suk, Seoul 06289 (KR); PARK, Sungha, Seoul 06004 (KR); YOO, Tae-Hyun, Seoul 03733 (KR); PARK, Junyoung, Seoul 03726 (KR); IM, Weonbin, Yongin-si, Gyeonggi-do 16899 (KR); PARK, Eok, Seoul 07630 (KR); JUNG, Chunwon, Yongin-si, Gyeonggi-do 16836 (KR); MIN, Byong-Keol, Yongin-si, Gyeonggi-do 16838 (KR); KIM, Taewon, Seoul 04094 (KR); CHOI, Seoyoung, Yongin-si, Gyeonggi-do 16865 (KR)
(74) Representative: Bates, Philip Ian
(86) International application number: PCT/KR2022/008257
(87) International publication number: WO 2022/260494

(57) **Abstract**

The present invention relates to a novel compound having inhibitory activity against pendrin, and pharmaceutical uses thereof, and provides a compound represented by formula 1 below, an E- or Z-isomer thereof, an optical isomer thereof, a precursor thereof, a pharmaceutically acceptable salt thereof, a solvate thereof or a mixture of two or more isomers thereof, and a composition for preventing, relieving or treating respiratory diseases and a diuretic composition, each composition utilizing the inhibitory activity of the compound against pendrin.

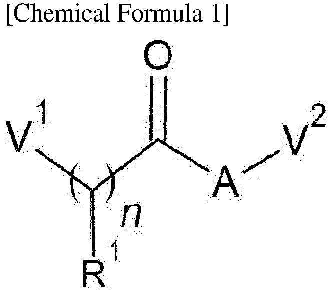

## Description

### [TECHNICAL FIELD]

The present disclosure relates to a novel compounds having pendrin inhibitory activity and a pharmaceutical use thereof, and provides a compound represented by Chemical Formula 1 below, an E- or Z-isomer thereof, an optical isomer thereof, a precursor thereof, a pharmaceutically acceptable salt thereof, a solvate thereof or a mixture of two isomers thereof, and a composition for preventing, ameliorating or treating respiratory diseases and a diuretic composition, each composition utilizing the inhibitory activity of the compound against pendrin.

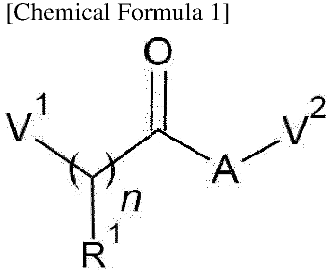

### [BACK GROUND ART]

Aberrant anion exchange is implicated in a variety of human diseases and disorders. For example, solute imbalances in plasma or urine may indicate renal, cardiac, or hepatic issues. In cystic fibrosis, deficient Cl⁻ and HCO₃⁻ transport alters the volume and composition of airway surface liquid, which may lead to a variety of complications such as impaired immune function and CD disease pathogenesis. Thus, cellular mechanisms involved in anion exchange are of interest as potential therapeutic targets.

Pendrin (hereinafter also named as "PDS") is encoded by the SLC26A4 (solute carrier family 26, member 4) gene which is a member of the SLC26 gene family, and is an anion exchanger expressed in a variety of tissues, including the epithelium of inflamed airways, the inner ear, the thyroid, the adrenal gland, and the kidneys, which exchanges Cl⁻ with anions such as HCO₃⁻, I⁻, OH⁻, SCN⁻ and HCO₂⁻. Pendrin is a cell membrane protein expressed in the luminal membrane of airway epithelial cells. However, pendrin expression is strongly upregulated in inflammatory airway diseases, such as chronic obstructive pulmonary disease (COPD), allergic rhinitis, asthma, Bordetella pertussis infection, acute lung injury (ALI), acute respiratory distress syndrome (ARDS), and the common cold caused by rhinoviruses, and upregulation of pendrin is observed when primary airway epithelial cells are cultured with IL-4, IL-13, and IL-17A. Interestingly, pendrin knockout (KO) improves airway inflammation in all mouse models of COPD, allergic rhinitis, asthma, Bordetella pertussis infection, and rhinovirus infection. The pathophysiological role of pendrin in airway inflammation has not been clearly found out. However, new evidence indicates that pendrin is involved in preserving airway surface liquid (ASL) volume and regulating mucus production in inflammatory airway diseases.

In primary mouse tracheal epithelial cell culture with IL-13, the increase in ASL volume was significantly higher in pendrin KO mice compared to control WT mice. In primary human nasal epithelial (HNE) cell culture from deaf patients carrying a pendrin mutant (DFNB4), the increase in IL-13-induced ASL volume was significantly higher than that in a normal control. Furthermore, inhibition of pendrin by a pendrin inhibitor significantly increased IL-13-induced ASL volume in primary cultures of human tracheal epithelial cells. These findings suggest that downregulation of pendrin may have beneficial effects on the regulation of ASL volume homeostasis in inflammatory airway diseases.

Over-production of mucus is a common feature of inflammatory airway diseases such as asthma and COPD. Overexpression of pendrin significantly increased MUC5AC gene expression in human lung cancer NCI-H292 cell line and mouse lung tissue. IL-13 treatment significantly increased MUC5AC gene expression in HNE cells from a normal subject, but IL-13-induced upregulation of MUC5AC was completely ablated in HNE cells from deaf patients carrying pendrin mutant. These findings suggest that downregulation of pendrin may be beneficial in the treatment of asthma and COPD.

As the prior art, U.S. Patent Application Publication No. 2019/0054071 discloses a small molecule inhibitor of pendrin ion exchange of a compound represented by Chemical Formula I below and its pharmaceutical use as a therapeutic agent for respiratory diseases or as a diuretic.

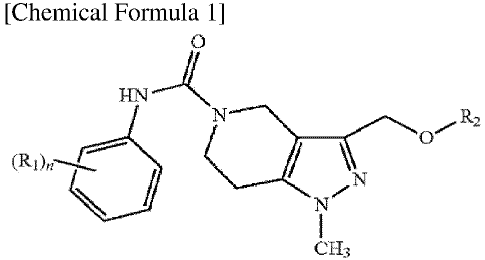

### [DETAILED DESCRIPTION OF THE INVENTION]

### [Technical Problem]

The present disclosure is based on the discovery that a certain novel compound acts as a pendrin inhibitor and can be used as a therapeutic agent or diuretic for related diseases through pendrin inhibitory activity.

Therefore, an object of the present disclosure is to provide a novel compound represented by Chemical Formula 1 below, a composition for preventing, ameliorating or treating respiratory diseases through its pendrin inhibitory activity, and a diuretic composition.

Another object of the present disclosure is to provide a novel compound represented by Chemical Formula 1 below, a method for preventing, ameliorating or treating respiratory diseases, and a diuretic method through its pendrin inhibitory activity.

### [Technical Solution]

In order to achieve the above-mentioned object, according to the present disclosure, there is provided a compound represented by Chemical Formula 1 below, an E- or Z-isomer thereof, an optical isomer thereof, a precursor thereof, a pharmaceutically acceptable salt thereof, a solvate thereof or a mixture of two isomers thereof: 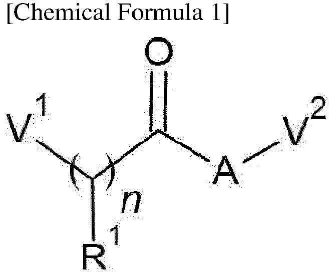
wherein, in Chemical Formula 1,
V¹ may be a C₅∼C₁₀ heteroaryl containing 1 to 3 nitrogen atoms, which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂∼C₁₀ alkenyl, C₂∼C₁₀ alkynyl, C(O)OR², C(O)R², OR², and OCR²F₂,
A may be a C₅∼C₇ heteroaryl containing 1 to 3 nitrogen atoms or a C₅∼C₇ heterocycloalkyl containing 1 to 3 heteroatoms, which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂∼C₁₀ alkenyl, C₂∼C₁₀ alkynyl, C(O)OR², C(O)R², OR², OCR²F₂ and OCOR²,
V² may be an aryl, a C₁~C₁₀ alkyl, a C₅∼C₇ cycloalkyl, or a C(O)OR², which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂∼C₁₀ alkenyl, C₂∼C₁₀ alkynyl, C(O)OR², C(O)R², OR², OCR²F₂ and OCOR²,
R¹ may be one or more independently selected from the group consisting of hydrogen, halogen, C₁∼C₁₀ alkyl, C₂~C₆ alkenyl, and C₂~C₆ alkynyl,
R² may be independently selected from the group consisting of hydrogen, trifluoromethyl, trifluoromethoxy, aryl, aryl (C₁~C₁₀ alkyl), C₁∼C₁₀ alkylaryl, heteroaryl, heteroaryl (C₁~C₁₀ alkyl), C₁∼C₁₀ alkylheteroaryl, C₁∼C₁₀ alkyl, C₂~C₆ alkenyl, C₂~C₆ alkynyl, C₃~C₆ cycloalkyl and heterocycloalkyl, and
n may be an integer from 1 to 5.

According to a preferred embodiment of the present disclosure, in Chemical Formula 1, V¹ may be indolyl, isoindolyl, 3H-indolyl, 1H-indolyl, quinolyl, isoquinolyl, 5,6,7,8-tetrahydroquinolyl, 5,6,7,8-tetrahydroisoquinolyl, quinolizinyl, quinazolinyl, phthalazinyl, cinnolinyl or naphthyridinyl, which is substituted with one or more groups independently selected from the group consisting of hydrogen, halogen, trifluoromethyl, C₃∼C₇ cycloalkyl, C₁∼C₁₀ alkyl, C₂~C₁₀ alkenyl, C₂~C₁₀ alkynyl, C(O)OR², C(O)R², OR², OCR²F₂ and OCOR²,
A may be pyrrolidinyl, piperidinyl, piperazinyl or decahydroisoquinolinyl, which is substituted with one or more groups independently selected from the group consisting of hydrogen, halogen, trifluoromethyl, C₃∼C₇ cycloalkyl, C₁∼C₁₀ alkyl, C_{2∼}C₁₀ alkenyl and C₂∼C₁₀ alkynyl,
V² may be a C₅∼C₇ aryl, a C₁∼C₁₀ alkyl, a C₅∼C₇ cycloalkyl or C(O)OR², which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂∼C₁₀ alkenyl, C₂∼C₁₀ alkynyl, C₃~C₆ cycloalkyl, C(O)OR², C(O)R², OR² and OCR²F₂,
R¹ may be one or more independently selected from the group consisting of hydrogen, halogen, C₁∼C₁₀ alkyl, C₂~C₆ alkenyl, and C₂~C₆ alkynyl,
R² may be independently selected from the group consisting of hydrogen, trifluoromethyl, aryl, aryl (C₁~C₁₀ alkyl), C₁∼C₁₀ alkylaryl, C₁∼C₁₀ alkyl, C₂~C₆ alkenyl, C₂~C₆ alkynyl, and C₃~C₆ cycloalkyl, and
n may be an integer from 1 to 5.

According to another preferred embodiment of the present disclosure, the compound represented by Chemical Formula 1, an E- or Z-isomer thereof, an optical isomer thereof, a precursor thereof, a pharmaceutically acceptable salt thereof, a solvate thereof or a mixture of two isomers thereof includes a compound represented by Chemical Formula 1a below, an E- or Z-isomer thereof, an optical isomer thereof, a precursor thereof, a pharmaceutically acceptable salt thereof, a solvate thereof or a mixture of two isomers thereof: 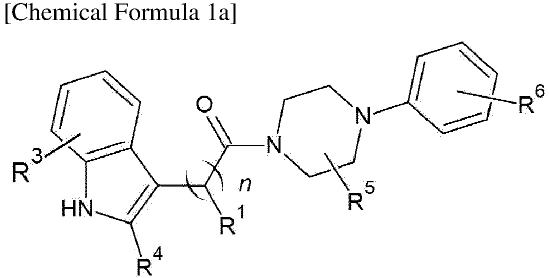
wherein, in Chemical Formula 1a,
R¹ may be one or more independently selected from the group consisting of hydrogen, halogen, C₁∼C₁₀ alkyl, C₂~C₆ alkenyl, and C₂~C₆ alkynyl,
R³ may be one or more groups independently selected from the group consisting of hydrogen, halogen, trifluoromethyl, C₃∼C₇ cycloalkyl, C₁∼C₁₀ alkyl, C₂∼C₁₀ alkenyl, C₂~C₁₀ alkynyl, C(O)OR², C(O)R², OR² and OCR²F₂,
R⁴ may be a group independently selected from the group consisting of C(O)OR², C(O)R², OR², OCR²F₂ and OCOR²,
R⁵ may be one or more groups independently selected from the group consisting of hydrogen, C₃∼C₇ cycloalkyl, C₁∼C₁₀ alkyl, C₂~C₁₀ alkenyl and C₂~C₁₀ alkynyl,
R⁶ may be one or more groups independently selected from the group consisting of hydrogen, halogen, trifluoromethyl, C₃∼C₇ cycloalkyl, C₁~C₁₀ alkyl, C₂∼C₁₀ alkenyl, C₂~C₁₀ alkynyl, C(O)OR², C(O)R², OR² and OCR²F₂,
R² may be independently selected from the group consisting of hydrogen, trifluoromethyl, aryl, aryl (C₁~C₁₀ alkyl), C₁∼C₁₀ alkylaryl, C₁~C₁₀ alkyl, C₂~C₆ alkenyl, C₂~C₆ alkynyl, and C₃~C₆ cycloalkyl, and
n may be an integer from 1 to 5.

According to yet another preferred embodiment of the present disclosure, the compound represented by Chemical Formula 1, an E- or Z-isomer thereof, an optical isomer thereof, a precursor thereof, a pharmaceutically acceptable salt thereof, a solvate thereof or a mixture of two isomers thereof includes a compound represented by Chemical Formula 1b below, an E- or Z-isomer thereof, an optical isomer thereof, a precursor thereof, a pharmaceutically acceptable salt thereof, a solvate thereof or a mixture of two isomers thereof: 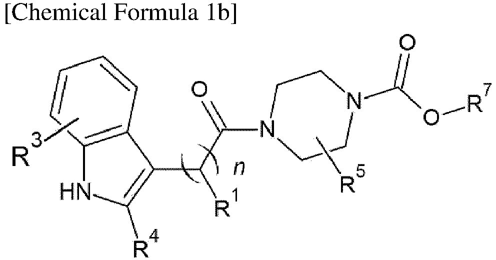
wherein, in Chemical Formula 1b,
R¹ may be one or more independently selected from the group consisting of hydrogen, halogen, C₁∼C₁₀ alkyl, C₂~C₆ alkenyl, and C₂~C₆ alkynyl,
R³ may be one or more groups independently selected from the group consisting of hydrogen, halogen, trifluoromethyl, C₃∼C₇ cycloalkyl, C₁∼C₁₀ alkyl, C₂∼C₁₀ alkenyl, C₂~C₁₀ alkynyl, C(O)OR², C(O)R², OR² and OCR²F₂,
R⁴ may be a group independently selected from the group consisting of C(O)OR², C(O)R², OR², OCR²F₂ and OCOR²,
R⁵ may be one or more groups independently selected from the group consisting of hydrogen, C₃∼C₇ cycloalkyl, C₁∼C₁₀ alkyl, C₂~C₁₀ alkenyl and C₂~C₁₀ alkynyl,
R⁷ may be one or more groups independently selected from the group consisting of hydrogen, halogen, trifluoromethyl, C₃∼C₇ cycloalkyl, C₁~C₁₀ alkyl, C₂~C₁₀ alkenyl, and C₂~C₁₀ alkynyl,
R² may be independently selected from the group consisting of hydrogen, trifluoromethyl, aryl, aryl (C₁~C₁₀ alkyl), C₁∼C₁₀ alkylaryl, C₁~C₁₀ alkyl, C₂~C₆ alkenyl, C₂~C₆ alkynyl, and C₃~C₆ cycloalkyl, and
n may be an integer from 1 to 5.

According to another preferred embodiment of the present disclosure, the compound may be:
(1) 5-chloro-3-(2-oxo-2-(4-(4-(trifluoromethyl)phenyl)piperazin-1-yl)ethyl)-1H-indole-2-carboxylic acid;
(2) 5-chloro-3-(2-oxo-2-(4-(2-(trifluoromethyl)phenyl)piperazin-1-yl)ethyl)-1H-indole-2-carboxylic acid;
(3) 5-chloro-3-(2-(4-(2,6-dimethylphenyl)piperazin-1-yl)-2-oxoethyl)-1H-indole-2-carboxylic acid;
(4) 5-chloro-3-(2-(4-(2,4-dimethylphenyl)piperazin-1-yl)-2-oxoethyl)-1H-indole-2-carboxylic acid;
(5) 5-chloro-3-(2-(4-(3-methoxyphenyl)piperazin-1-yl)-2-oxoethyl)-1H-indole-2-carboxylic acid;
(6) 5-chloro-3-(2-(4-(2-chlorophenyl)piperazin-1-yl)-2-oxoethyl)-1H-indole-2-carboxylic acid;
(7) 5-chloro-3-(2-(4-(4-chlorophenyl)piperazin-1-yl)-2-oxoethyl)-1H-indole-2-carboxylic acid;
(8) 3-(2-(4-(2-bromophenyl)piperazin-1-yl)-2-oxoethyl)-5-chloro-1H-indole-2-carboxylic acid;
(9) 3-(2-(4-(3-bromophenyl)piperazin-1-yl)-2-oxoethyl)-5-chloro-1H-indole-2-carboxylic acid;
(10) 3-(2-(4-(4-bromophenyl)piperazin-1-yl)-2-oxoethyl)-5-chloro-1H-indole-2-carboxylic acid;
(11) 5-chloro-3-(2-oxo-2-(4-(*o*-tolyl)piperazin-1-yl)ethyl)-1H-indole-2-carboxylic acid;
(12) 5-chloro-3-(2-oxo-2-(4-(*m*-tolyl)piperazin-1-yl)ethyl)-1H-indole-2-carboxylic acid;
(13) 5-chloro-3-(2-oxo-2-(4-(*p*-tolyl)piperazin-1-yl)ethyl)-1H-indole-2-carboxylic acid;
(14) 3-(2-(4-(*tert*-butoxycarbonyl)piperazin-1-yl)-2-oxoethyl)-5-chloro-1H-indole-2-carboxylic acid;
(15) 5-chloro-3-(2-(4-(2-chloro-6-methylphenyl)piperazin-1-yl)-2-oxoethyl)-1H-indole-2-carboxylic acid;
(16) 5-chloro-3-(2-(4-(2-ethoxyphenyl)piperazin-1-yl)-2-oxoethyl)-1H-indole-2-carboxylic acid;
(17) 5-chloro-3-(2-(3-methyl-4-phenylpiperazin-1-yl)-2-oxoethyl)-1H-indole-2-carboxylic acid;
(18) 5-chloro-3-(2-(4-(4-chlorophenyl)-2,2-dimethylpiperazin-1-yl)-2-oxoethyl)-1H-indole-2-carboxylic acid;
(19) 5-chloro-3-(2-(4-(2-chloro-4-fluorophenyl)piperazin-1-yl)-2-oxoethyl)-1H-indole-2-carboxylic acid;
(20) 5-chloro-3-(2-(4-(2-chloro-5-methoxyphenyl)piperazin-1-yl)-2-oxoethyl)-1H-indole-2-carboxylic acid;
(21) 5-chloro-3-(2-(4-(2,4-dichlorophenyl)piperazin-1-yl)-2-oxoethyl)-1H-indole-2-carboxylic acid;
(22) (R)-5-chloro-3-(2-(2-methyl-4-phenylpiperazin-1-yl)-2-oxoethyl)-1H-indole-2-carboxylic acid;
(23) 5-chloro-3-(2-(4-(2,4-difluorophenyl)piperazin-1-yl)-2-oxoethyl)-1H-indole-2-carboxylic acid;
(24) 5-chloro-3-(2-oxo-2-(4-(3,4,5-trichlorophenyl)piperazin-1-yl)ethyl)-1H-indole-2-carboxylic acid;
(25) 5-chloro-3-(2-(4-(2-fluorophenyl)piperazin-1-yl)-2-oxoethyl)-1H-indole-2-carboxylic acid;
(26) 5-chloro-3-(2-(4-(3-chloro-2-methylphenyl)piperazin-1-yl)-2-oxoethyl)-1H-indole-2-carboxylic acid;
(27) 3-(2-(4-(4-carboxyphenyl)piperazin-1-yl)-2-oxoethyl)-5-chloro-1H-indole-2-carboxylic acid;
(28) 3-(2-(4-(4-acetylphenyl)piperazin-1-yl)-2-oxoethyl)-5-chloro-1H-indole-2-carboxylic acid;
(29) 5-chloro-3-(2-(4-(4-ethoxyphenyl)piperazin-1-yl)-2-oxoethyl)-1H-indole-2-carboxylic acid;
(30) 3-(2-(4-(4-(*tert*-butyl)phenyl)piperazin-1-yl)-2-oxoethyl)-5-chloro-1H-indole-2-carboxylic acid;
(31) 3-(2-(4-(4-(*tert*-butyl)phenyl)piperazin-1-yl)-2-oxoethyl)-5-methyl-1H-indole-2-carboxylic acid;
(32) 3-(2-(4-(4-(*tert*-butyl)phenyl)piperazin-1-yl-2-oxoethyl)-5,7-dimethyl-1H-indole-2-carboxylic acid;
(33) 3-(2-(4-(2,6-dimethylphenyl)piperazin-1-yl)-2-oxoethyl)-5-methyl-1H-indole-2-carboxylic acid;
(34) 3-(2-(4-(2,6-dimethylphenyl)piperazin-1-yl)-2-oxoethyl)-5,7-dimethyl-1H-indole-2-carboxylic acid;
(35) 3-(2-(4-(2-chloro-6-methylphenyl)piperazin-1-yl)-2-oxoethyl)-5-methyl-1H-indole-2-carboxylic acid;
(36) 3-(2-(4-(2-chloro-6-methylphenyl)piperazin-1-yl)-2-oxoethyl)-5,7-dimethyl-1H-indole-2-carboxylic acid;
(37) 3-(2-(4-(2-chloro-6-methylphenyl)piperazin-1-yl)-2-oxoethyl)-5-fluoro-1H-indole-2-carboxylic acid;
(38) 3-(2-(4-(2,6-dimethylphenyl)piperazin-1-yl)-2-oxoethyl)-5-fluoro-1H-indole-2-carboxylic acid;
(39) 3-(2-(4-(4-(*tert*-butyl)phenyl)piperazin-1-yl)-2-oxoethyl)-5-fluoro-1H-indole-2-carboxylic acid;
(40) 3-(2-(4-([1,1'-biphenyl]-4-yl)piperazin-1-yl)-2-oxoethyl)-5-chloro-1H-indole-2- carboxylic acid; or
(41) 5-chloro-3-(2-(4-(2,5-dimethylphenyl)piperazin-1-yl-)-2-oxoethyl)-1H-indole-2-carboxylic acid.

In another aspect of the present disclosure, there is provided a pharmaceutical composition for preventing or treating respiratory diseases which comprises, as an active ingredient, the above-mentioned compound, an E- or Z-isomer thereof, an optical isomer thereof, a precursor thereof, a pharmaceutically acceptable salt thereof, a solvate thereof or a mixture of two isomers thereof, and/or a health functional food for preventing or ameliorating respiratory diseases which comprises the same active ingredient.

According to a preferred embodiment of the present disclosure, the respiratory diseases may be inflammatory airway diseases.

According to another preferred embodiment of the present disclosure, the inflammatory airway diseases may be one or more selected from the group consisting of asthma, acute or chronic bronchitis, allergic rhinitis, acute respiratory tract infection, acute upper respiratory tract infection, cystic fibrosis, acute respiratory distress syndrome (ARDS), acute lung injury (ALI) and chronic obstructive pulmonary disease (COPD).

According to yet another preferred embodiment of the present disclosure, the active ingredient may act as a pendrin inhibitor.

According to another preferred embodiment of the present disclosure, the active ingredient may preserve or increase the volume of airway surface liquid (ASL).

According to yet another preferred embodiment of the present disclosure, the pharmaceutical composition may further comprise other pharmaceutical ingredients.

In a further aspect of the present disclosure, there is provided a diuretic pharmaceutical composition which comprises, as an active ingredient, the above-mentioned compound, an E- or Z-isomer thereof, an optical isomer thereof, a precursor thereof, a pharmaceutically acceptable salt thereof, a solvate thereof or a mixture of two isomers thereof, and/or a diuretic health functional food composition which comprises the same active ingredient.

According to a preferred embodiment of the present disclosure, the composition may be co-administered with other diuretics.

According to another preferred embodiment of the present disclosure, the other diuretic is furosemide, and the pharmaceutical composition and furosemide may be administered in a weight ratio of 1:10.

In a further aspect of the present disclosure, there is provided a method for preventing, ameliorating or treating respiratory diseases and a diuretic method, which comprises administering the above-mentioned compound, an E- or Z-isomer thereof, an optical isomer thereof, a precursor thereof, a pharmaceutically acceptable salt thereof, a solvate thereof or a mixture of two isomers thereof.

### [Advantageous Effects]

A compound represented by Chemical Formula 1, an E- or Z-isomer thereof, an optical isomer thereof, a precursor thereof, a pharmaceutically acceptable salt thereof, a solvate thereof or a mixture of two or more isomers thereof according to the present disclosure acts as a pendrin inhibitor, and is useful for the prevention, amelioration or treatment of respiratory diseases, such as inflammatory airway diseases, especially asthma or acute lung injury, and can also be used as a diuretic.

### [BRIEF DESCRIPTION OF THE DRAWINGS]

Figure 1 shows YFP fluorescence changes which illustrate the inhibitory effects of YPI-H05 and YPI-A4948 on the Cl⁻/I⁻ exchange activity of pendrin in CHO-K1-YFP cells stably expressing human pendrin.
Figure 2 shows YFP fluorescence changes which illustrate the inhibitory effects of YPI-H05 and YPI-A4948 on the Cl⁻/I⁻ exchange activity of SLC26A3 in LN-215-YFP cells stably expressing human SLC26A3.

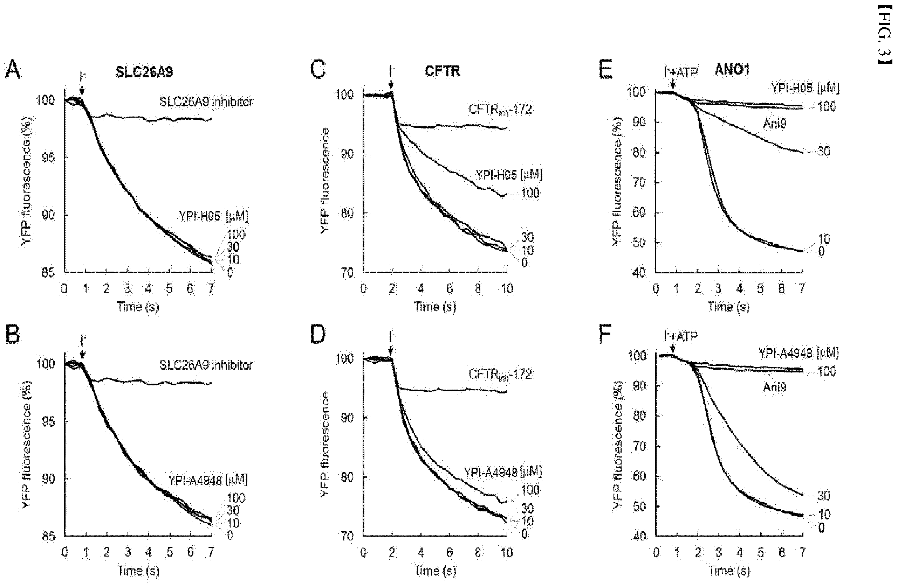

Figure 3 shows the effect of YPI-H05 and YPI-A4948 on the activities of SLC26A9, CFTR, and ANO1, which are anion transporters, wherein (A) and (B) show YFP fluorescence changes which illustrate the effects of YPI-H05 and YPI-A4948 on the Cl⁻/I⁻exchange activity of SLC26A9 in LN-215-YFP cells stably expressing human SLC26A9; (C) and (D) show YFP fluorescence changes which illustrate the inhibitory effects of YPI-H05 and YPI-A4948 on the I⁻ transport activity of CFTR in FRT-YFP cells stably expressing human CFTR; and (E) and (F) show YFP fluorescence changes which illustrate the inhibitory effects of YPI-H05 and YPI-A4948 on the I⁻ transport activity of ANO1 in FRT-YFP cells stably expressing human ANO1.
Figure 4 shows the effect of YPI-H05 and YPI-A4948 on cell viability in CHO-K1 cells. Cells were treated with YPI-H05 and YPI-A4948 for 24 hours, and the cell viability was determined by MTS colorimetric assay (mean ± SEM, n = 3).
Figure 5 shows the effect of YPI-H05 on airway surface liquid (ASL) which was decreased by IL-4 treatment in the differentiated state of primary cultured cells from human nasal epithelial cells (HNE) (mean ± SEM, n = 3). *P<0.05.
Figure 6 is the administration schedule designed to confirm the protective effect due to the pre- and post-YPI-A4948 treatment in the LPS-induced acute lung injury model.
Figure 7 confirms the inhibitory effect of acute lung injury phenotype due to pre- and post-YPI-A4948 treatment in the LPS-induced acute lung injury model, wherein (A) shows the average body weight of mice, (B) shows the BALF total cell count, and (C) shows BALF protein concentration.
Figure 8 confirms the reduction effect of lung injury due to pre- and post- YPI-A4948 treatment in an LPS-induced acute lung injury model, wherein the top panel shows the results of BALF cytospin staining; and the bottom panel shows the results of H&E lung tissue staining.
Figure 9 confirms the diuretic effect due to YPI-A4948 treatment in a mouse model.
Figure 10 shows the Adriamycin administration schedule for establishing a heart failure mouse model.
Figure 11 shows the results of cardiac ultrasound imaging before and after administration of Adriamycin.
Figure 12 confirms the diuretic effect due to the administration of YPI-A4948 alone or the co-administration of YPI-A4948 and furosemide in an Adriamycin-induced heart failure mouse model.
Figure 13a is a diagram confirming the effect of YPI-A4948 on the Cl⁻/SCN⁻exchange activity of pendrin in human alveolar epithelial cells (hAEC) overexpressing pendrin. Figure 13b shows protein and mRNA expression levels depending on YPI-A4948 concentration after administration of lipid polysaccharide (LPS) in human alveolar epithelial cells (hAEC).

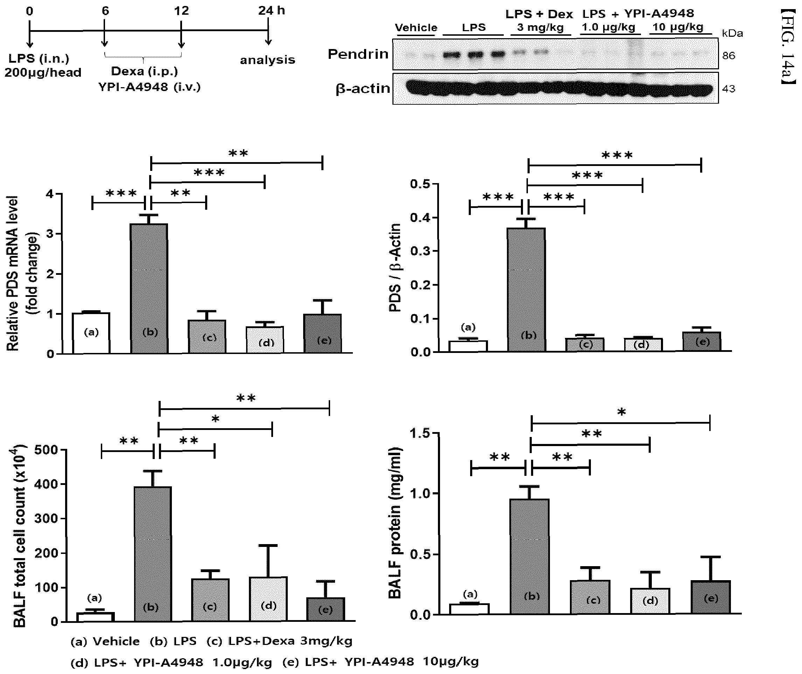

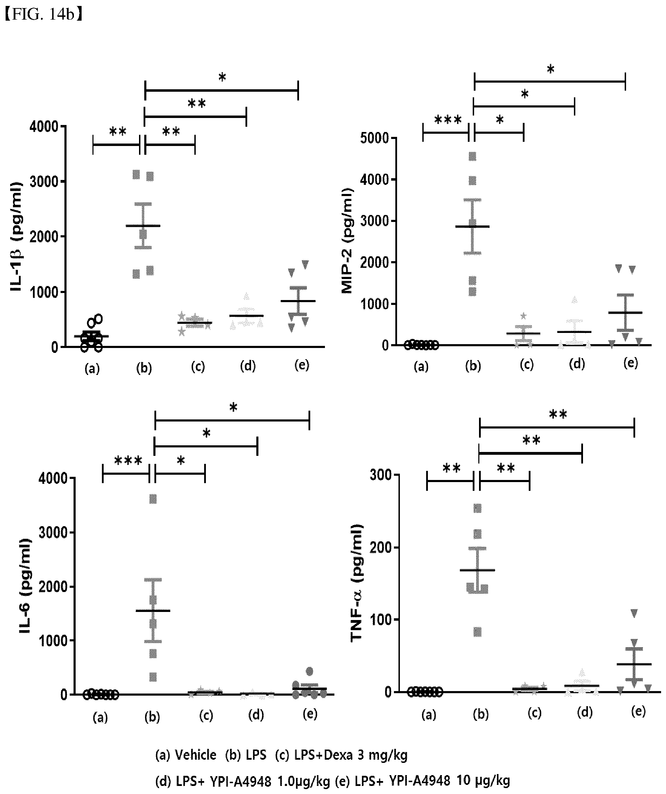

Figure 14a confirms the inhibitory effect of acute lung injury by YPI-A4948 treatment in an LPS-induced lung injury mouse model, which is the results of confirming the administration schedule and the expression of pendrin (PDS) by Western blot from the upper left, a graph digitalizing them, and a graph showing the expression level of pendrin mRNA, the bronchoalveolar lavage fluid (BALF) total cell count, and the BALF protein concentration. Figure 14b is a graph confirming the levels of four types of inflammatory cytokines in the lungs due to YPI-A4948 treatment. In Figures 14a and 14b, *P < 0.05, ** P < 0.01, *** P < 0.001; analysis is carried out using one-way ANOVA with Bonferroni's post-hoc test.

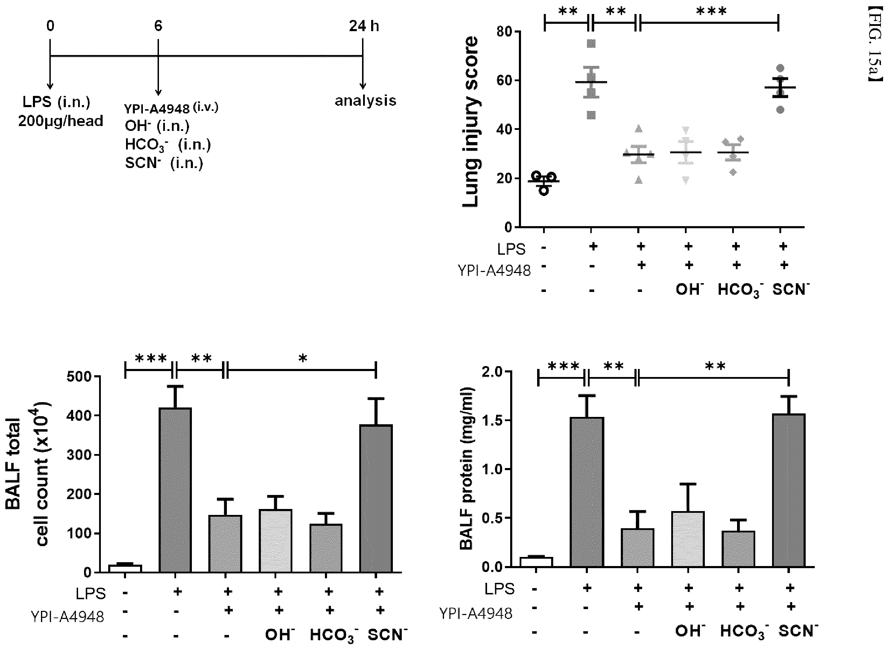

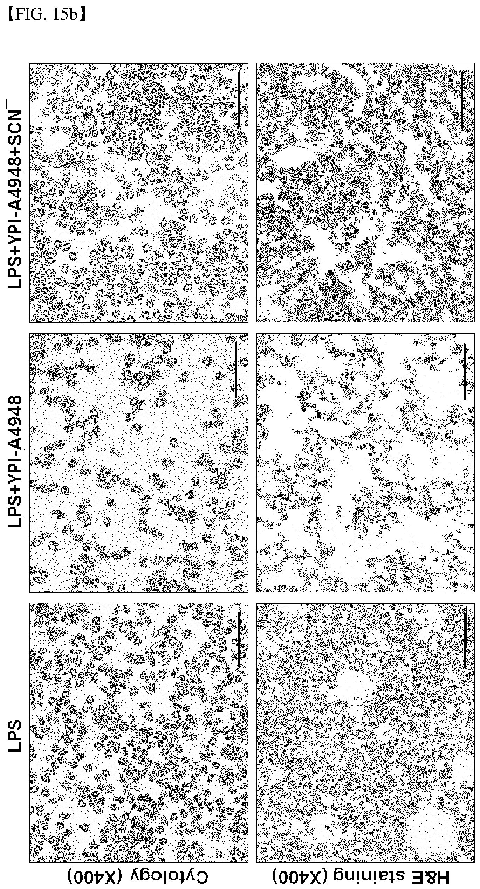

Figures 15a and b are graphs confirming the change in YPI-A4948 efficacy when anion was further supplied from outside in an LPS-induced lung injury mouse model. Figure 15a is a graph showing the administration schedule, lung injury score, BALF total cell number, and BALF protein concentration from the upper left. In Figure 15b, the upper panel shows the cytopathological staining results of BALF, and the lower panel shows the H&E staining results of lung tissue. In Figures 15a and 15b, *P < 0.05, **P < 0.01, ***P < 0.001, and analysis was carried out using one-way ANOVA with Bonferroni's post-hoc test.

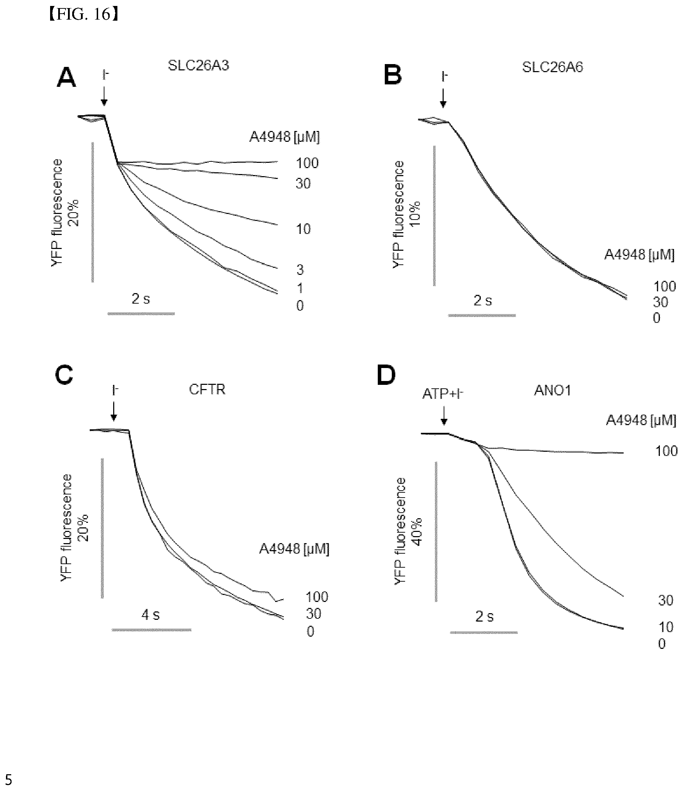

Figure 16 shows the effect of YPI-A4948 on the activities of SLC26A3 and SLC26A6, CFTR, and ANO1, which are anion transporters, wherein (A) shows YFP fluorescence changes which illustrate the effects of YPI-A4948 on the Cl⁻/I⁻ exchange activity of SLC26A9 in LN-215-YFP cells stably expressing human SLC26A3; (B) shows YFP fluorescence changes which illustrate the effect of YPI-A4948 on the Cl⁻/I⁻exchange activity of SLC26A9 in LN-215-YFP cells stably expressing human SLC26A6; (C) shows YFP fluorescence changes which illustrate the inhibitory effect of YPI-A4948 on the I⁻ transport activity of CFTR in FRT-YFP cells stably expressing human CFTR; and (D) shows YFP fluorescence changes which illustrate the inhibitory effect of YPI-A4948 on the I⁻ transport activity of ANO1 in FRT-YFP cells stably expressing human ANO1.

### [DETAILED DESCRIPTION OF THE EMBODIMENTS]

### 1. Definition

Unless otherwise defined, all technical and scientific terms used herein have the same meaning as commonly understood by one of ordinary skill in the art to which the present disclosure belongs. The following references provide those skilled in the art with general definitions of many terms used herein: The Cambridge Dictionary of Science and Technology (Walker ed., 1988); The Glossary of Genetics, 5th Ed., R. Rieger et. al. (eds.), Springer Verlag (1991); and Hale & Marham, The Harper Collins Dictionary of Biology (1991). As used herein, the following terms have the meanings stated below, unless otherwise specified.

Unless specifically stated or obvious from context, as used herein, the term "or" is understood to be inclusive.

Unless specifically stated or obvious from context, as used herein, the term "about" is understood as within a range of normal tolerance in the art, for example within 2 standard deviations of the mean. The term "about" means within 10%, 9%, 8%, 7%, 6%, 5%, 4%, 3%, 2%, 1%, 0.5%, 0.1%, 0.05%, or 0.01% of a given value. Unless otherwise clear from context, all numerical values provided herein are modified by the term about.

The terms "active agent," "drug," and "pharmaceutical active agent" are used interchangeably herein to refer to a chemical material or compound which, when administered to any subject (e.g., any animal, including human or non-human animals) by any means described herein, induces a desired pharmacological effect (e.g., reduction of inflammation).

The term "additive" as used herein can refer to any additional ingredient that can be added to the compositions and formulas described herein. For example, while providing that the additional ingredient is pharmaceutically acceptable for the particular condition being treated, the additives may include excipients (e.g., one or more excipients), antioxidants (e.g., one or more antioxidants), stabilizers (e.g., one or more stabilizers), preservatives (e.g., one or more preservatives), pH adjusting and/or buffering agents (e.g., one or more pH adjusting and/or buffering agents), isotonicity adjusting agents (e.g., one or more isotonicity adjusting agents), thickening agents (e.g., one or more thickening agents), suspending agents (e.g., one or more suspending agents), binding agents (e.g., one or more binding agents), viscosity-increasing agents (e.g., one or more viscosity-increasing agents), and the like. Further, the additive may include treatment agents, such as calcium phosphate, magnesium stearate, talc, monosaccharide, disaccharide, starch, gelatin, cellulose, methyl cellulose, sodium carboxymethyl cellulose, dextrose, hydroxypropyl-beta-cyclodextrin, polyvinylpyrrolidone, low-melting wax, ion exchange resin, and the like, and drug delivery modifiers, enhancers and a combination of any two or more thereof. Other suitable pharmaceutically acceptable excipients are described in "Remington's Pharmaceutical Sciences" Mack Pub. Co., New Jersey (1991), and "Remington: The Science and Practice of Pharmacy", Lippincott Williams & Wilkins, Philadelphia, 20th edition (2003) and 21st edition (2005), which is incorporated herein by reference. The additive described herein can be used as any suitable drug.

As used herein, the term "administrating" refers to oral administration, administration as a suppository, topical contact, intravenous, parenteral, intraperitoneal, intramuscular, intralesional, intrathecal, intranasal, intravitreal or subcutaneous administration to a subject, or implantation of a sustained-release device, such as a mini-osmotic pump. Administration is by any route, including parenteral and transmucosal routes (e.g., oral, nasal, pulmonary, rectal, buccal, vaginal, ocular and transdermal).

"Analog" and "derivative" are used herein interchangeably, and refers to a compound that possesses the same core as the parent compound, but differs from the parent compound in bond order, in the absence or presence of one or more atoms and/or groups of atoms, and a combination thereof. The derivative may differ from the parent compound, for example, in one or more substituents present on the core, which may include one or more atoms, functional groups or substructures. Further, the derivative may differ from the parent compound in bond order between atoms within the core. In general, a derivative can be predicted, at least theoretically, to be formed from the parent compound via chemical and/or physical processes.

As used herein, "antioxidants" may refer to man-made or natural substances that may prevent or delay some types of cell damage and/or oxidation. Antioxidants are found in many foods, including fruits and vegetables. Further, they are available as dietary supplements. Exemplary antioxidants may include β-carotene, lutein, lycopene, selenium, vitamin A, vitamin C and vitamin E. Additionally, other antioxidants known to those skilled in the art can be used. The antioxidants described herein can be used in any suitable amount.

By "co-administration" it is meant that a compound or composition described herein is administered at the same time, just prior to, or just after the administration of additional therapies or active agents or additives described herein. The compound or the composition of the present disclosure can be administered alone or can be co-administered to a patient. Co-administration is meant to include simultaneous or sequential administration of the compound individually or in combination (more than one compound or agent). The preparations can also be combined, when desired, with other active substances.

As used herein, the terms "comprises", "comprising", "containing" and "having" and the like can have the meaning ascribed to them, and can mean "includes", "including", and the like; "consisting essentially of" or "consists essentially of" likewise has the meaning as ascribed to them" and the term is open-ended, allowing for the presence of more than that which is recited so long as basic or novel characteristics of that which is recited is not changed by the presence of more than that which is recited, but excludes prior art embodiments.

As used herein, "concurrent administration" includes overlapping in duration at least in part. For example, when two agents (e.g., any compositions described herein that has bioactivity) are administered concurrently, their administration occurs within a certain desired time. The agents' administration may begin and end on the same day. The administration of one agent can also precede the administration of a second agent by day(s) as long as both agents are taken on the same day at least once. Similarly, the administration of one agent can extend beyond the administration of a second agent as long as both agents are taken on the same day at least once. The bioactive agents/agents do not have to be taken at the same time each day to include concurrent administration.

As used herein, "effective amount" or "therapeutically effective amount" refers to the amount of an agent that is sufficient to affect a desired biological effect, such as a beneficial results, including clinical results. The effective amount therefore depends upon the context in which it is applied. An effective amount may vary according to factors known in the art, such as the disease state, age, sex and weight of the individual being treated. Several divided doses may be administered daily or the dose may be proportionally reduced as indicated by the urgency of the therapeutic situation. In addition, the compositions/agents of the present disclosure can be administered as frequently as necessary to achieve a therapeutic amount.

As used herein, the term "gel" can refer to a material that is not a readily flowable liquid but is not a solid, i.e., semi-solid. Gels can be formed from naturally occurring or synthetic materials. The gels are not aligned but slightly aligned, showing some birefringence, liquid crystal properties. The gel may be administered topically.

As used herein, the term "respiratory disease" has its conventional medical meaning and includes, but not limited to, asthma, acute or chronic bronchitis, allergic rhinitis, acute respiratory infection, acute upper respiratory infection, cystic fibrosis, acute respiratory distress syndrome(ARDS), acute lung injury(ALI), chronic obstructive pulmonary disease(COPD), and diseases and disorder closely related to respiratory diseases.

As used herein, the term "inhibit" means to prevent, decrease, slow-down or arrest. In one embodiment, when the amount or rate of a process or reaction occurring in the presence of the compound or composition is decreased by at least about 10% as compared to the amount or rate of the process or reaction in the absence of the compound or composition, the composition or compound is considered to inhibit the viability of at least one protein (e.g., pendrin). In another embodiment, when the amount or rate of a process or reaction occurring in the presence of the compound or composition is decreased by at least about 20% as compared to the amount or rate of the process or reaction in the absence of the compound or composition, the composition or compound is considered to inhibit a process or reaction. In yet another embodiment, when the amount or rate of a process or reaction occurring in the presence of the compound or composition is decreased by about 25% or more, about 30%, about 40%, about 50%, about 60%, about 70%, about 75% or about 80% as compared to the amount or rate of the process or reaction in the absence of the compound or composition, the composition or compound is considered to inhibit one or more proteins (e.g., pendrin). In yet another embodiment, the compound or composition is considered to inhibit the viability of one or more proteins, i.e., to arrest their development.

As used herein, "intermittent administration" includes the administration of an agent for a period of time (which can be considered a "first period of administration"), followed by a time during which the agent is not taken or is taken at a lower maintenance dose (which can be considered "off-period") followed by a period during which the agent is administered again (which can be considered a "second period of administration"). Generally, during the second period of administration, the dosage level of the agent will match that administered during the first period of administration but can be increased or decreased as medically necessary.

"Jelly" according to the present disclosure is a class of gels, which are semisolid systems that consist of suspensions made up either small inorganic particles or large organic molecules interpenetrated by a liquid, in which the structural coherent matrix contains a high portion of liquid, usually water.

"Liquid" as used herein is a dosage form consisting of a composition in its liquid state. A liquid is pourable; it flows and conforms to its container at room temperature. Liquids display Newtonian or pseudoplastic flow behavior.

In embodiments, a "semi-liquid" as used herein may have properties of both a liquid and another formulation (i.e., a suspension, an emulsion, a solution, a cream, a gel, a jelly, and the like).

As used herein, the term "ointment" may refer to a highly viscous liquid or semi-liquid formulation that can be used for the therapeutic treatment of a disease, syndrome or condition.

As used herein, "pharmaceutically acceptable carrier" is intended to include any and all solvents, dispersion media, coatings, antibacterial and antifungal compounds, isotonic and absorption delaying compounds, and the like, which are physiologically compatible. The type of carrier may be selected based on the intended route of administration. The pharmaceutically acceptable carriers include sterile aqueous solutions or dispersions and sterile powders for the extemporaneous preparation of sterile topical solutions or dispersions. The use of such media and agents for pharmaceutically active substances is well known in the art. Except insofar as any conventional media or agent is incompatible with the composition (e.g., Chemical Formula 1, a derivative or analog of Chemical Formula 1, or a pharmaceutically acceptable salt, solvent, hydrate or polymorph thereof described herein), use thereof in the compositions for the disclosure is contemplated.

As used herein, "pharmaceutical carriers" or "carriers" may further include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of the physiologically acceptable carrier include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharide, disaccharide, and other carbohydrates including glucose, mannose, or dextrin; chelating agents such as EDTA; sugar alcohols such as mannitol or sorbitol; salt-forming counterions such as sodium; and/or nonionic surfactants such as Tween^{™}, polyethylene glycol (PEG), and Pluronics^{™}. Additionally, 'pharmaceutically acceptable' means being approved, or capable of being approved, by the federal and state governments exercise regulatory authority, or by such authority in a country other than the United States, or listed in the United States Pharmacopoeia or other generally recognized pharmacopoeia for use in animals, and more particularly in humans.

The term "pharmaceutically acceptable salts or complexes" refers to salts or complexes of the below-specified compound represented by Chemical Formula 1. Examples of such salts include, but are not limited to, base addition salts formed by reaction of compounds of Chemical Formula I with organic or inorganic bases such as hydroxide, carbonate or bicarbonate of a metal cation such as those selected in the group consisting of alkali metals (e.g., sodium, potassium or lithium), alkaline earth metals (e.g., calcium or magnesium), or with a primary, secondary or tertiary alkyl amine. Amine salts derived from methylamine, dimethylamine, trimethylamine, ethylamine, diethylamine, triethylamine, morpholine, N-methyl-D-glucamine, N,N'-bis(phenylmethyl)-1,2-ethanediamine, tromethamine, ethanolamine, diethanolamine, ethylene diamine, N-methylmorpholine, procaine, piperidine, piperazine, and the like are contemplated as being within the scope of this invention.

Also, as used herein, "salt" or "salt form" or "pharmaceutically acceptable salt" may include base addition salts derived from inorganic bases such as, for example, sodium, potassium, ammonium, calcium or ferric hydroxide, and organic bases such as, for example, isopropylamine, trimethylamine, 2-ethylamino-ethanol, histidine, procaine, and the like (formed with free carboxyl or other anionic groups). Such salts are formed as acid addition salts having any free cationic groups, and are generally formed with inorganic acids such as, for example, hydrochloric acid, sulfuric acid or phosphoric acid, or organic acids such as, for example, acetic acid, citric acid, p-toluenesulfonic acid, methanesulfonic acid, oxalic acid, tartaric acid, mandelic acid and the like. The salts of the present disclosure may include amine salts formed by protonation of amino groups with an inorganic acid such as hydrochloric acid, hydrobromic acid, hydroiodic acid, sulfuric acid, phosphoric acid, and the like. Further, the salts of the present disclosure may include amine salts formed by protonation of amino groups with a suitable organic acid such as p-toluenesulfonic acid, acetic acid, and the like.

As used herein, the term "pH agent" or "buffer" may refer to compounds or buffers that are useful as a pH adjusting agent. These may include, but are not limited to, glycerol buffers, citrate buffers, borate buffers, acetate buffers, gluconate buffers, phosphate buffers, or citric acid-phosphate buffers. The pH agent or buffer may be used in any suitable amount.

The term, "preservative" as described herein may refer to a substance or chemical material that prevents undesirable chemical changes of the compound or compositions or formulas described herein. Suitable preservatives may include, for example, benzalkonium chloride, thimerosal, chlorobutanol, methyl paraben, propyl paraben, phenylethyl alcohol, edetate disodium sorbic acid, Onamer M Polyquat, cetyl bromide, cetyl pyridinium chloride, benzyl bromide, EDTA, phenylmercury nitrate, phenylmercury acetate, thimerosal, merthiolate, acetate and phenylmercury borate, polymyxin B sulphate, methyl and propyl parabens, quaternary ammonium chloride, sodium benzoate, sodium propionate, and sodium perborate, and other agents known to those skilled in the art, or a combination thereof. The preservative may be used in any suitable amount.

The terms "prevent," "preventing," or "prevention," and other grammatical equivalents as used herein, include to keep from developing, occur, hinder or avert a disease or condition symptoms as well as to decrease the occurrence of symptoms. The prevention may be complete (i.e., no detectable symptoms) or partial, so that fewer symptoms may be observed than would likely occur absent treatment. The terms further include a prophylactic benefit. For a disease or condition to be prevented, the compositions may be administered to a patient at risk of developing a particular disease, or to a patient reporting one or more of the physiological symptoms of a disease, even though a diagnosis of this disease may not have been made.

Ranges provided herein are understood to be shorthand for all of the values within the range. For example, a range of 1 to 10 is understood to include any number, combination of numbers, or sub-range from the group consisting 1, 2, 3, 4, 5, 6, 7, 8, 9 or 10 as well as all intervening decimal values between the aforementioned integers such as, for example, 1.1, 1.2, 1.3, 1.4, 1.5, 1.6, 1.7, 1.8 and 1.9. With respect to sub-ranges, "nested sub-ranges" that extend from either end point of the range are specifically contemplated. For example, a nested sub-range of an exemplary range of 1 to 50 may comprise 1 to 10, 1 to 20, 1 to 30, and 1 to 40 in one direction, or 50 to 40, 50 to 30, 50 to 20, and 50 to 1 0 in the other direction. Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it is understood that the particular value forms another aspect. It is further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. It is also understood that throughout the application, data are provided in a number of different formats and that this data represent endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11 , 12, 13, and 14 are also disclosed.

Additional excipients which are contemplated for use in the practice of the present disclosure may be those available to those of ordinary skill in the art, and for example, the relevant contents are found in United States Pharmacopoeia Vol. XXII and National Formulary Vol. XVII, U.S. Pharmacopoeia Convention, Inc., Rockville, Md. (1989), which is incorporated herein by reference.

The "semisolid gel" according to the present disclosure is a semisolid. The semisolid formulation apparent viscosity may increase with concentration.

As used herein, "sequential administration" includes that the administration of two agents (e.g., the compounds or compositions described herein) occurs separately on the same day or do not occur on a same day (e.g., occurs on consecutive days).

The term "solution" according to the present disclosure may be a clear, homogeneous liquid dosage form that contains one or more chemical substances dissolved in a solvent or mixture of mutually miscible solvents. A solution is a liquid preparation that contains one or more dissolved chemical substances in a suitable solvent or mixture of mutually miscible solvents. Because molecules of a drug substance in solution are uniformly dispersed, the use of solutions as dosage forms generally provides assurance of uniform dosage upon administration and good accuracy when the solution is diluted or otherwise mixed.

The term "solvent" as used herein refers to a liquid solvent either aqueous or nonaqueous. The selection of the solvent depends notably on the solubility of the composition on said solvent and on the mode of administration. Aqueous solvent may consist solely of water, or may consist of water plus one or more miscible solvents, and may contain dissolved solutes such as sugars, buffers, salts or other excipients. The more commonly used non-aqueous solvents are the short-chain organic alcohols, such as, methanol, ethanol, propanol, short-chain ketones, such as acetone, and poly alcohols, such as glycerol.

By "subject" or "patient" is meant either a human or non-human animal, such as a mammal. "Subject" may include any animal, including horses, dogs, cats, pigs, goats, rabbits, hamsters, monkeys, guinea pigs, rats, mice, lizards, snakes, sheep, cattle, fish, and birds. A human subject may be referred to as a patient.

The "suspension" as used herein is a liquid dosage form that contains solid particles dispersed in a liquid vehicle.

As used herein, "viscosity" refers to a fluid's resistance to flow. Viscosity agents may be used herein and include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose, other agents known to those skilled in the art, or a combination thereof.

The term "weight percent" or "% (w/w)" refers to a percentage of a component in a solution that is calculated on the basis of weight for the component and the solvent. For example, a 1%(w/w) solution of a component would have 1 g of the component dissolved in a 100 g of solvent. The term "volume percent" or "%(v/v)" refers to a percentage of a component in a solution that is calculated on the basis of volume for the component and the solvent. For example, a 1% (v/v) solution of a component would have 1 ml of the component dissolved in a 100 ml of solvent. The term "weight/volume percent" or "%(w/v)" refers to a percentage of a component in a solution that is calculated on the basis of weight for the component and on the basis of volume for the solvent. For example, a 1.0%(w/v) solution of a component would have 1 g of the component dissolved in a 100 ml of solvent.

As used herein, the term "syndrome" may refer to a group of symptoms that consistently occur together or a condition characterized by a set of associated symptoms. A syndrome (e.g., acute respiratory distress syndrome) may be a set of medical signs and symptoms that are correlated with each other and often, are correlated with a specific disease. On the other hand, a disease may be a health condition that has a clearly defined reason behind it. However, a syndrome (from the Greek word meaning 'run together') may produce a number of symptoms without an identifiable cause. They may suggest the possibility of an underlying disease or even the chances of developing a disease.

The terms "treat," "treating" or "treatment," and other grammatical equivalents as used herein, include alleviating, abating, ameliorating, or preventing a disease, condition (e.g., acute respiratory distress syndrome) or symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition, and are intended to include prophylaxis. The terms further include achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder.

The term "health functional food" refers to foods or food supplements produced or processed with raw materials, functional ingredients, active pharmaceutical ingredients or additives, which are useful for improving the physiological functions of the human body and/or for nutrition and/or preservation.

The term "acute respiratory distress syndrome (ARDS)" refers to a medical condition occurring in critically ill patients characterized by widespread inflammation in the lungs. ARDS is a clinical phenotype which may be triggered by various pathologies such as pneumonia and sepsis. Extensive damage to the cells that form the alveolar barrier, surfactant dysfunction, abnormal coagulation, and activation of the innate immune response are hallmarks of ARDS.

The term "acute lung injury (ALI)" refers to a syndrome of inflammation and increased permeability that is associated with hypoxemia and classical radiographic appearance. At the most severe end of this spectrum lies ARDS.

The term "airway surface liquid (ASL)" refers to the thin layer of fluid coating the apical surface of airway epithelium cells at an air interface. ASL plays a pivotal role in maintaining airway homeostasis. ASL volume, pH and ionic balance are directly involved in the regulation of antibacterial activity, ciliary function and mucosal clearance.

The term "inflammatory airway disease" refers to various inflammatory airway disorders including asthma, acute or chronic bronchitis, allergic rhinitis, acute respiratory infection, acute upper respiratory infection, cystic fibrosis, acute respiratory distress syndrome(ARDS), acute lung injury(ALI), chronic obstructive pulmonary disease (COPD), and the like.

The term "inhibitor" used in the context of the invention is defined as a molecule, two or more molecules or pharmaceutical compositions that completely or partially inhibit the activity of a target or two or more targets that induce a desired biological effect. Non-limiting examples of targets include enzymes, receptors, ion-channels or transporters (e.g., pendrin), and the like. An "inhibitor" can reversibly or irreversibly inhibit a target, and reversible inhibition includes a competitive inhibition, an uncompetitive inhibition, a non-competitive inhibition, and a mixed inhibition.

The term "alkyl", when used alone or in combination with other terms, comprises a straight chain or branched C₁-C₂₀ alkyl which refers to monovalent alkyl groups having 1 to 20 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethyl propyl, n-hexyl, 2-methylpentyl, 3-methylpentyl, 4-methylpentyl, n-heptyl, 2-methylhexyl, 3-methylhexyl, 4-methylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl and n-eicosanil, and the like. Preferably, these include C₁-C₉ alkyl, more preferably C₁-C₆ alkyl, particularly preferably C₁-C₄ alkyl, which, by analogy, refer respectively to monovalent alkyl group having 1 to 9 carbon atoms, a monovalent alkyl group having 1 to 6 carbon atoms and a monovalent alkyl group having 1 to 4 carbon atoms.

The term "alkenyl" when used alone or in combination with other terms, comprises a straight chain or branched C₂-C₂₀ alkenyl. It may have any available number of double bonds in any available positions, and the configuration of the double bond may be the (E) or (Z) configuration. This term is exemplified by groups such as vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 1-heptenyl, 1-octenyl, geranyl, 1-decenyl, 1-tetradecenyl, 1-octadecenyl, 9-octadecenyl, 1-eicosenyl, 3,7, 11, 15-tetramethyl-1-hexadecenyl, and the like. Preferably, they include C₂-C₈ alkenyl, more preferably C₂-C₆ alkenyl. Among others, especially preferred are vinyl or ethenyl (-CH=CH₂), n-2-propenyl (allyl, -CH₂CH=CH₂), isopropenyl, 1-propenyl, 2-methyl-1-propenyl, 1-butenyl, 2-butenyl, 3-methyl-2-butenyl, and the like.

The term "alkynyl" when used alone or in combination with other terms, comprises a straight chain or branched C₂-C₂₀ alkynyl. It may have any available number of triple bonds in any available position. This term is exemplified by groups such as alkynyl groups that may have a carbon number of 2 to 20, and optionally a double bond or triple bond, such as ethynyl (-C=CH), 1-propynyl, 2-propynyl (propargyl: -CH₂C≡CH), 2-butynyl, 2-pentene-4-ynyl, and the like. Particularly, these include C₂-C₈ alkynyl, more preferably C₂-C₆ alkynyl and the like. Preferably those include C₂-C₆ alkynyl which refers to groups having 2 to 6 carbon atoms and having at least 1 or 2 sites of alkynyl unsaturation.

The term "heteroalkyl" refers to C₁-C₁₂-alkyl, preferably C₁-C₆-alkyl, wherein the at least one carbon has been replaced by a heteroatom selected from O, N or S, including 2-methoxy ethyl and the like.

The term "aryl" refers to an unsaturated aromatic carbocyclic group of from 6 to 14 carbon atoms having a single ring (e.g., phenyl) or multiple condensed rings (e.g., indenyl, naphthyl, 2,3-dihydro-1H-indenyl, 1,2,3,4-tetrahydronaphthyl). Aryl includes phenyl, naphthyl, anthryl, phenanthrenyl, and the like.

The term "C₁-C₆ alkyl aryl" refers to aryl groups having a C₁-C₆ alkyl substituent, including methyl phenyl, ethyl phenyl, t-butyl phenyl, and the like.

The term "aryl C₁-C₆ alkyl" refers to a C₁-C₆ alkyl group having an aryl substituent, including 3-phenylpropanyl, benzyl, and the like.

The term "heteroaryl" refers to a monocyclic heteroaromatic, or a bicyclic or a tricyclic fused-ring heteroaromatic group. Particular examples of heteroaromatic groups include optionally substituted pyridyl, pyrrolyl, pyrimidinyl, furyl, thienyl, imidazolyl, oxazolyl, isoxazolyl, thiazolyl, isothiazolyl, pyrazolyl, 1H-pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, phthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl, benzoquinolyl, benzo[d][1,3]dioxol-5-yl, 3,4-dihydro-1H-pyrano[4,3-c]pyridyl, quinolin-2(1H)-one, 4H-chromene, 1H-indole, and the like.

The term "C₁-C₆ alkyl heteroaryl" refers to a heteroaryl group having a C₁-C₆ alkyl substituent, including methyl furyl, t-butyl furyl, and the like.

The term "heteroaryl C₁-C₆ alkyl" refers to a C₁-C₆ alkyl group having a heteroaryl substituent, including furyl methyl, and the like.

The term "C₂-C₆ alkenyl aryl" refers to an aryl group having a C₂-C₆ alkenyl substituent, including vinyl phenyl, and the like.

The term "aryl C₂-C₆ alkenyl" refers to a C₂-C₆ alkenyl group having an aryl substituent, including phenyl vinyl, and the like.

The term "C₂-C₆ alkenyl heteroaryl" refers to a heteroaryl group having a C₂-C₆ alkenyl substituent, including vinyl pyridinyl, and the like.

The term "heteroaryl C₂-C₆ alkenyl" refers to a C₁-C₆ alkenyl group having a heteroaryl substituent, including pyridinyl vinyl, and the like.

The term "C₃-C₈-cycloalkyl" refers to a saturated carbocyclic group of 3 to 8 carbon atoms having a single ring (e.g., cyclohexyl) or multiple condensed rings (e.g., norbomyl). C₃-C₈-cycloalkyl includes cyclopentyl, cyclohexyl, norbornyl, and the like.

The term "heterocycloalkyl" refers to a C₃-C₈-cycloalkyl group or multiple condensed rings according to the definition above, in which up to 3 carbon atoms are replaced by heteroatoms selected from the group consisting of O, S and NR (where R is defined as hydrogen or methyl). Heterocycloalkyl includes lactam or lactone. Non-limiting examples are pyrrolidinyl, piperidinyl, piperazinyl, morpholinyl, tetrahydrofuranyl, decahydroisoquinolinyl, octahydro-1H-pyrano[3,4-c]pyridinyl, 4-methylene-5(4H)-one, pyrrolidin-2-one, and the like.

The term "C₁-C₆ alkyl C₃-C₈-cycloalkyl" refers to a C₃-C₈-cycloalkyl group having a C₁-C₆ alkyl substituent, including methyl cyclopentyl, and the like.

The term "C₃-C₈-cycloalkyl C₁-C₆ alkyl" refers to a C₁-C₆ alkyl group having a C₃-C₈-cycloalkyl substituent, including 3-cyclopentyl propyl, and the like.

The term "C₁-C₆ alkyl heterocycloalkyl" refers to heterocycloalkyl groups having a C₁-C₆ alkyl substituent, including 4-methylpiperidinyl, and the like.

The term "heterocycloalkyl C₁-C₆ alkyl" refers to a C₁-C₆ alkyl group having a heterocycloalkyl substituent, including (1-methylpiperidin-4-yl) methyl, and the like.

The term "carboxy" refers to the group -C(O)OH.

The term "carboxy C₁-C₆ alkyl" refers to a C₁-C₆ alkyl group having a carboxy substituent, including 2-carboxyethyl, and the like.

The term "acyl" refers to the group -C(O)R where R includes H, "alkyl," preferably "C₁-C₆ alkyl," "aryl ," "heteroaryl ," "C₃-C₈-cycloalkyl," "heterocycloalkyl," "aryl C₁-C₆ alkyl," "heteroaryl C₁-C₆ alkyl," "C₃-C₈-cycloalkyl C₁-C₆ alkyl" or "heterocycloalkyl C₁-C₆ alkyl", including acetyl and the like.

The term "acyl C₁-C₆ alkyl" refers to a C₁-C₆ alkyl group having an acyl substituent, including 2-acetylethyl, and the like.

The term "acyl aryl" refers to ab aryl group having an acyl substituent, including 2-acetylphenyl, and the like.

The term "acyloxy" refers to the group -OC(O)R where R includes H, "C₁-C₆ alkyl", "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl", including acetyloxy, and the like.

The term "acyloxy C₁-C₆ alkyl" refers to a C₁-C₆ alkyl group having an acyloxy substituent, including 2-(ethylcarbonyloxy)ethyl, and the like.

The term "alkoxy" refers to the group -OR where R includes "C₁-C₆ alkyl", "aryl", "heteroaryl", "aryl C₁-C₆ alkyl" or "heteroaryl C₁-C₆ alkyl". Preferred alkoxy groups include for example, methoxy, ethoxy, phenoxy, and the like.

The term "alkoxy C₁-C₆ alkyl" refers to a C₁-C₆ alkyl group having an alkoxy substituent, including methoxyethyl and the like.

The term "alkoxycarbonyl" refers to the group -C(O)OR where R includes "C₁-C₆ alkyl", "aryl", "heteroaryl", "aryl C₁-C₆ alkyl", "heteroaryl C₁-C₆ alkyl" or "heteroalkyl".

The term "alkoxycarbonyl C₁-C₆ alkyl" refers to a C₁-C₆ alkyl group having an alkoxycarbonyl substituent, including 2-(benzyloxycarbonyl)ethyl, and the like.

The term "aminocarbonyl" refers to the group -C(O)NRR' where R and R' are independently H, C₁-C₆ alkyl, aryl, heteroaryl, "aryl C₁-C₆ alkyl" or "heteroaryl C₁-C₆ alkyl," including N-phenyl carbonyl, and the like.

The term "aminocarbonyl C₁-C₆ alkyl" refers to an alkyl group having an aminocarbonyl substituent, including 2-(dimethylaminocarbonyl)ethyl, N-ethyl acetamidyl, N,N-diethyl-acetamidyl, and the like.

The term "acylamino" refers to the group -NRC(O)R' where R and R' are independently H, "C₁-C₆ alkyl," "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "cycloalkyl C₁-C₆ alkyl," or "heterocycloalkyl C₁-C₆ alkyl", including acetylamino, and the like.

The term "acylamino C₁-C₆ alkyl" refers to a C₁-C₆ alkyl group having an acylamino substituent, including 2-(propionylamino)ethyl, and the like.

The term "ureido" refers to the group -NRC(O)NR'R" where R, R' and R" are independently H, "C₁-C₆ alkyl," "alkenyl," "alkynyl," "C₃-C₈-cycloalkyl," "heterocycloalkyl," "C₁-C₆ aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "cycloalkyl C₁-C₆ alkyl," or "heterocycloalkyl C₁-C₆ alkyl," and where R' and R," together with the nitrogen atom to which they are attached, can optionally form a 3-8-membered heterocycloalkyl ring.

The term "ureido C₁-C₆ alkyl" refers to a C₁-C₆ alkyl group having an ureido substituent, including 2-(N'-methylureido)ethyl, and the like.

The term "carbamate" refers to the group -NRC(O)OR' where R and R' are independently "C₁-C₆ alkyl," "C₂-C₆ alkenyl," "C₂-C₆ alkynyl," "C₃-C₈-cycloalkyl," "heterocycloalkyl," "aryl," "heteroaryl "C₁-C₆ alkyl aryl," "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "cycloalkyl C₁-C₆ alkyl," or "heterocycloalkyl C₁-C₆ alkyl" and optionally R can also be hydrogen.

The term "amino" refers to the group -NRR' where R and R' are independently H , "C₁-C₆ alkyl", "aryl", "heteroaryl", "C₁-C₆ alkyl aryl", "C₁-C₆ alkyl heteroaryl," "cycloalkyl," or "heterocycloalkyl," and where R and R', together with the nitrogen atom to which they are attached, can optionally form a 3-8-membered heterocycloalkyl ring.

The term "amino alkyl" refers to alkyl groups having an amino substituent, including 2-(1-pyrrolidinyl)ethyl, and the like.

The term "ammonium" refers to a positively charged group -N⁺RR'R" where R, R' and R" are independently "C₁-C₆ alkyl", "C₁-C₆ alkyl aryl", "C₁-C₆ alkyl heteroaryl," "cycloalkyl," or "heterocycloalkyl," and where R and R', together with the nitrogen atom to which they are attached, can optionally form a 3-8-membered heterocycloalkyl ring.

The term "ammonium alkyl" refers to an alkyl group having an ammonium substituent, including 1-ethylpyrrolidinium, and the like.

The term "halogen" refers to fluoro, chloro, bromo and iodo atoms.

The term "sulfonyloxy" refers to a group -OSO₂R wherein R is selected from "C₁-C₆ alkyl," "C₁-C₆ alkyl" substituted with halogens, e.g., an -OSO₂CF₃ group, "C₂-C₆ alkenyl," "alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "cycloalkyl C₁-C₆ alkyl," or "heterocycloalkyl alkyl".

The term "sulfonyloxy C₁-C₆ alkyl" refers to an alkyl group having a sulfonyloxy substituent, including 2-(methylsulfonyloxy)ethyl, and the like.

The term "sulfonyl" refers to a group "-SO₂R" wherein R is selected from "aryl," "heteroaryl," "C₁-C₆ alkyl," "C₁-C₆ alkyl" substituted with halogens, e.g., an - SO₂CF₃ group, "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "cycloalkyl C₁-C₆ alkyl," or "heterocycloalkyl C₁-C₆ alkyl".

The term "sulfonyl C₁-C₆ alkyl" refers to alkyl groups having a sulfonyl substituent, including 2-(methylsulfonyl) ethyl, and the like.

The term "sulfinyl" refers to a group "-S(O)-R" where R is selected from "alkyl", "alkyl" substituted with halogens, e.g., a -SOCF₃ group, "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "heteroaryl C₂-C₆ alkynyl," "C₃-C₈-cycloalkyl C₁-C₆ alkyl," or "heterocycloalkyl C₁-C₆ alkyl".

The term "sulfinyl alkyl" refers to an alkyl group having a sulfinyl substituent, including 2-(methylsulfinyl)ethyl, and the like.

The term "sulfanyl" refers to a group -SR where R includes H, "C₁-C₆ alkyl," "C₁-C₆ alkyl" substituted with halogens, e.g., a -SCF₃ group, "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkenyl," "heteroaryl C₂-C₆ alkenyl," "aryl C₂-C₆ alkynyl," "alkynylheteroaryl," "cycloalkyl C₁-C₆ alkyl," or "heterocycloalkyl C₁-C₆ alkyl". Preferred sulfanyl group includes methylsulfanyl, ethylsulfanyl, and the like.

The term "sulfanyl C₁-C₆ alkyl" refers to a C₁-C₅-alkyl group having a sulfanyl substituent, including 2-(ethylsulfanyl)ethyl, and the like.

The term "sulfonylamino" refers to a group -NRSO₂R' where R and R' are independently "C₁-C₆ alkyl," "C₂-C₆ alkenyl," "C₂-C₆ alkynyl," "C₃-C₈-cycloalkyl," "heterocycloalkyl," "aryl," "heteroaryl," "aryl C₁-C₆ alkyl", "heteroaryl C₁-C₆ alkyl," "aryl C₂-C₆ alkynyl," "heteroaryl C₂-C₆ alkynyl, "aryl C₂-C₆ alkynyl", "heteroaryl C₂-C₆ alkynyl ," "C₃-C₈-cycloalkyl C₁-C₆ alkyl," or "heterocycloalkyl C₁-C₆ alkyl".

The term "sulfonylamino C₁-C₆ alkyl" refers to an alkyl group having an aminosulfonyl substituent, including 2-(ethylsulfonylamino)ethyl, and the like.

The term "aminosulfonyl" refers to a group -SO₂NRR' where R and R' are independently H , "C₁-C₆ alkyl," "C₂-C₆ alkenyl," " C₂-C₆ alkynyl," " C₃-C₈-cycloalkyl," "heterocycloalkyl," "aryl," "heteroaryl," "aryl C₁-C₆ alkyl", "heteroaryl C₁-C₆ alkyl," "aryl alkenyl," "heteroaryl C₂-C₆ alkenyl," "aryl C₂-C₆ alkynyl," "heteroaryl C₂-C₆ alkynyl," " C₃-C₈-cycloalkyl C₁-C₆ alkyl," or "heterocycloalkyl C₁-C₆ alkyl", and where R and R', together with the nitrogen atom to which they are attached, can optionally form a 3-8-membered heterocycloalkyl ring. Aminosulfonyl groups include cyclohexylaminosulfonyl, piperidinylsulfonyl, and the like.

The term "aminosulfonyl C₁-C₆ alkyl" refers to a C₁-C₆ alkyl group having an aminosulfonyl substituent, including 2-(cyclohexylaminosulfonyl)ethyl, and the like.

Unless otherwise constrained by the definition of the individual substituent, all the above substituents should be understood as being all optionally substituted.

Unless otherwise constrained by the definition of the individual substituent, the term "substituted" refers to groups substituted with 1 to 5 substituents selected from the group consisting of "C₁-C₆ alkyl," "C₂-C₆ alkenyl," "C₂-C₆ alkynyl," "C₃-C₈-cycloalkyl," "heterocycloalkyl," "C₁-C₆ alkyl aryl," "C₁-C₆ alkyl heteroaryl," "C₁-C₆ alkyl cycloalkyl," "C₁-C₆ alkyl heterocycloalkyl," "amino," "aminosulfonyl," "ammonium," "acyl amino," "amino carbonyl," "aryl," "heteroaryl," "sulfinyl," "sulfonyl," "alkoxy," "alkoxy carbonyl," "carbamate," "sulfanyl," "halogen," trihalomethyl, cyano, hydroxy, mercapto, nitro, and the like.

### 2. Compound

In a first aspect of the present disclosure, there is provided a compound represented by Chemical Formula 1 below, an E- or Z-isomer thereof, an optical isomer thereof, a precursor thereof, a pharmaceutically acceptable salt thereof, a solvate thereof or a mixture of two isomers thereof: 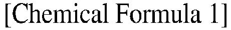 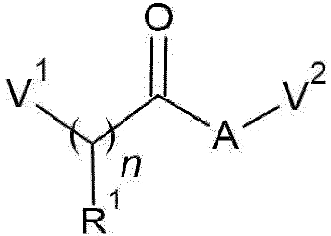
wherein, in Chemical Formula 1,
V¹ may be a C₅∼C₁₀ heteroaryl containing 1 to 3 nitrogen atoms, which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂~C₁₀ alkenyl, C₂~C₁₀ alkynyl, C(O)OR², C(O)R², OR², and OCR²F₂,
A may be a C₅∼C₇ heteroaryl containing 1 to 3 nitrogen atoms or a C₅∼C₇ heterocycloalkyl containing 1 to 3 heteroatoms, which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂~C₁₀ alkenyl, C₂~C₁₀ alkynyl, C(O)OR², C(O)R², OR², OCR²F₂ and OCOR²,
V² may be an aryl, a C₁~C₁₀ alkyl, a C₅∼C₇ cycloalkyl, or a C(O)OR², which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂~C₁₀ alkenyl, C₂~C₁₀ alkynyl, C(O)OR², C(O)R², OR², OCR²F₂ and OCOR²,
R¹ may be one or more independently selected from the group consisting of hydrogen, halogen, C₁∼C₁₀ alkyl, C₂~C₆ alkenyl, and C₂~C₆ alkynyl,
R² may be independently selected from the group consisting of hydrogen, trifluoromethyl, trifluoromethoxy, aryl, aryl (C₁~C₁₀ alkyl), C₁∼C₁₀ alkylaryl, heteroaryl, heteroaryl (C₁~C₁₀ alkyl), C₁∼C₁₀ alkylheteroaryl, C₁∼C₁₀ alkyl, C₂~C₆ alkenyl, C₂~C₆ alkynyl, C₃~C₆ cycloalkyl and heterocycloalkyl, and
n may be an integer from 1 to 5.

More specifically, in Chemical Formula 1, V¹ may be indolyl, isoindolyl, 3H-indolyl, 1H-indolyl, quinolyl, isoquinolyl, 5,6,7,8-tetrahydroquinolyl, 5,6,7,8-tetrahydroisoquinolyl, quinolizinyl, quinazolinyl, phthalazinyl, cinnolinyl or naphthyridinyl, which is substituted with one or more groups independently selected from the group consisting of hydrogen, halogen, trifluoromethyl, C₃∼C₇ cycloalkyl, C₁~C₁₀ alkyl, C₂~C₁₀ alkenyl, C₂~C₁₀ alkynyl, C(O)OR², C(O)R², OR², OCR²F₂ and OCOR²,
A may be pyrrolidinyl, piperidinyl, piperazinyl or decahydroisoquinolinyl, which is substituted with one or more groups independently selected from the group consisting of hydrogen, halogen, trifluoromethyl, C₃∼C₇ cycloalkyl, C₁∼C₁₀ alkyl, C₂~C₁₀ alkenyl and C₂~C₁₀ alkynyl,
V² may be a C₅∼C₇ aryl, or a C(O)OR², which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂~C₁₀ alkenyl, C₂~C₁₀ alkynyl, C₃~C₆ cycloalkyl, C(O)OR², C(O)R², OR² and OCR²F₂,
R² may be independently selected from the group consisting of hydrogen, trifluoromethyl, aryl, aryl (C₁~C₁₀ alkyl), C₁∼C₁₀ alkylaryl, C₁∼C₁₀ alkyl, C₂~C₆ alkenyl, C₂~C₆ alkynyl, and C₃~C₆ cycloalkyl, and
n may be an integer from 1 to 5.

More specifically, the present disclosure relates to a compound represented by Chemical Formula 1a below, an E- or Z-isomer thereof, an optical isomer thereof, a precursor thereof, a pharmaceutically acceptable salt thereof, a solvate thereof or a mixture of two isomers thereof: 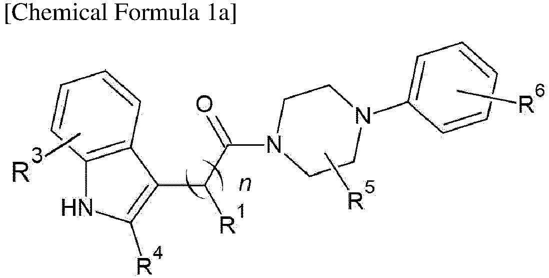
wherein, in Chemical Formula 1a,
R¹ may be one or more independently selected from the group consisting of hydrogen, halogen, C₁∼C₁₀ alkyl, C₂~C₆ alkenyl, and C₂~C₆ alkynyl,
R³ may be one or more groups independently selected from the group consisting of hydrogen, halogen, trifluoromethyl, C₃∼C₇ cycloalkyl, C₁∼C₁₀ alkyl, C₂∼C₁₀ alkenyl, C₂~C₁₀ alkynyl, C(O)OR², C(O)R², OR² and OCR²F₂,
R⁴ may be a group independently selected from the group consisting of C(O)OR², C(O)R², OR², OCR²F₂ and OCOR²,
R⁵ may be one or more groups independently selected from the group consisting of hydrogen, C₃∼C₇ cycloalkyl, C₁∼C₁₀ alkyl, C₂~C₁₀ alkenyl and C₂~C₁₀ alkynyl,
R⁶ may be one or more groups independently selected from the group consisting of hydrogen, halogen, trifluoromethyl, C₃∼C₇ cycloalkyl, C₁~C₁₀ alkyl, C₂∼C₁₀ alkenyl, C₂~C₁₀ alkynyl, C(O)OR², C(O)R², OR² and OCR²F₂,
R² may be independently selected from the group consisting of hydrogen, trifluoromethyl, aryl, aryl (C₁~C₁₀ alkyl), C₁∼C₁₀ alkylaryl, C₁~C₁₀ alkyl, C₂~C₆ alkenyl, C₂~C₆ alkynyl, and C₃~C₆ cycloalkyl, and
n may be an integer from 1 to 5.

More specifically, the present disclosure relates to a compound represented by Chemical Formula 1b below, an E- or Z-isomer thereof, an optical isomer thereof, a precursor thereof, a pharmaceutically acceptable salt thereof, a solvate thereof or a mixture of two isomers thereof: 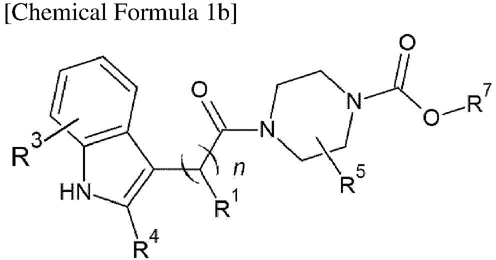
wherein, in Chemical Formula 1b,
R¹ may be one or more independently selected from the group consisting of hydrogen, halogen, C₁∼C₁₀ alkyl, C₂~C₆ alkenyl, and C₂~C₆ alkynyl,
R³ may be one or more groups independently selected from the group consisting of hydrogen, halogen, trifluoromethyl, C₃∼C₇ cycloalkyl, C₁∼C₁₀ alkyl, C₂∼C₁₀ alkenyl, C₂~C₁₀ alkynyl, C(O)OR², C(O)R², OR² and OCR²F₂,
R⁴ may be a group independently selected from the group consisting of C(O)OR², C(O)R², OR², OCR²F₂ and OCOR²,
R⁵ may be one or more groups independently selected from the group consisting of hydrogen, C₃∼C₇ cycloalkyl, C₁∼C₁₀ alkyl, C₂~C₁₀ alkenyl and C₂~C₁₀ alkynyl,
R⁷ may be one or more groups independently selected from the group consisting of hydrogen, halogen, trifluoromethyl, C₃∼C₇ cycloalkyl, C₁~C₁₀ alkyl, C₂~C₁₀ alkenyl, and C₂~C₁₀ alkynyl,
R² may be independently selected from the group consisting of hydrogen, trifluoromethyl, aryl, aryl (C₁~C₁₀ alkyl), C₁∼C₁₀ alkylaryl, C₁~C₁₀ alkyl, C₂~C₆ alkenyl, C₂~C₆ alkynyl, and C₃~C₆ cycloalkyl, and
n may be an integer from 1 to 5.

Preferably, the aforementioned compounds include, but are not limited to:
(1) 5-chloro-3-(2-oxo-2-(4-(4-(trifluoromethyl)phenyl)piperazin-1-yl)ethyl)-1H-indole-2-carboxylic acid;
(2) 5-chloro-3-(2-oxo-2-(4-(2-(trifluoromethyl)phenyl)piperazin-1-yl)ethyl)-1H-indole-2-carboxylic acid;
(3) 5-chloro-3-(2-(4-(2,6-dimethylphenyl)piperazin-1-yl)-2-oxoethyl)-1H-indole-2-carboxylic acid;
(4) 5-chloro-3-(2-(4-(2,4-dimethylphenyl)piperazin-1-yl)-2-oxoethyl)-1H-indole-2-carboxylic acid;
(5) 5-chloro-3-(2-(4-(3-methoxyphenyl)piperazin-1-yl)-2-oxoethyl)-1H-indole-2-carboxylic acid;
(6) 5-chloro-3-(2-(4-(2-chlorophenyl)piperazin-1-yl)-2-oxoethyl)-1H-indole-2-carboxylic acid;
(7) 5-chloro-3-(2-(4-(4-chlorophenyl)piperazin-1-yl)-2-oxoethyl)-1H-indole-2-carboxylic acid;
(8) 3-(2-(4-(2-bromophenyl)piperazin-1-yl)-2-oxoethyl)-5-chloro-1H-indole-2-carboxylic acid;
(9) 3-(2-(4-(3-bromophenyl)piperazin-1-yl)-2-oxoethyl)-5-chloro-1H-indole-2-carboxylic acid;
(10) 3-(2-(4-(4-bromophenyl)piperazin-1-yl)-2-oxoethyl)-5-chloro-1H-indole-2-carboxylic acid;
(11) 5-chloro-3-(2-oxo-2-(4-(*o*-tolyl)piperazin-1-yl)ethyl)-1H-indole-2-carboxylic acid;
(12) 5-chloro-3-(2-oxo-2-(4-(*m*-tolyl)piperazin-1-yl)ethyl)-1H-indole-2-carboxylic acid;
(13) 5-chloro-3-(2-oxo-2-(4-(*p*-tolyl)piperazin-1-yl)ethyl)-1H-indole-2-carboxylic acid;
(14) 3-(2-(4-(*tert*-butoxycarbonyl)piperazin-1-yl)-2-oxoethyl)-5-chloro-1H-indole-2-carboxylic acid;
(15) 5-chloro-3-(2-(4-(2-chloro-6-methylphenyl)piperazin-1-yl)-2-oxoethyl)-1H-indole-2-carboxylic acid;
(16) 5-chloro-3-(2-(4-(2-ethoxyphenyl)piperazin-1-yl)-2-oxoethyl)-1H-indole-2-carboxylic acid;
(17) 5-chloro-3-(2-(3-methyl-4-phenylpiperazin-1-yl)-2-oxoethyl)-1H-indole-2-carboxylic acid;
(18) 5-chloro-3-(2-(4-(4-chlorophenyl)-2,2-dimethylpiperazin-1-yl)-2-oxoethyl)-1H-indole-2-carboxylic acid;
(19) 5-chloro-3-(2-(4-(2-chloro-4-fluorophenyl)piperazin-1-yl)-2-oxoethyl)-1H-indole-2-carboxylic acid;
(20) 5-chloro-3-(2-(4-(2-chloro-5-methoxyphenyl)piperazin-1-yl)-2-oxoethyl)-1H-indole-2-carboxylic acid;
(21) 5-chloro-3-(2-(4-(2,4-dichlorophenyl)piperazin-1-yl)-2-oxoethyl)-1H-indole-2-carboxylic acid;
(22) (R)-5-chloro-3-(2-(2-methyl-4-phenylpiperazin-1-yl)-2-oxoethyl)-1H-indole-2-carboxylic acid;
(23) 5-chloro-3-(2-(4-(2,4-difluorophenyl)piperazin-1-yl)-2-oxoethyl)-1H-indole-2-carboxylic acid;
(24) 5-chloro-3-(2-oxo-2-(4-(3,4,5-trichlorophenyl)piperazin-1-yl)ethyl)-1H-indole-2-carboxylic acid;
(25) 5-chloro-3-(2-(4-(2-fluorophenyl)piperazin-1-yl)-2-oxoethyl)-1H-indole-2-carboxylic acid;
(26) 5-chloro-3-(2-(4-(3-chloro-2-methylphenyl)piperazin-1-yl)-2-oxoethyl)-1H-indole-2-carboxylic acid;
(27) 3-(2-(4-(4-carboxyphenyl)piperazin-1-yl)-2-oxoethyl)-5-chloro-1H-indole-2-carboxylic acid;
(28) 3-(2-(4-(4-acetylphenyl)piperazin-1-yl)-2-oxoethyl)-5-chloro-1H-indole-2-carboxylic acid;
(29) 5-chloro-3-(2-(4-(4-ethoxyphenyl)piperazin-1-yl)-2-oxoethyl)-1H-indole-2-carboxylic acid;
(30) 3-(2-(4-(4-(*tert*-butyl)phenyl)piperazin-1-yl)-2-oxoethyl)-5-chloro-1H-indole-2-carboxylic acid;
(31) 3-(2-(4-(4-(*tert*-butyl)phenyl)piperazin-1-yl)-2-oxoethyl)-5-methyl-1H-indole-2-carboxylic acid;
(32) 3-(2-(4-(4-(*tert*-butyl)phenyl)piperazin-1-yl-2-oxoethyl)-5,7-dimethyl-1H-indole-2-carboxylic acid;
(33) 3-(2-(4-(2,6-dimethylphenyl)piperazin-1-yl)-2-oxoethyl)-5-methyl-1H-indole-2-carboxylic acid;
(34) 3-(2-(4-(2,6-dimethylphenyl)piperazin-1-yl)-2-oxoethyl)-5,7-dimethyl-1H-indole-2-carboxylic acid;
(35) 3-(2-(4-(2-chloro-6-methylphenyl)piperazin-1-yl)-2-oxoethyl)-5-methyl-1H-indole-2-carboxylic acid;
(36) 3-(2-(4-(2-chloro-6-methylphenyl)piperazin-1-yl)-2-oxoethyl)-5,7-dimethyl-1H-indole-2-carboxylic acid;
(37) 3-(2-(4-(2-chloro-6-methylphenyl)piperazin-1-yl)-2-oxoethyl)-5-fluoro-1H-indole-2-carboxylic acid;
(38) 3-(2-(4-(2,6-dimethylphenyl)piperazin-1-yl)-2-oxoethyl)-5-fluoro-1H-indole-2-carboxylic acid;
(39) 3-(2-(4-(4-(*tert*-butyl)phenyl)piperazin-1-yl)-2-oxoethyl)-5-fluoro-1H-indole-2-carboxylic acid;
(40) 3-(2-(4-([1,1'-biphenyl]-4-yl)piperazin-1-yl)-2-oxoethyl)-5-chloro-1H-indole-2- carboxylic acid; or
(41) 5-chloro-3-(2-(4-(2,5-dimethylphenyl)piperazin-1-yl-)-2-oxoethyl)-1H-indole-2-carboxylic acid.

The term 'compound of the invention' and equivalent expressions herein include the compound represented by Chemical Formula 1 described above, and such expressions include an E- or Z-isomer thereof, an optical isomer thereof, a precursor thereof, a pharmaceutically acceptable salt thereof, a solvate thereof or a mixture of two isomers thereof, which are newly synthesized.

The present disclosure is further illustrated by the following examples which do not limit the scope of the invention in any way.

### 3. Preparation Method

In the first aspect of the present invention, further provided herein is a method for preparing a compound represented by Chemical Formula 1, Chemical Formula 1a, and Chemical Formula 1b. The synthesis method of **YPI-A4948,** which is a typical compound according to Chemical Formula 1 of the present disclosure is described in detail in Example 1, and the novel compounds of the present disclosure can be prepared based on the synthetic method described in Example 1.

In some embodiments, bases used during organic synthesis may be organic or inorganic bases. Non-limiting examples of the organic base include pyridine, trimethylamine, N,N-diisopropylethylamine(DIPEA) and 1,8-diazabicyclo[5.4.0]undec-7-ene(DBU). Non-limiting examples of the inorganic base include sodium hydroxide, sodium carbonate, potassium carbonate, cesium carbonate and sodium hydride. These may be used alone or in combination in stoichiometric or excess amounts. Non-limiting examples of solvents that can be used are ethers (e.g., tetrahydrofuran(THF), diethyl ether and 1,2-dimethoxyethane), alcohols (e.g., methanol, ethanol, propanol and butanol), dimethylformamide(DMF), dimethylsulfoxide(DMSO), dichloromethane(DCM), dichloroethane, water, and acetone. The above solvents may be used alone or in combination.

### 4. Compositions and Formulations

The present disclosure encompasses a pharmaceutical composition comprising the compound as described herein and formulation suitable for administering the compound as described herein. Formulations of pharmaceutical compositions suitable for administrating by a medically acceptable means are optionally encompassed in the present disclosure. A pharmaceutical formulation may contain a pharmaceutically acceptable additive or carrier and a pharmaceutically acceptable compound (composition) suitable for the means of administration.

The compounds described herein may be formulations (including pharmaceutical compositions) with additives, such as excipients (e.g., one or more excipients), antioxidants (e.g., one or more antioxidants), stabilizers (e.g., one or more stabilizers), preservatives (e.g., one or more preservatives), pH adjusting and/or buffering agents (e.g., one or more pH adjusting and/or buffering agents), isotonicity adjusting agents (e.g., one or more isotonicity adjusting agents), thickening agents (e.g., one or more thickening agents), suspending agents (e.g., one or more suspending agents), binding agents (e.g., one or more binding agent), viscosity-increasing agents (e.g., one or more viscosity-increasing agents), and the like, and is provided as an additional pharmaceutically acceptable ingredient for the particular condition being treated. In some embodiments, the formulations can include a combination of additional ingredients as described herein (e.g., 2, 3, 4, 5, 6, 7, 8 or more additional ingredients). In some embodiments, the additive may include, for example, treatment agents, such as calcium phosphate, magnesium stearate, talc, monosaccharide, disaccharide, starch, gelatin, cellulose, methyl cellulose, sodium carboxymethyl cellulose, dextrose, hydroxypropyl-beta-cyclodextrin, polyvinylpyrrolidone, low-melting wax, ion exchange resin, and the like, and drug delivery modifiers, enhancers and a combination of any two or more thereof.

Other suitable pharmaceutically acceptable excipients are described in "Remington's Pharmaceutical Sciences" Mack Pub. Co., New Jersey (1991), and "Remington: The Science and Practice of Pharmacy", Lippincott Williams & Wilkins, Philadelphia, 20th edition (2003) and 21st edition (2005), which is incorporated herein by reference.

Formulations of the compositions described herein may be suitable for oral administration, which may consist of inhalation, nasal spray, intravenous, intramuscular injection, intravitreal injection, ointments or solutions, suspensions, semi-liquids, semi-solids, gels, semi-solid gels, jellies, emulsions, ointments, tablets, liquids and creams. Tablet form may include one or more selected from lactose, sucrose, mannitol, sorbitol, calcium phosphate, corn starch, potato starch, microcrystalline cellulose, gelatin, colloidal silicon dioxide, talc, magnesium stearate, stearic acid and other excipients, colorants, fillers, binders, diluents, buffering agent, humectants, preservatives, flavoring agents, dyes, disintegrants and pharmaceutically suitable carriers. Capsules may contain suitable excipients along with the compound, or the compound may be used alone in the shell. All of these formulated compounds may be administered alone, co-administered, intermittently, sequentially or concurrently.

### 5. Administration

The compositions of this invention may be administered orally, parenterally, sublingually, transdermally, rectally, transmucosally, topically, by inhalation, buccally, intranasally, or using any route of combination thereof, but is not limited thereto. The term parenteral includes intravenous, intraarterial, intraperitoneal, subcutaneous, intramuscular, intrathecal and intraarterial, but is not limited thereto. Further, the composition of this invention can be administered as an implant, which allows slow controlled intravenous administration as well as slow release of the composition.

The drug dose administered to an individual in single or multiple doses will vary depending on several factors, including pharmacokinetic characteristics, patient condition and characteristics (gender, age, weight, health, size), severity of symptoms, concomitant therapy, treatment frequency, and desired effect.

According to one embodiment of the invention, the compounds according to the present disclosure and pharmaceutical formulations thereof may be administered alone or in combination with adjuvants useful in the treatment of respiratory disorders or diseases. According to another embodiment of the present disclosure, the compounds according to the present disclosure and pharmaceutical formulations thereof may be administered in conjunction with radiotherapy.

The present disclosure encompasses the administration of a compound according to the present disclosure or a pharmaceutical formulation thereof, wherein the compound according to the invention or the pharmaceutical formulation thereof is administered in a therapeutically effective amount to a subject simultaneously or sequentially prior to other therapeutic regimens or adjuvants (e.g., multidrug regimens) useful for cancer treatment. A compound according to the present disclosure or a pharmaceutical formulation thereof administered concurrently with the adjuvant may be administered in the same or different composition(s) and by the same or different route(s) of administration.

In one embodiment, a patient according to the invention is a patient suffering from respiratory disorders or diseases, such as bronchial asthma, bronchitis, allergic rhinitis, adult respiratory syndrome, cystic fibrosis, pulmonary viral infection (influenza), pulmonary hypertension, idiopathic pulmonary fibrosis, chronic obstructive pulmonary disease (COPD), and the like.

In another embodiment, the compounds according to the present disclosure and pharmaceutical formulations thereof may be administered alone or in combination with one or more other diuretics. The diuretic may be selected from the group consisting of furosemide, torasemide, bumetanide, etacrynic acid, azosemide, muzolimine, piretanide, tripamide, bendroflumethazide, chlorothiazide, hydrochlorothiazide, hydroflumethiazide, methychlothiazide, polythiazide, trichlormethiazide, chlorthialidone, indapamide, metolazone, quinethazone, etozolin, triamteren, amiloride, and pharmaceutically acceptable derivatives thereof, but are not limited thereto.

Preferably, the compounds according to the present disclosure or pharmaceutical formulations thereof are administered together with furosemide, which can be administered in an appropriate ratio considering the toxicity of each drug. For example, the compounds or the pharmaceutical formulations thereof and furosemide may be administered in a weight ratio of about 1:10, but is not limited thereto.

### 6. Use of the compound according to the present disclosure

In a second aspect of the present disclosure, there is provided a use of a compound represented by Chemical Formula 1, Chemical Formula 1a or Chemical Formula 1b, a mixture of compounds or a pharmaceutical composition thereof for the prevention, amelioration or treatment of respiratory diseases (inflammatory airway diseases).

In another embodiment, the present disclosure provides a use of a compound represented by Chemical Formula 1, Chemical Formula 1a or Chemical Formula 1b, and a pharmaceutical composition thereof as a pendrin inhibitor.

In another embodiment, there is provided use of compounds represented by Chemical Formula 1, Chemical Formula 1a or Chemical Formula 1b, and pharmaceutical compositions thereof, which preserves the volume of airway surface fluid (ASL).

In another embodiment, the present disclosure provides a use for respiratory diseases (inflammatory airway diseases) in one or more selected from the group consisting of asthma, acute or chronic bronchitis, allergic rhinitis, acute respiratory infection, acute upper respiratory infection, cystic fibrosis, acute respiratory distress syndrome (ARDS), acute lung injury (ALI), chronic obstructive pulmonary disease (COPD), and the like.

In another embodiment, the present disclosure provides a use of a compound represented by Chemical Formula 1, Chemical Formula 1a or Chemical Formula 1b, a mixture of compounds, or a health functional food composition thereof for the prevention or amelioration of respiratory diseases (inflammatory airway diseases) as active ingredients in health functional foods.

In another embodiment, the present disclosure provides a use of the compound represented by Chemical Formula 1, Chemical Formula 1a or Chemical Formula 1b, and a health functional food composition thereof as a pendrin inhibitor for the prevention or amelioration of respiratory diseases (inflammatory airway diseases) as active ingredients in health functional foods.

In another embodiment, the present invention provides a use of a compound represented by Chemical Formula 1, Chemical Formula 1a or Chemical Formula 1b, and a pharmaceutical composition thereof, which preserves or increases the volume of airway surface liquid (ASL) for the prevention or amelioration of respiratory diseases (inflammatory airway diseases) as active ingredients in health functional foods.

In another embodiment, the present disclosure provides a use for the prevention or amelioration of respiratory diseases (inflammatory airway diseases) as an active ingredient in health functional foods, wherein the respiratory disease (inflammatory airway disease) selects one or more from the group consisting of asthma, acute or chronic bronchitis, allergic rhinitis, acute respiratory infection, acute upper respiratory infection, cystic fibrosis, acute respiratory distress syndrome (ARDS), acute lung injury (ALI) or chronic obstructive pulmonary disease (COPD).

A third aspect of the present disclosure provides a use of a compound represented by Chemical Formula 1, Chemical Formula 1a or Chemical Formula 1b, a mixture of compounds or a pharmaceutical composition thereof, which exhibits a diuretic effect.

In another embodiment, the present disclosure provides a use of a compound represented by Chemical Formula 1, Chemical Formula 1a or Chemical Formula 1b, a mixture of compounds, or a health functional food composition thereof, which exhibits a diuretic effect as active ingredients in health functional foods.

Non-limiting examples of detailed experiments that do not limit the entire experiment are described herein. It should be understood that the details of the invention described herein serves as examples, and modifications and variations can be made to other specific areas or forms by those skilled in the art without changing the technical idea or essential features of the invention. The examples described are provided for illustrative purpose only, and are not intended to limit the scope of the invention.

The names of the compound were generated by ChemDraw Professional V.15.1. The compound according to the present disclosure includes not only the compound represented by Chemical Formula 1, a tautomer thereof, a geometric isomer thereof (e.g., e, z-isomer), optically active form thereof as optical isomer thereof, diastereomer thereof and racemate thereof, but also pharmaceutically acceptable salt thereof. The derivatives exemplified herein can be prepared from readily available starting materials using the following general methods and procedures. It will be appreciated that given typical or preferred experimental conditions (i.e., reaction temperature, time, moles of reagents, solvents, etc.), other experimental conditions may be used unless otherwise stated. Optimal reaction conditions may vary depending on the specific reactants or solvents used, but such conditions can be determined by one skilled in the art using routine optimization procedures.

References cited herein are incorporated herein by reference in their entirety. The invention is not limited in scope to the specific embodiments described herein, which are intended as single examples of individual aspects of the invention, and functionally equivalent methods and components are within the scope of the invention. Indeed, various modifications of the present disclosure, in addition to those shown and described herein, will become apparent to those skilled in the art from the foregoing description and accompanying drawings. Such modifications are intended to fall within the scope of the appended claims.

### [Example 1]

### Preparation of new compound

The method for synthesizing a new compound according to the present disclosure is as follows.

### (1) 3-(carboxymethyl)-5-methyl-1H-indole-2-carboxylic acid

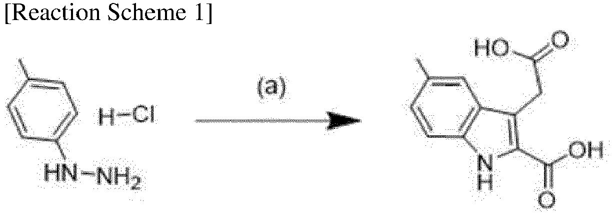

(a) 2-ketoglutaric acid, distilled water, heated under reflux at 100°C for 16 hours.

1 Equivalent (3 g) of (4-chlorophenyl)hydrazine hydrochloride and 1.5 equivalents of 2-ketoglutaric acid were dissolved in distilled water and heated under reflux at 100°C for 16 hours. After confirming the completion of the reaction, the mixture was cooled to room temperature, and extraction was repeated three times using distilled water and ethyl acetate. The collected organic layer was dehydrated using sodium sulfate, and then sodium sulfate was removed using filter paper. The filtrate was distilled under reduced pressure.

### (2) Ethyl 3-(2-ethoxy-2-oxoethyl)-5-methyl-1H-indole-2-carboxylate

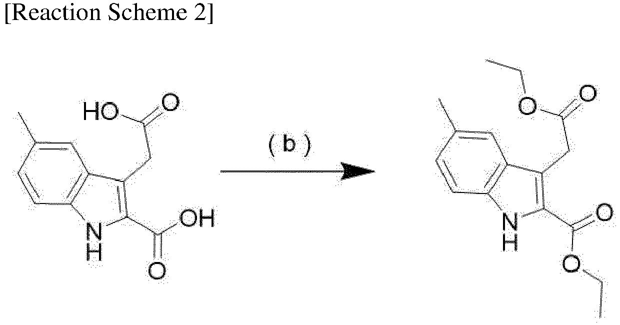

### (b) Concentrated sulfuric acid (95%), ethanol, heated under reflux at 78°C for 16 hours.

The stock solution (3.5 g) obtained in the previous step was dissolved in ethanol, and then treated with 7 mL of concentrated sulfuric acid while carefully stirring using a glass rod. After completely treating with concentrated sulfuric acid, the mixture was heated under at 78°C for 16 hours. After confirming the completion of the reaction, the reaction mixture was distilled under reduced pressure. Then, distilled water and ethyl acetate were added to the stock solution, and then neutralized using sodium bicarbonate. After neutralization was completed, extraction was repeated three times using distilled water and ethyl acetate. The collected organic layer was dehydrated using sodium sulfate, and then the sodium sulfate was removed using filter paper. The filtrate was distilled under reduced pressure, and then purified by column chromatography. (mobile phase: ethyl acetate: n-hexane (1:10 v/v))

### (3) 2-(2-(ethoxycarbonyl)-5-methyl-1H-indol-3-yl)acetic acid

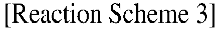

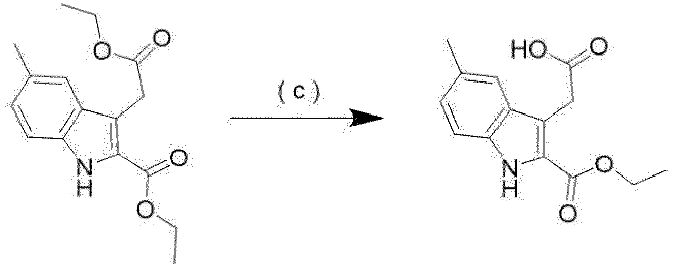

### (c) Sodium hydroxide, tetrahydrofuran, ethanol, distilled water, stirred at -10°C for 16 hours

The compound purified in the previous step (1.6 g) was dissolved in 20 mL of tetrahydrofuran and 20 mL of ethanol. Then, 1.1 equivalents of sodium hydroxide was dissolved in 4 mL of distilled water. The solution was stirred at -10°C using a low-temperature reactor for 16 hours. After confirming the completion of the reaction, the reaction mixture was neutralized using 10% hydrochloric acid solution. Then, extraction was repeated three times using distilled water and ethyl acetate. The collected organic layer was dehydrated using sodium sulfate, and then sodium sulfate was removed using filter paper. The filtrate was distilled under reduced pressure and then purified by column chromatography. (ethyl acetate: n-hexane = 1:10 → 1:1)

### (4) Ethyl 3-(2-(4-(2-chloro-6-methylphenyl)piperazin-1-yl)-2-oxoethyl)-5-methyl-1H-indole-2-carboxylate

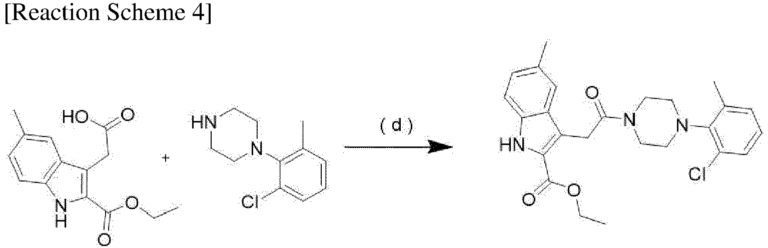

### (d) Hexafluorophosphate azabenzotriazole tetramethyluronium, N,N-diisopropylethylamine, tetrahydrofuran, stirred at room temperature for 5 hours

The compound (50 mg) purified in the previous step was dissolved in tetrahydrofuran, and then treated with 1.2 equivalents of phenyl piperazine, 1.1 equivalents of hexafluorophosphate azabenzotriazole tetramethyluronium(HATU), and 3 equivalents of N,N-diisopropylethylamine (DIPEA). Stirring was performed at room temperature for 5 hours. After confirming the completion of the reaction, the result was distilled under reduced pressure and purified by column chromatography. (ethyl acetate: n-hexane = 1:5)

### (5) 3-(2-(4-(2-chloro-6-methylphenyl)piperazin-1-yl)-2-oxoethyl)-5-methyl-1H-indole-2-carboxylic acid (YPI-A4948)

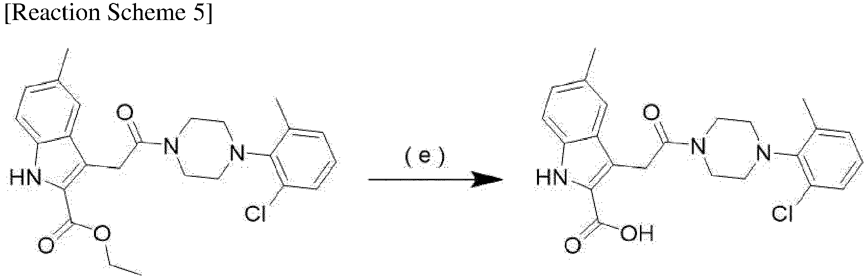

### (e) Lithium hydroxide, tetrahydrofuran, distilled water, stirred at room temperature for 16 hours

The compound (70 mg) purified in the previous step was dissolved in tetrahydrofuran, and then 5 equivalents of lithium hydroxide was dissolved and treated in 5 mL of distilled water. After all lithium hydroxide has been treated, stirring was performed at room temperature for 16 hours. When the completion of the reaction was confirmed, distillation was performed under reduced pressure. Then, in a state where all remaining solvents were removed, distilled water was added, and then treated with a 10% hydrochloric acid solution to precipitate carboxylic acid. The precipitated carboxylic acid was filtered using filter paper, and then washed with distilled water and purified.

### 1-1. 5-chloro-3-(2-(4-(2,5-dimethylphenyl)piperazin-1-yl-)-2-oxoethyl)-1H-indole-2-carboxylic acid (YPI-H05)

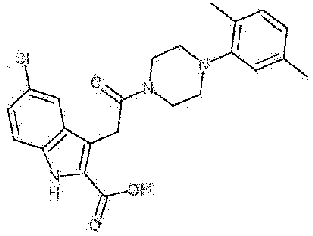

### 1-2. 5-Chloro-3-(2-oxo-2-(4-(4-(trifluoromethyl)phenyl)piperazin-1-yl)ethyl)-1H-indole-2-carboxylic acid (YPI-A4779)

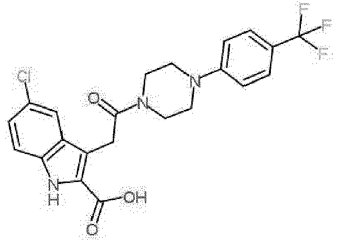

¹H NMR (300 MHz, DMSO-*d₆*) δ 13.28 (s, 1H), 11.79 (s, 1H), 7.76-7.75 (m, 1H), 7.54-7.51 (m, 2H), 7.42-7.40 (m, 1H), 7.25-7.22 (m, 1H), 7.09-7.07 (m, 2H), 4.21 (s, 2H), 3.78-3.62 (m, 4H), 3.37-3.26 (m, 4H).

ESI (m/z) 464(MH-).

### 1-3. 5-Chloro-3-(2-oxo-2-(4-(2-(trifluoromethyl)phenyl)piperazin-1-yl)ethyl)-1H-indole-2-carboxylic acid (YPI-A4781)

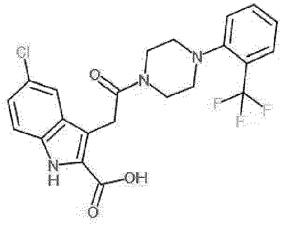

1H NMR (300 MHz, DMSO-d6) δ 13.28 (s, 1H), 11.78 (s, 1H), 7.77 (m, 1H), 7.70-7.65 (m, 2H), 7.53-7.51 (m, 1H), 7.44-7.41 (m, 1H), 7.39-7.35 (m, 1H), 7.27-7.24 (m, 1H), 4.20 (s, 2H), 3.74-3.59 (m, 4H), 2.85-2.81 (m, 4H).

ESI (m/z) 466(MH+), 464(MH-).

### 1-4. 5-Chloro-3-(2-(4-(2,6-dimethylphenyl)piperazin-1-yl)-2-oxoethyl)-1H-indole-2-carboxylic acid (YPI-A4783)

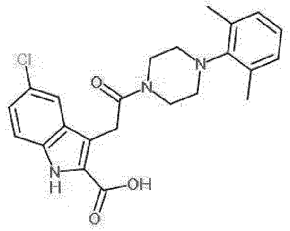

1H NMR (300 MHz, DMSO-d6) δ 13.26 (s, 1H), 11.77 (s, 1H), 7.76 (m, 1H), 7.43-7.41 (m, 1H), 7.26-7.23 (m, 1H), 6.97-6.89 (m, 3H), 4.21 (s, 2H), 3.66-3.58 (m, 4H), 2.90-2.83 (m, 4H), 2.18 (s, 6H).

ESI (m/z) 425(MH+), 424(MH-).

### 1-5. 5-Chloro-3-(2-(4-(2,3-dimethylphenyl)piperazin-1-yl)-2-oxoethyl)-1H-indole-2-carboxylic acid (YPI-A4799)

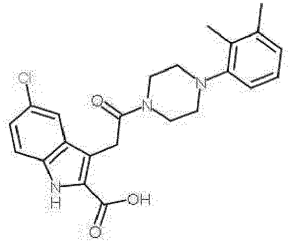

1H NMR (300 MHz, DMSO-d6) δ 11.75 (s, 1H), 7.70-7.69 (m, 1H), 7.61-7.59 (m, 1H), 7.05-7.03 (m, 1H), 6.97-6.94 (m, 1H), 6.84-6.82 (m, 1H), 6.67-6.65 (m, 1H), 4.38 (s, 2H), 3.86-3.58 (m, 4H), 2.61 (s, 2H), 2.46 (s, 2H), 2.16 (s, 3H), 2.08 (s, 3H).

ESI (m/z) 425(MH+), 424(MH-).

### 1-6. 5-Chloro-3-(2-(4-(2,4-dimethylphenyl)piperazin-1-yl)-2-oxoethyl)-1H-indole-2-carboxylic acid (YPI-A4801)

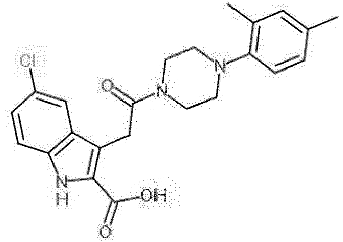

1H NMR (300 MHz, DMSO-d6) δ 11.56 (s, 1H), 7.69 (m, 1H), 7.51-7.49 (m, 1H), 7.08-7.05 (m, 1H), 6.93 (m, 1H), 6.87-6.85 (m, 1H), 6.71-6.69 (m, 1H), 4.34 (s, 2H), 3.81-3.56 (m, 4H), 2.62-2.50 (m, 4H), 2.17 (s, 3H), 2.15 (s, 3H).

ESI (m/z) 426(MH+), 424(MH-).

### 1-7. 5-Chloro-3-(2-(4-(2-methoxyphenyl)piperazin-1-yl)-2-oxoethyl)-1H-indole-2-carboxylic acid (YPI-A4876)

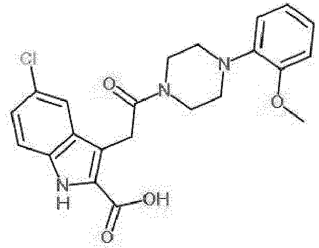

1H NMR (300 MHz, DMSO-d6) δ 13.24 (s, 1H), 11.75 (s, 1H), 7.75-7.74 (m, 1H), 7.43-7.40 (m, 1H), 7.26-7.22 (m, 1H), 6.98-6.85 (m, 4H), 4.19 (s, 2H), 3.79 (s, 3H), 3.75-3.60 (m, 4H), 2.90 (m, 4H).

ESI (m/z) 426(MH-).

### 1-8. 5-Chloro-3-(2-(4-(3-methoxyphenyl)piperazin-1-yl)-2-oxoethyl)-1H-indole-2-carboxylic acid (YPI-A4877)

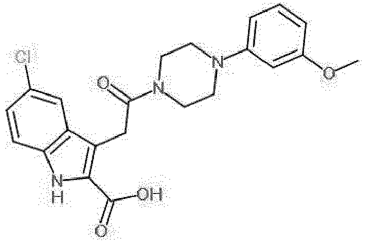

1H NMR (300 MHz, DMSO-d6) δ 11.59 (s, 1H), 7.71-7.70 (m, 1H), 7.50-7.47 (m, 1H), 7.11-7.03 (m, 2H), 6.44-6.33 (m, 3H), 4.33 (s, 2H), 3.83-3.56 (m, 4H), 3.68 (s, 3H), 2.98-2.89 (m, 4H).

ESI (m/z) 428(MH+), 426(MH-).

### 1-9. 5-Chloro-3-(2-(4-(4-methoxyphenyl)piperazin-1-yl)-2-oxoethyl)-1H-indole-2-carboxylic acid (YPI-A4878)

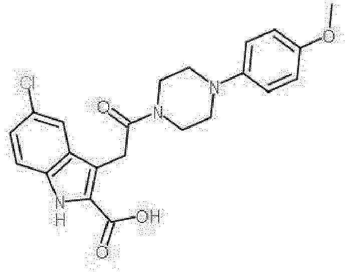

1H NMR (300 MHz, DMSO-d6) δ 11.55 (s, 1H), 7.71-7.70 (m, 1H), 7.49-7.46 (m, 1H), 7.10-7.07 (m, 1H), 6.82-6.74 (m, 4H), 4.33 (s, 2H), 3.84-3.57 (m, 4H), 3.66 (s, 3H), 2.84-2.74 (m, 4H).

ESI (m/z) 428(MH+), 426(MH-).

### 1-10. 5-Chloro-3-(2-(4-(2-chlorophenyl)piperazin-1-yl)-2-oxoethyl)-1H-indole-2-carboxylic acid (YPI-A4803)

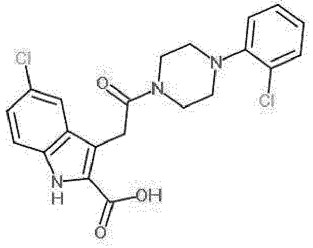

1H NMR (300 MHz, DMSO-d6) δ 11.57 (s, 1H), 7.70 (m, 1H), 7.53-7.51 (m, 1H), 7.38-7.36 (m, 1H), 7.23-7.21 (m, 1H), 7.08-6.96 (m, 3H), 4.35 (s, 2H), 3.88-3.59 (m, 4H), 2.80-2.69 (m, 4H).

ESI (m/z) 430(MH-), 432(MH-), 434(MH-).

### 1-11. 5-Chloro-3-(2-(4-(4-chlorophenyl)piperazin-1-yl)-2-oxoethyl)-1H-indole-2-carboxylic acid (YPI-A4843)

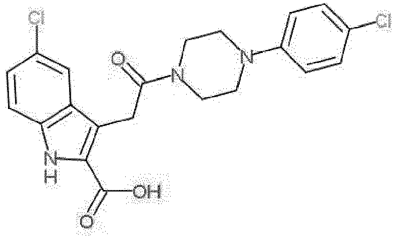

1H NMR (300 MHz, DMSO-d6) δ 13.26 (s, 1H), 11.75 (s, 1H), 7.74 (m, 1H), 7.42-7.40 (m, 1H), 7.25-7.22 (m, 3H), 6.97-6.94 (m, 2H), 4.20 (s, 2H), 3.75-7.60 (m, 4H), 3.12-3.08 (m, 4H).

ESI (m/z) 432(MH+), 434(MH+), 436(MH+).

### 1-12. 3-(2-(4-(2-Bromophenyl)piperazin-1-yl)-2-oxoethyl)-5-chloro-1H-indole-2-carboxylic acid (YPI-A4844)

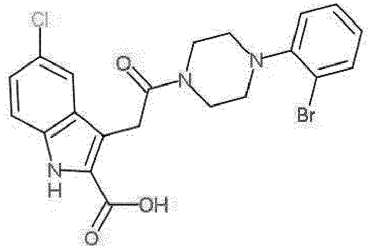

1H NMR (300 MHz, DMSO-d6) δ 11.41 (s, 1H), 7.69 (m, 1H), 7.57-7.54 (m, 1H), 7.49-7.46 (m, 1H), 7.30-7.25 (m, 1H), 7.06-7.03 (m, 1H), 6.98-6.92 (m, 2H), 4.36 (s, 2H), 3.89-3.58 (m, 4H), 2.79-2.66 (m, 4H).

ESI (m/z) 476(MH+), 474(MH-).

### 1-13. 3-(2-(4-(3-Bromophenyl)piperazin-1-yl)-2-oxoethyl)-5-chloro-1H-indole-2-carboxylic acid (YPI-A4845)

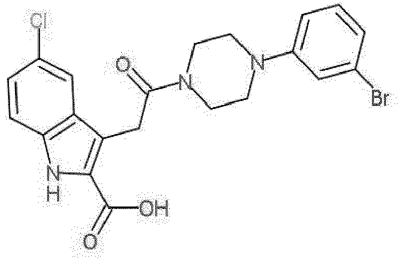

1H NMR (300 MHz, DMSO-d6) δ 13.23 (s, 1H), 11.75 (s, 1H), 7.74 (m, 1H), 7.42-7.39 (m, 1H), 7.25-7.21 (m, 1H), 7.19-7.09 (m, 2H), 6.96-6.92 (m, 2H), 4.20 (s, 2H), 3.75-3.60 (m, 4H), 3.17-3.13 (m, 4H).

ESI (m/z) 474(MH-), 476(MH-), 478(MH-).

### 1-14. 3-(2-(4-(4-Bromophenyl)piperazin-1-yl)-2-oxoethyl)-5-chloro-1H-indole-2-carboxylic acid (YPI-A4846)

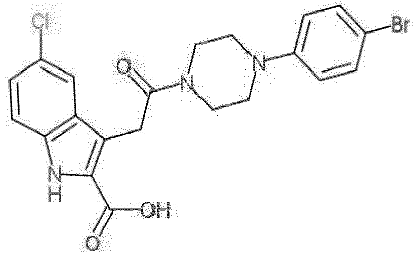

1H NMR (300 MHz, DMSO-d6) δ 11.49 (s. 1H), 7.74 (m, 1H), 7.39-7.37 (m, 1H), 7.32-7.30 (m, 2H), 7.15-7.13 (m, 1H), 6.86-6.84 (m, 2H), 4.26 (s, 2H), 3.80-3.57 (m, 4H), 3.00 (m, 4H).

ESI (m/z) 474(MH-), 476(MH-).

### 1-15. 5-Chloro-3-(2-oxo-2-(4-(o-tolyl)piperazin-1-yl)ethyl)-1H-indole-2-carboxylic acid (YPI-A4847)

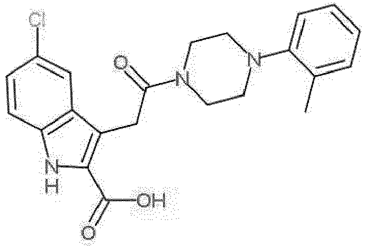

1H NMR (300 MHz, DMSO-d6) δ 11.51 (s, 1H), 7.70-7.69 (m, 1H), 7.52-7.49 (m, 1H), 7.13-7.05 (m, 3H), 6.94-6.89 (m, 1H), 6.83-6.81 (m, 1H), 4.35 (s, 2H), 3.84-3.57 (m, 4H), 2.66-2.54 (m, 4H), 2.19 (s, 3H).

ESI (m/z) 412(MH+), 414(MH+), 410(MH-), 412(MH-).

### 1-16. 5-Chloro-3-(2-oxo-2-(4-(m-tolyl)piperazin-1-yl)ethyl)-1H-indole-2-carboxylic acid (YPI-A4848)

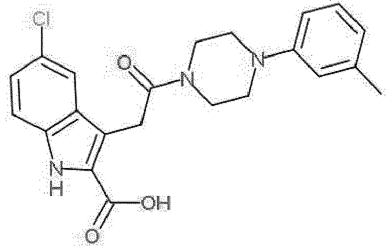

1H NMR (300 MHz, DMSO-d6) δ 13.23 (s, 1H), 11.75 (s, 1H), 7.74 (m, 1H), 7.42-7.39 (m, 1H), 7.25-7.21 (m, 1H), 7.12-7.07 (m, 1H), 6.76-6.61 (m, 3H), 4.20 (s, 2H), 3.75-3.60 (m, 4H), 3.07 (m, 4H), 2.25 (s, 3H).

ESI (m/z) 412(MH+), 414(MH+), 410(MH-), 412(MH-).

### 1-17. 5-Chloro-3-(2-oxo-2-(4-(p-tolyl)piperazin-1-yl)ethyl)-1H-indole-2-carboxylic acid (YPI-A4849)

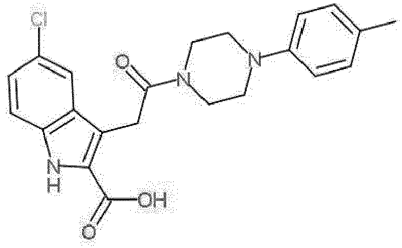

1H NMR (300 MHz, DMSO-d6) δ 11.47 (s, 1H), 7.70-7.69 (m, 1H), 7.46-7.43 (m, 1H), 7.09-7.05 (m, 1H), 6.99-6.96 (m, 2H), 6.75-6.73 (m, 2H), 4.32 (s, 2H), 3.83-3.56 (m, 4H), 2.89-2.79 (m, 4H), 2.17 (s, 3H).

ESI (m/z) 412(MH+), 414(MH+), 410(MH-), 412(MH-).

### 1-18. 3-(2-(4-(tert-butoxycarbonyl)piperazin-1-yl)-2-oxoethyl)-5-chloro-1H-indole-2-carboxylic acid (YPI-A4879)

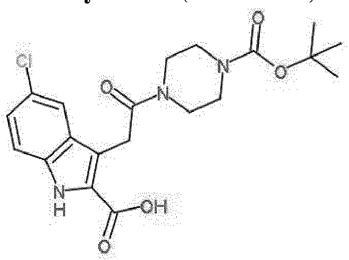

1H NMR (300 MHz, DMSO-d6) δ 13.22 (s, 1H), 11.75 (s, 1H), 7.73-7.72 (m, 1H), 7.42-7.39 (m, 1H), 7.25-7.21 (m, 1H), 4.15 (s, 2H), 3.60-3.44 (m, 4H), 3.32 (m, 4H), 1.41 (s, 9H).

ESI (m/z) 422(MH+), 424(MH+), 420(MH-), 422(MH-).

### 1-19. 5-Chloro-3-(2-(4-(2-chloro-6-methylphenyl)piperazin-1-yl)-2-oxoethyl)-1H-indole-2-carboxylic acid (YPI-A4880)

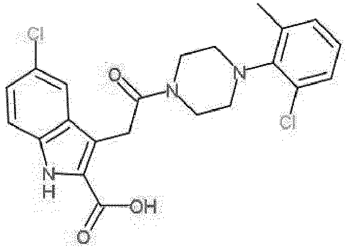

1H NMR (300 MHz, DMSO-d6) δ 13.30 (s, 1H), 11.77 (s, 1H), 7.77-7.76 (m, 1H), 7.45-7.42 (m, 1H), 7.27-7.16 (m, 3H), 7.08-7.03 (m, 1H), 4.25 (q, 2H, J = 30.0 Hz), 4.04-3.89 (m, 2H), 3.51-3.04 (m, 4H), 2.83-2.74 (m, 2H), 2.29 (s, 3H).

ESI (m/z) 444(MH-), 446(MH-), 448(MH-).

### 1-20. 5-Chloro-3-(2-oxo-2-(4-phenylpiperazin-1-yl)ethyl)-1H-indole-2-carboxylic acid (YPI-A4881)

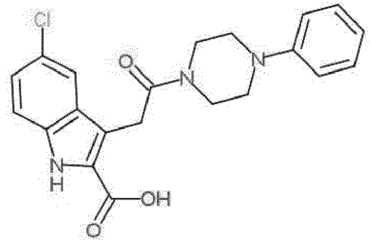

1H NMR (300 MHz, DMSO-d6) δ 11.67 (s, 1H), 7.74-7.73 (m, 1H), 7.41-7.38 (m, 1H), 7.24-7.18 (m, 3H), 6.94-6.91 (m, 2H), 6.82-6.77 (m, 1H), 4.22 (s, 2H), 3.77-3.60 (m, 4H), 3.07 (m, 4H).

ESI (m/z) 396(MH-), 398(MH-).

### 1-21. 5-Chloro-3-(2-(4-(2-ethoxyphenyl)piperazin-1-yl)-2-oxoethyl)-1H-indole-2-carboxylic acid (YPI-A4882)

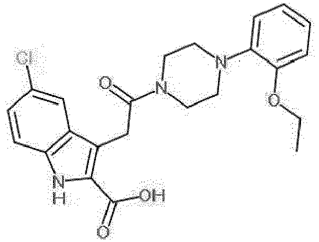

1H NMR (300 MHz, DMSO-d6) δ 13.33 (s, 1H), 11.72 (s, 1H), 7.75-7.74 (m, 1H), 7.42-7.39 (m, 1H), 7.25-7.21 (m, 1H), 6.94-6.82 (m, 4H), 4.20 (s, 2H), 4.03 (q, 2H, J = 9.0 Hz), 3.75-3.60 (m, 4H), 2.92 (m, 4H), 1.35 (t, 3H, J = 7.5 Hz).

ESI (m/z) 442(MH+), 444(MH+), 440(MH-), 442(MH-).

### 1-22. 5-Chloro-3-(2-(3-methyl-4-phenylpiperazin-1-yl)-2-oxoethyl)-1H-indole-2-carboxylic acid (YPI-A4883)

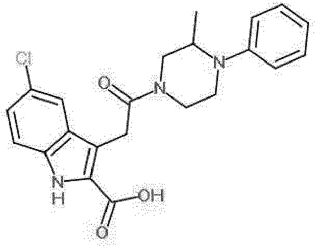

1H NMR (300 MHz, DMSO-d6) δ 11.71 (s, 1H), 7.76-7.73 (m, 1H), 7.43-7.40 (m, 1H), 7.24-7.19 (m, 3H), 6.92-6.86 (m, 2H), 6.80-6.74 (m, 1H), 4.40-3.99 (m, 3H), 4.22 (s, 2H), 3.55-2.87 (m, 4H), 0.88-0.77 (m, 3H).

ESI (m/z) 412(MH+), 414(MH+), 410(MH-), 412(MH-).

### 1-23. 5-Chloro-3-(2-(4-(4-chlorophenyl)-2,2-dimethylpiperazin-1-yl)-2-oxoethyl)-1H-indole-2-carboxylic acid (YPI-A4884)

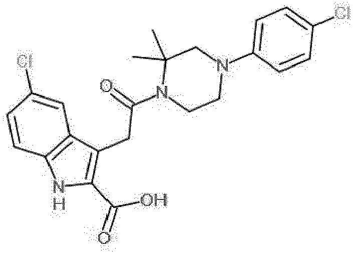

1H NMR (300 MHz, DMSO-d6) δ 13.18 (s, 1H), 11.73 (s, 1H), 7.73 (m, 1H), 7.43-7.40 (m, 1H), 7.24-7.15 (m, 3H), 6.71-6.68 (m, 2H), 4.15 (s, 2H), 3.89-3.87 (m, 2H), 3.33-3.25 (m, 4H), 1.36 (s, 6H).

ESI (m/z) 458(MH-), 460(MH-), 462(MH-).

### 1-24. 5-Chloro-3-(2-(4-(2-chloro-4-fluorophenyl)piperazin-1-yl)-2-oxoethyl)-1H-indole-2-carboxylic acid (YPI-A4885)

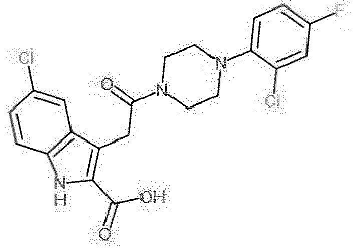

1H NMR (300 MHz, DMSO-d6) δ 13.23 (s, 1H), 11.74 (s, 1H), 7.75-7.74 (m, 1H), 7.44-7.40 (m, 2H), 7.25-7.16 (m, 3H), 4.20 (s, 2H), 3.78-3.62 (m, 4H), 2.89 (m, 4H).

ESI (m/z) 448(MH-), 450(MH-), 452(MH-).

### 1-25. 5-Chloro-3-(2-(4-(2-chloro-5-methoxyphenyl)piperazin-1-yl)-2-oxoethyl)-1H-indole-2-carboxylic acid (YPI-A4886)

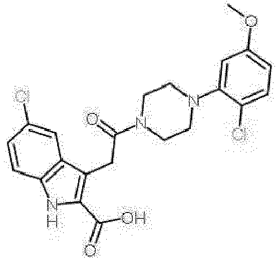

1H NMR (300 MHz, DMSO-d6) δ 13.22 (s, 1H), 11.75 (s, 1H), 7.75 (m, 1H), 7.43-7.40 (m, 1H), 7.33-7.30 (m, 1H), 7.26-7.22 (m, 1H), 6.67-6.64 (m, 2H), 4.20 (s, 2H), 3.77-3.62 (m, 4H), 3.75 (s, 3H), 2.93 (m, 4H).

ESI (m/z) 460(MH-), 462(MH-), 464(MH-).

### 1-26. 5-Chloro-3-(2-(4-(2,4-dichlorophenyl)piperazin-1-yl)-2-oxoethyl)-1H-indole-2-carboxylic acid (YPI-A4887)

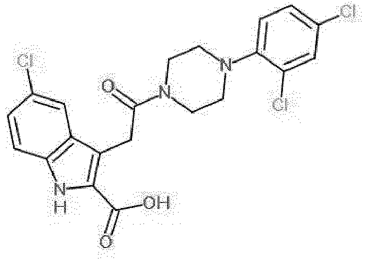

1H NMR (300 MHz, DMSO-d6) δ 11.70 (s, 1H), 7.70-7.69 (m, 1H), 7.58-7.55 (m, 1H), 7.50-7.49 (m, 1H), 7.28-7.25 (m, 1H), 7.08-7.04 (m, 1H), 6.98-6.95 (m, 1H), 4.36 (s, 2H), 3.88-3.58 (m, 4H), 2.79-2.67 (m, 4H).

ESI (m/z) 464(MH-), 466(MH-), 468(MH-).

### 1-27. (R)-5-chloro-3-(2-(2-methyl-4-phenylpiperazin-1-yl)-2-oxoethyl)-1H-indole-2-carboxylic acid (YPI-A4914)

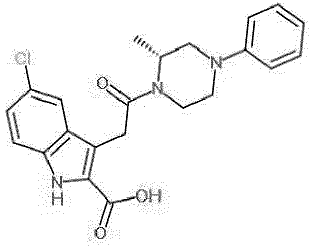

1H NMR (300 MHz, DMSO-d6) δ 13.19 (s, 1H), 11.75 (s, 1H), 7.73 (m, 1H), 7.42-7.40 (m, 1H), 7.22 (m, 3H), 6.92-6.79 (m, 3H), 4.64-4.03 (m, 2H), 4.19 (s, 2H), 3.59-2.78 (m, 5H), 1.30-1.17 (m, 3H).

ESI (m/z) 412(MH+), 414(MH+), 410(MH-), 412(MH-)

### 1-28. 5-Chloro-3-(2-(4-(2,4-difluorophenyl)piperazin-1-yl)-2-oxoethyl)-1H-indole-2-carboxylic acid (YPI-A4915)

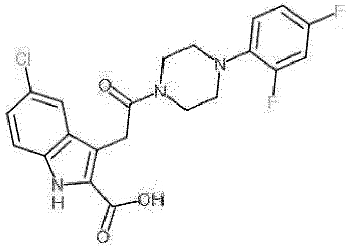

1H NMR (300 MHz, DMSO-d6) δ 13.19 (s, 1H), 11.75 (s, 1H), 7.74 (m, 1H), 7.43-7.40 (m, 1H), 7.25-7.18 (m, 2H), 7.07-6.97 (m, 2H), 4.19 (s, 2H), 3.77-3.62 (m, 4H), 2.94-2.90 (m, 4H).

ESI (m/z) 432(MH-), 434(MH-).

### 1-29. 5-Chloro-3-(2-oxo-2-(4-(3,4,5-trichlorophenyl)piperazin-1-yl)ethyl)-1H-indole-2-carboxylic acid (YPI-A4916)

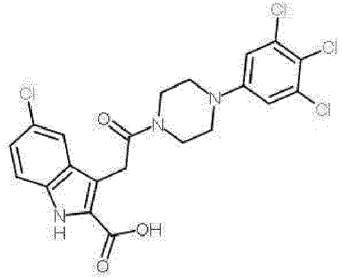

1H NMR (300 MHz, DMSO-d6) δ 13.11 (s, 1H), 11.75 (s, 1H), 7.73 (m, 1H), 7.42-7.40 (m, 1H), 7.24-7.18 (m, 3H), 4.19 (s, 2H), 3.73-3.58 (m, 4H), 3.27-3.21 (m, 4H).

ESI (m/z) 498(MH-), 500(MH-), 502(MH-).

### 1-30. 5-Chloro-3-(2-(4-(2-fluorophenyl)piperazin-1-yl)-2-oxoethyl)-1H-indole-2-carboxylic acid (YPI-A4917)

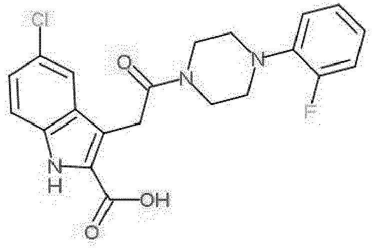

1H NMR (300 MHz, DMSO-d6) δ 13.18 (s, 1H), 11.74 (s, 1H), 7.74 (m, 1H), 7.43-7.40 (m, 1H), 7.25-7.00 (m, 5H), 4.20 (s, 2H), 3.78-3.62 (m, 4H), 2.98-2.95 (m, 4H).

ESI (m/z) 416(MH+), 418(MH+), 414(MH-), 416(MH-).

### 1-31. 5-Chloro-3-(2-(4-(3-chloro-2-methylphenyl)piperazin-1-yl)-2-oxoethyl)-1H-indole-2-carboxylic acid (YPI-A4918)

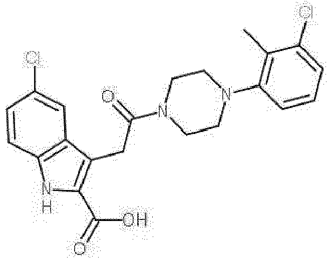

1H NMR (300 MHz, DMSO-d6) δ 11.76 (s, 1H), 7.75 (m, 1H), 7.41 (m, 1H), 7.23-7.16 (m, 3H), 6.98 (m, 1H), 4.20 (s, 2H), 3.77-3.63 (m, 4H), 2.79-2.32 (m, 4H), 1.36-0.80 (m, 3H).

ESI (m/z) 444(MH-), 446(MH-), 448(MH-).

### 1-32. 3-(2-(4-(4-Carboxyphenyl)piperazin-1-yl)-2-oxoethyl)-5-chloro-1H-indole-2-carboxylic acid (YPI-A4919)

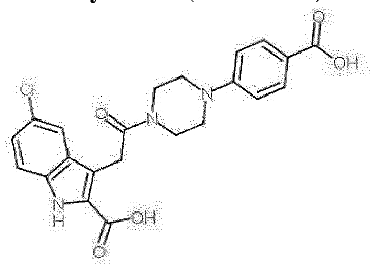

1H NMR (300 MHz, DMSO-d6) δ 12.77 (s, 2H), 11.76 (s, 1H), 7.81-7.75 (m, 3H), 7.43-7.40 (m, 1H), 7.26-7.22 (m, 1H), 6.99-6.96 (m, 2H), 4.21 (s, 2H), 3.79-3.62 (m, 4H), 3.34 (m, 4H).

ESI (m/z) 440(MH-), 442(MH-).

### 1-33. 3-(2-(4-(4-Acetylphenyl)piperazin-1-yl)-2-oxoethyl)-5-chloro-1H-indole-2-carboxylic acid (YPI-A4920)

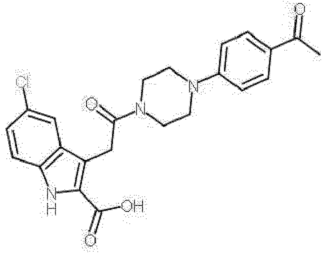

1H NMR (300 MHz, DMSO-d6) δ 13.23 (s, 1H), 11.75 (s, 1H), 7.83-7.81 (m, 2H), 7.74 (m, 1H), 7.42-7.40 (m, 1H), 7.24-7.22 (m, 1H), 6.98-6.96 (m, 2H), 4.20 (s, 2H), 3.78-3.62 (m, 4H), 3.32 (m, 4H), 2.46 (s, 3H).

ESI (m/z) 438(MH-), 440(MH-).

### 1-34. 5-Chloro-3-(2-(4-(4-ethoxyphenyl)piperazin-1-yl)-2-oxoethyl)-1H-indole-2-carboxylic acid (YPI-A4921)

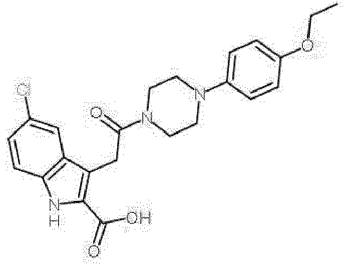

1H NMR (300 MHz, DMSO-d6) δ 11.53 (s, 1H), 7.72 (m, 1H), 7.42-7.40 (m, 1H), 7.15-7.13 (m, 1H), 6.82-6.74 (m, 4H), 4.27 (s, 2H), 3.91 (q, 2H, J = 6.0 Hz), 3.79-3.57 (m, 4H), 2.85-2.83 (m, 4H), 1.27 (t, 3H, J = 6.0 Hz).

ESI (m/z) 442(MH+), 444(MH+), 440(MH-), 442(MH-).

### 1-35. 3-(2-(4-(4-(Tert-butyl)phenyl)piperazin-1-yl)-2-oxoethyl)-5-chloro-1H-indole-2-carboxylic acid (YPI-A4943)

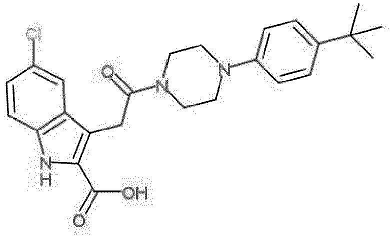

1H NMR (300 MHz, DMSO-d6) δ 13.23 (s, 1H), 11.76 (s, 1H), 7.74 (m, 1H), 7.42-7.40 (m, 1H), 7.24-7.22 (m, 3H), 6.88-6.86 (m, 2H), 4.20 (s, 2H), 3.75-3.60 (m, 4H), 3.05-3.02 (m, 4H), 1.24 (s, 9H).

ESI (m/z) 452(MH-), 454(MH-).

### 1-36. 3-(2-(4-(4-(Tert-butyl)phenyl)piperazin-1-yl)-2-oxoethyl)-5-methyl-1H-indole-2-carboxylic acid (YPI-A4944)

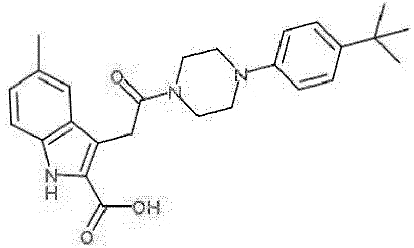

1H NMR (300 MHz, DMSO-d6) δ 12.99 (s, 1H), 11.41 (s, 1H), 7.42 (s, 1H), 7.30-7.28 (m, 1H), 7.24-7.22 (m, 2H), 7.07-7.05 (m, 1H), 6.87-6.85 (m, 2H), 4.17 (s, 2H), 3.75-3.60 (m, 4H), 3.02-3.01 (m, 4H), 2.35 (s, 3H), 1.23 (s, 9H).

ESI (m/z) 434(MH+), 432(MH-).

### 1-37. 3-(2-(4-(4-(Tert-butyl)phenyl)piperazin-1-yl-2-oxoethyl)-5,7-dimethyl-1H-indole-2-carboxylic acid (YPI-A4945)

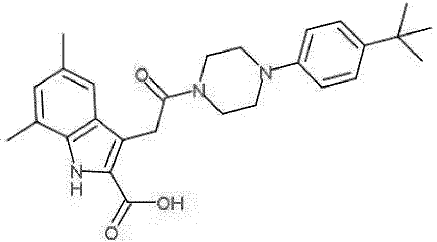

1H NMR (300 MHz, DMSO-d6) δ 11.16 (s, 1H), 724 (m, 2H), 7.21 (s, 1H), 6.87 (s, 1H), 6.84 (m, 2H), 4.17 (s, 2H), 3.75-3.59 (m, 4H), 3.02 (m, 4H), 2.46 (s, 3H), 2.31 (s, 3H), 1.23 (s, 9H).

ESI (m/z) 448(MH+), 446(MH-).

### 1-38. 3-(2-(4-(2,6-Dimethylphenyl)piperazin-1-yl)-2-oxoethyl)-5-methyl-1H-indole-2-carboxylic acid (YPI-A4946)

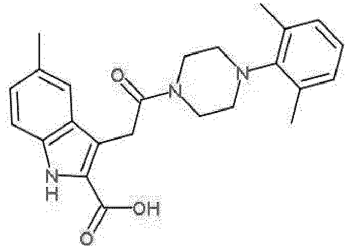

1H NMR (300 MHz, DMSO-d6) δ 13.04 (s, 1H), 11.42 (s, 1H), 7.46 (s, 1H), 7.31-7.29 (m, 1H), 7.09-7.06 (m, 1H), 6.95-6.91 (m, 3H), 4.19 (s. 2H), 3.66-3.57 (m, 4H), 2.89-2.79 (m, 4H), 2.37 (s, 3H), 2.16 (s, 6H).

ESI (m/z) 406(MH+), 404(MH-).

### 1-39. 3-(2-(4-(2,6-dimethylphenyl)piperazin-1-yl)-2-oxoethyl)-5,7-dimethyl-1H-indole-2-carboxylic acid (YPI-A4947)

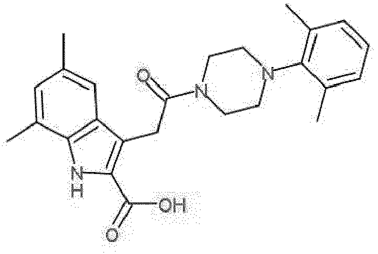

1H NMR (300 MHz, DMSO-d6) δ 13.01 (s, 1H), 11.21 (s, 1H), 7.28 (s, 1H), 6.97-6.91 (m, 3H), 6.87 (s, 1H), 4.18 (s, 2H), 3.66-3.57 (m, 4H), 2.88-2.81 (m, 4H), 2.47 (s, 3H), 2.33 (s, 3H), 2.16 (s, 6H) .

ESI (m/z) 420(MH+), 418(MH-).

### 1-40. 3-(2-(4-(2-Chloro-6-methylphenyl)piperazin-1-yl)-2-oxoethyl)-5-methyl-1H-indole-2-carboxylic acid (YPI-A4948)

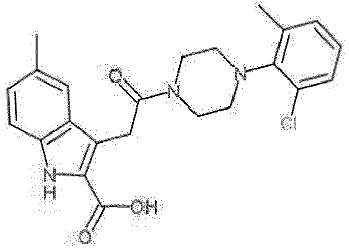

1H NMR (300 MHz, DMSO-d6) δ 11.37 (s, 1H), 7.45 (s, 1H), 7.30-7.28 (m, 1H), 7.19-7.14 (m, 2H), 7.07-7.03 (m, 2H), 4.20 (q, 2H, J = 12 Hz), 4.00-3.90 (m, 2H), 3.18-3.02 (m, 4H), 2.81-2.70 (m, 2H), 2.36 (s, 3H), 2.27 (s, 3H).

ESI (m/z) 426(MH+), 428(MH+), 424(MH-), 426(MH-).

### 1-41. 3-(2-(4-(2-Chloro-6-methylphenyl)piperazin-1-yl)-2-oxoethyl)-5,7-dimethyl-1H-indole-2-carboxylic acid (YPI-A4949)

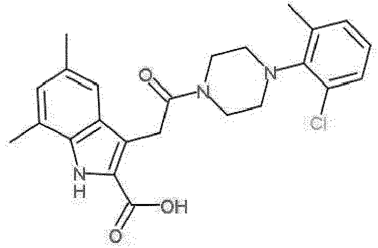

1H NMR (300 MHz, DMSO-d6) δ 12.97 (s, 1H), 11.20 (s, 1H), 7.27 (s, 1H), 7.20-7.15 (m, 2H), 7.07-7.02 (m, 1H), 6.87 (s, 1H), 4.18 (q, 2H, J = 15 Hz), 3.96-3.89 (m, 2H), 3.22-3.06 (m, 4H), 2.81-2.73 (m, 2H), 2.47 (s, 3H), 2.33 (s, 3H), 2.28 (s, 3H).

ESI (m/z) 440(MH+), 442(MH+), 438(MH-), 440(MH-).

### 1-42. 3-(2-(4-(2-Chloro-6-methylphenyl)piperazin-1-yl)-2-oxoethyl)-5-fluoro-1H-indole-2-carboxylic acid (YPI-A4963)

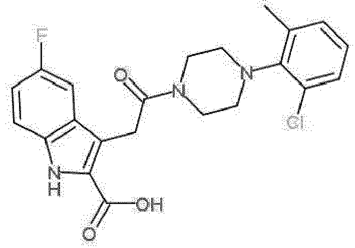

1H NMR (300 MHz, DMSO-d6) δ 13.23 (s, 1H), 11.67 (s, 1H), 7.47-7.39 (m, 2H), 7.20-7.03 (m, 4H), 4.23 (q, 2H, J = 30 Hz), 3.50-3.47 (m, 2H), 3.22-3.02 (m, 4H), 2.82-2.73 (m, 2H), 2.28 (s, 3H).

ESI (m/z) 430(MH+), 432(MH+), 428(MH-), 430(MH-).

### 1-43. 3-(2-(4-(2,6-Dimethylphenyl)piperazin-1-yl)-2-oxoethyl)-5-fluoro-1H-indole-2-carboxylic acid (YPI-A4964)

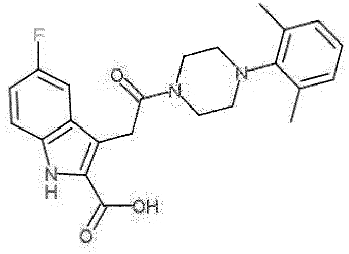

1H NMR (300 MHz, DMSO-d6) δ 13.12 (s, 1H), 11.66 (s, 1H), 7.46-7.40 (m, 2H), 7.14-7.09 (m, 1H), 6.95-6.90 (m, 3H), 4.20 (s, 2H), 3.65-3.57 (m, 4H), 2.88-2.81 (m, 4H), 2.16 (s, 6H).

ESI (m/z) 410(MH+), 408(MH-).

### 1-44. 3-(2-(4-(4-(Tert-butyl)phenyl)piperazin-1-yl)-2-oxoethyl)-5-fluoro-1H-indole-2-carboxylic acid (YPI-A4965)

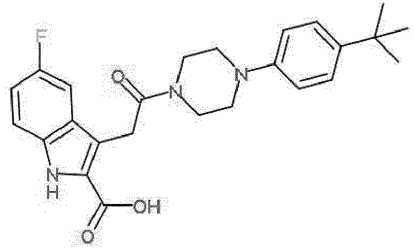

1H NMR (300 MHz, DMSO-d6) δ 13.21 (s, 1H), 11.66 (s, 1H), 7.45-7.38 (m, 2H), 7.25-7.22 (m, 2H), 7.13-7.07 (m, 1H), 6.88-6.85 (m, 2H), 4.18 (s, 2H), 3.75-3.61 (m, 4H), 3.03 (m, 4H), 1.24 (s, 9H).

ESI (m/z) 438(MH+), 436(MH-).

### 1-45. 3-(2-(4-([1,1'-biphenyl]-4-yl)piperazin-1-yl)-2-oxoethyl)-5-chloro-1H-indole-2- carboxylic acid (YPI-A4966)

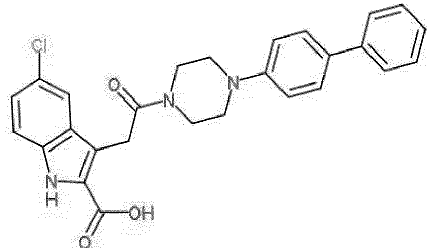

1H NMR (300 MHz, DMSO-d6) δ 11.76 (s, 1H), 7.75 (m, 1H), 7.62-7.53 (m, 4H), 7.43-7.39 (m, 3H), 7.28-7.22 (m, 2H), 7.05-7.02 (m, 2H), 4.22 (s, 2H), 3.78-3.63 (m, 4H), 3.16 (m, 4H).

ESI (m/z) 474(MH+), 476(MH+), 472(MH-), 474(MH-).

### [Example 2]

### Confirmation of inhibitory activity of novel compound against pendrin

### 2-1: Cell Culture

Chinese hamster ovary (CHO)-K1 cells were cultured in DMEM medium containing 10% fetal bovine serum (FBS), 100 units/ml of penicillin and 100 *µ*g/ml streptomycin. CHO-K1 cells were stably transfected with pcDNA3.1 encoding the halide sensor YFP-H148Q/I152L/F46L and human wild-type (WT)-pendrin.

### 2-2. Assay of pendrin Cl⁻/I⁻ exchange inhibitory activity of compounds

CHO-K1 cells expressing human WT pendrin and YFP-F46L/H148Q/I152L were plated in 96-well microplates at a density of 2 × 10⁴ cells per well, and cultured for 48 hours. Each well of the cell cultured 96-well plate was washed 2 times with 200 µL of PBS, and filled with 50 µL of HEPES buffered solution (140mM NaCl, 5mM KCl, 1mM MgCl₂, 1mM CaCl₂, lOmM glucose and lOmM HEPES (pH 7.4)). Test compounds (1 µL) were added to a final concentration of 1, 3, 10, 30 and 100 µM. After culturing at 37°C for 10 minutes, the 96-well plate was located on the FLUOstar Omega Microplate Reader (BMG Labtech, Ortenberg, Germany) for a fluorescence assay. Fluorescence was recorded continuously (400 ms per point) for 1 second (baseline), whereby each well was assayed individually for pendrin-mediated I⁻influx. Then 50 µL of NaI-substituted HEPES buffered solution (replacing NaCl with NaI) was added using a liquid injector at 1 second, and YFP fluorescence was recorded for 5 seconds. The initial iodide influx rate was determined from the initial slope of fluorescence by nonlinear regression, after the injection of iodide.

Pendrin inhibitory activities (% inhibition) by concentration for the 45 compounds of Example 1 are shown in Table 1 below.

**[Table 1]**

| Compound name | Compound concentration | | | | |
|---|---|---|---|---|---|
| | 100 µM | 30 µM | 10 µM | 3 µM | 1 µM |
| YPI-H05 | 96.5 | 95.4 | 67.9 | 24.6 | < 20 |
| YPI-A4779 | 95.3 | 92.7 | 66.7 | < 20 | < 20 |
| YPI-A4781 | 95.6 | 93.8 | 71.4 | 21.5 | < 20 |
| YPI-A4783 | 100 | 100 | 100 | 74.5 | 39.9 |
| YPI-A4799 | 97.1 | 95.5 | 94.2 | 65.3 | 27.9 |
| YPI-A4801 | 97.4 | 96.7 | 95.4 | 72.8 | 40.9 |
| YPI-A4876 | 56.8 | < 20 | < 20 | < 20 | < 20 |
| YPI-A4877 | 67.5 | < 20 | < 20 | < 20 | < 20 |
| YPI-A4878 | 79.4 | < 20 | < 20 | < 20 | < 20 |
| YPI-A4803 | 95.7 | 73.9 | 39.4 | < 20 | < 20 |
| YPI-A4843 | 92.1 | 91.4 | 40.3 | < 20 | < 20 |
| YPI-A4844 | N/A | 92.5 | 59.7 | < 20 | < 20 |
| YPI-A4845 | N/A | 88.8 | 35.1 | < 20 | < 20 |
| YPI-A4846 | N/A | 94.7 | 73.3 | < 20 | < 20 |
| YPI-A4847 | 92.4 | 91.5 | 60.6 | < 20 | < 20 |
| YPI-A4848 | 90.5 | 58.9 | < 20 | < 20 | < 20 |
| YPI-A4849 | 92.3 | 90.9 | 36.8 | < 20 | < 20 |
| YPI-A4879 | < 20 | < 20 | < 20 | < 20 | < 20 |
| YPI-A4880 | 98.4 | 98.1 | 97.6 | 75.2 | 42.8 |
| YPI-A4881 | 40.2 | < 20 | < 20 | < 20 | < 20 |
| YPI-A4882 | 45.3 | < 20 | < 20 | < 20 | < 20 |
| YPI-A4883 | < 20 | < 20 | < 20 | < 20 | < 20 |
| YPI-A4884 | N/A | < 20 | < 20 | < 20 | < 20 |
| YPI-A4885 | 84.2 | 83.7 | 69.1 | < 20 | < 20 |
| YPI-A4886 | 79.9 | 66.2 | 33.7 | < 20 | < 20 |
| YPI-A4887 | 89.9 | 89.5 | 89.3 | 50.8 | < 20 |
| YPI-A4914 | 68.2 | < 20 | < 20 | < 20 | < 20 |
| YPI-A4915 | 93.3 | 65.2 | <20 | < 20 | < 20 |
| YPI-A4916 | 91.4 | 90.9 | 82.6 | 45.7 | < 20 |
| YPI-A4917 | 78.3 | < 20 | < 20 | < 20 | < 20 |
| YPI-A4918 | 95.6 | 94.8 | 87.5 | 34.7 | < 20 |
| YPI-A4919 | < 20 | < 20 | < 20 | < 20 | < 20 |
| YPI-A4920 | < 20 | < 20 | < 20 | < 20 | < 20 |
| YPI-A4921 | 73.8 | 72.5 | <20 | < 20 | < 20 |
| YPI-A4943 | 91.5 | 81.9 | 70.8 | 34.1 | < 20 |
| YPI-A4944 | 77.3 | 76.2 | 45.4 | < 20 | < 20 |
| YPI-A4945 | 38.6 | 38.5 | <20 | < 20 | < 20 |
| YPI-A4946 | 95.6 | 94.5 | 82.3 | 56.9 | 22.4 |
| YPI-A4947 | 41.9 | 41.3 | 40.8 | < 20 | < 20 |
| YPI-A4948 | 100 | 100 | 100 | 93.6 | 70.5 |
| YPI-A4949 | 79.6 | 79.3 | 78.9 | 39.5 | < 20 |
| YPI-A4963 | N/A | 100 | 89.6 | 52.3 | < 20 |
| YPI-A4964 | N/A | 88.9 | 51.4 | < 20 | < 20 |
| YPI-A4965 | N/A | 62.7 | < 20 | < 20 | < 20 |
| YPI-A4966 | < 20 | < 20 | < 20 | < 20 | < 20 |

### [Example 3]

### Confirmation of the function of novel compound as pendrin inhibitor

### 3-1. Cell culture

Chinese hamster ovary (CHO)-K1 cells were cultured in DMEM medium containing 10% fetal bovine serum (FBS), 100 units/ml of penicillin and 100 µg/ml streptomycin. CHO-K1 cells were stably transfected with pcDNA3.1 encoding the halide sensor YFP-H148Q/I152L/F46L and human wild-type (WT)-pendrin.

### 3-2: Assay of Cl⁻/I⁻ exchange activity

CHO-K1 cells expressing human WT pendrin and YFP-F46L/H148Q/I152L were plated in 96-well microplates at a density of 2 × 10⁴ cells per well, and cultured for 48 hours. Each well of the cell cultured 96-well plate was washed 2 times with 200 µL of PBS, and filled with 50 µL of HEPES buffered solution (140mM NaCl, 5mM KCl, 1mM MgCl₂, 1mM CaCl₂, lOmM glucose and lOmM HEPES (pH 7.4)). Test compounds (1 µL) were added to a final concentration of 50 µM. After culturing at 37°C for 10 minutes, the 96-well plate was located on the FLUOstar Omega Microplate Reader (BMG Labtech, Ortenberg, Germany) for a fluorescence assay. Fluorescence was recorded continuously (400 ms per point) for 1 second (baseline), whereby each well was assayed individually for pendrin-mediated I⁻influx. Then, 50 µL of NaI-substituted HEPES buffered solution (replacing NaCl with NaI) was added using a liquid injector at 1 second, and YFP fluorescence was recorded for 5 seconds. The initial iodide influx rate was determined from the initial slope of fluorescence by nonlinear regression, after the injection of iodide.

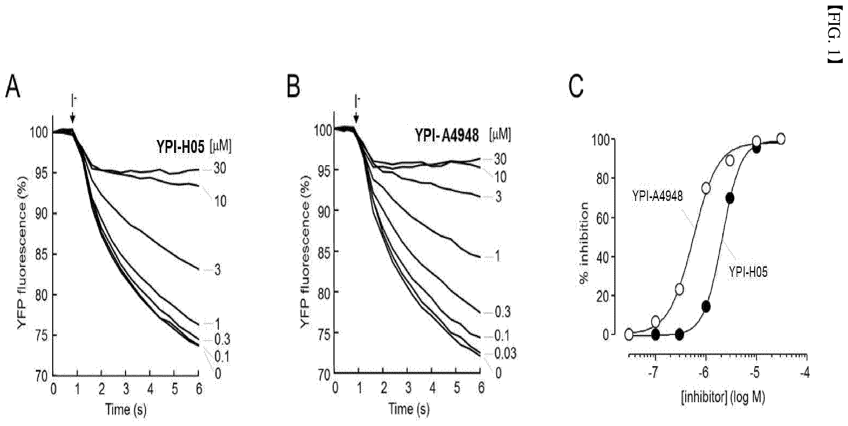

As a result, as seen in Figure 1, both **YPI-H05** and **YPI-A4948,** which are novel pendrin inhibitors, inhibited pendrin-induced Cl⁻/I⁻ anion exchange in a concentration-dependent manner. In particular, **YPI-A4948** (IC₅₀ = 570 nM) showed approximately 5 times higher efficacy than **YPI-05** (IC₅₀ = 2.7 µM).

### [Example 4]

### Confirmation of the effect of novel compounds on SLC26A3 activity

### 4-1. Cell culture

LN-215 cells were cultured in DMEM medium containing 10% fetal bovine serum (FBS), 100 units/ml of penicillin and 100 µg/ml streptomycin. LN-215 cells were stably transfected with pcDNA3.1 encoding the halide sensor YFP-H148Q/I152L/F46L and human wild-type (WT)- SLC26A3.

### 4-2: Assay of SLC26A3 Cl⁻/I⁻ exchange activity

LN-215 cells expressing human WT SLC26A3 and YFP-F46L/H148Q/I152L were plated in 96-well microplates at a density of 2 × 10⁴ cells per well, and cultured for 48 hours. Each well of the cell cultured 96-well plate was washed 2 times with 200 µL of PBS, and filled with 50 µL of HEPES buffered solution (140mM NaCl, 5mM KCl, 1mM MgCl₂, 1mM CaCl₂, lOmM glucose and lOmM HEPES (pH 7.4)). Test compounds (1 µL) were added to a final concentration of 100 µM. After culturing at 37°C for 10 minutes, the 96-well plate was located on the FLUOstar Omega Microplate Reader (BMG Labtech, Ortenberg, Germany) for a fluorescence assay. Fluorescence was recorded continuously (400 ms per point) for 1 second (baseline), whereby each well was assayed individually for SLC26A3-mediated I⁻influx. Then, 50 µL of NaI-substituted HEPES buffered solution (replacing NaCl with NaI) was added using a liquid injector at 1 second, and YFP fluorescence was recorded for 5 seconds. The initial iodide influx rate was determined from the initial slope of fluorescence by nonlinear regression, after the injection of iodide.

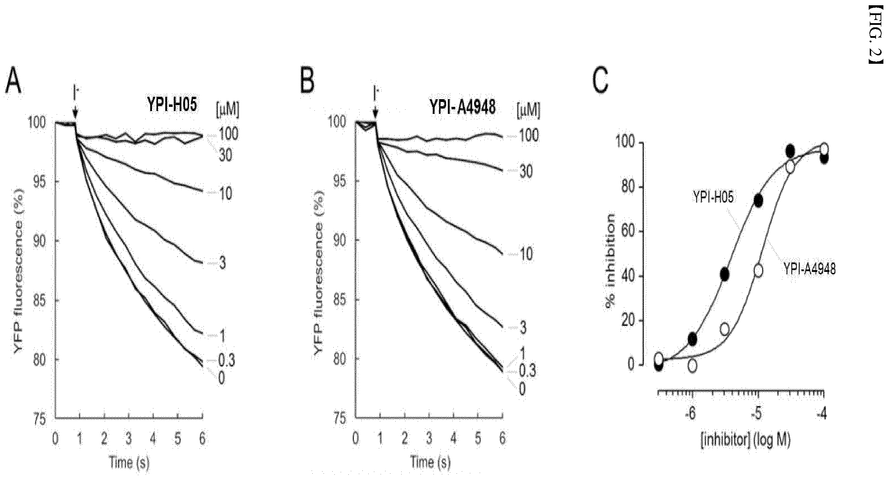

As a result of determining the effect of the novel pendrin inhibitors **YPI-H05** and **YPI-A4948** on Cl⁻/I⁻ anion exchange by SLC26A3, which is an anion exchanger closest to pendrin (SLC26A4) in the SLC26A family, **YPI-H05** (IC₅₀ = 3.8 µM) showed that it inhibited pendrin and SLC26A3 with similar efficacy as confirmed in Figure 2. However, **YPI-A4948** (IC₅₀ = 11.4 µM) had about 18 times higher selectivity for pendrin than SLC26A3. Therefore, **YPI-A4948** is a pendrin inhibitor with excellent selectivity.

### [Example 5]

### Confirmation of the effects of new compounds on SLC26A9, CFTR and ANO1 activities

### 5-1. Cell culture

Chinese hamster ovary (CHO)-K1 cells or LN-215 cells were cultured in DMEM medium containing 10% fetal bovine serum (FBS), 100 units/ml of penicillin and 100 µg/ml streptomycin. CHO-K1 cells or LN-215 cells were stably transfected with pcDNA3.1 encoding the halide sensor YFP-H148Q/I152L/F46L and human wild-type (WT)- SLC26A9. Fisher rat thyroid (FRT) cells stably transfected with YFP-H148Q/I152L/F46L and human wild type (WT)-CFTR or human wild type (WT)-ANO1 were cultured in DMEM/F-12 medium containing 10% fetal bovine serum (FBS), 100 units/ml of penicillin, and 100 µg/ml of streptomycin.

### 5-2. Assay of SLC26A9, CFTR, ANO1 activity

LN-215 cells expressing human WT SLC26A9 and YFP-F46L/H148Q/I152L, and FRT cells expressing human WT CFTR or ANO1 and YFP-F46L/H148Q/I152L were plated in 96-well microplates at a density of 2 × 10⁴ cells per well, and cultured for 48 hours. Each well of the cell cultured 96-well plate was washed 2 times with 200 µL of PBS, and filled with 50 µL of HEPES buffered solution (140mM NaCl, 5mM KCl, 1mM MgCl₂, 1mM CaCl₂, lOmM glucose and lOmM HEPES (pH 7.4)). Test compounds (1 µL) were added to a final concentration of 100 µM. In the case of CFTR activity assay, 10 µM Forskolin was treated together to activate CFTR. After culturing at 37°C for 10 minutes, the 96-well plate was located on the FLUOstar Omega Microplate Reader (BMG Labtech, Ortenberg, Germany) for a fluorescence assay. For assay of the activity of SLC29A9, fluorescence was recorded continuously (400 ms per point) for 1 second (baseline), whereby each well was assayed individually for SLC26A9-mediated I⁻influx. Then, 50 µL of NaI-substituted HEPES buffered solution (replacing NaCl with NaI) was added using a liquid injector at 1 second, and YFP fluorescence was recorded for 6 seconds. For assay of the activity of CFTR, fluorescence was recorded continuously (400 ms per point) for 2 seconds (baseline), whereby each well was assayed individually for CFTR-mediated I⁻ influx. Then, 50 µL of NaI-substituted HEPES buffered solution (replacing NaCl with NaI) was added using a liquid injector at 2 second, and YFP fluorescence was recorded for 8 seconds. For assay of the activity of ANO1, fluorescence was recorded continuously (400 ms per point) for 1 second (baseline), whereby each well was assayed individually for ANO1-mediated I⁻ influx. Then, 50 µL of NaI-substituted HEPES buffered solution (replacing NaCl with NaI) containing ATP (200 µM) was added using a liquid injector at 1 second, and YFP fluorescence was recorded for 6 seconds. The initial iodide influx rate was determined from the initial slope of fluorescence by nonlinear regression, after the injection of iodide.

As a result of determining the effect of the novel pendrin inhibitors **YPI-H05** and **YPI-A4948** on SLC26A9, which is a member of the SLC26A family, and CFTR and ANO1 anion channel, which is a typical anion transporter, both **YPI-H05** and **YPI-A4948** did not inhibit the activity of SLC26A9 at 100 µM, as confirmed in FIG. 3. In the case of CFTR, **YPI-H05** inhibited the activity by about 50% at 100 µM, but **YPI-A4948** had almost no effect on the activity of CFTR at 100 µM. In the case of ANO1 activity, **YPI-H05** inhibited the activity by about 80% at 30 µM, but **YPI-A4948** weakly inhibited ANO1 activity at 30 µM. Whereby, it was confirmed that **YPI-A4948** is a pendrin-specific inhibitor with excellent selectivity.

### [Example 6]

### Confirmation of the effect of new compounds on cell survival rate

### 6-1. Cell culture

Chinese hamster ovary (CHO)-K1 cells were cultured in DMEM medium containing 10% fetal bovine serum (FBS), 100 units/ml of penicillin and 100 µg/ml streptomycin.

### 6-2. Measurement of cell viability

The cell viability was measured using the CellTiter 96^{®} AQueous One Solution cell proliferation assay kit (Promega, Madison, WI, USA). CHO-K1 cells were cultured with medium supplemented with 10% FBS in 96-well plates for 24 hours, and then compounds or vehicle were treated in the medium, and after 24 hours, the medium was completely removed, and the MTS assay was performed as recommended in the supplier's protocol. The absorbance of Formazan was measured at a wavelength of 490 nm using an Infinite M200 microplate reader (Tecan, Austria).

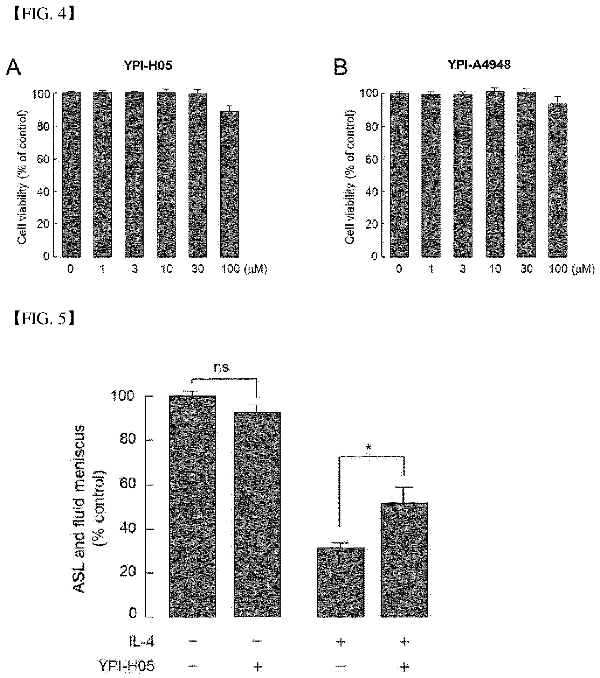

As a result of determining the cytotoxicity of the new pendrin inhibitors **YPI-H05** and **YPI-A4948** in CHO-K1 cell line, both YPI-H05 and YPI-A4948 showed no cytotoxicity at 30 µM as confirmed in Figure 4. Even at 100 µM, **YPI-H05** and **YPI-A4948** showed a change in survival rate of around 10%. Whereby, it was confirmed that that **YPI-H05** and **YPI-A4948** are compounds with very low cytotoxicity.

### [Example 7]

### Confirmation of the recovery function of impaired ASL by novel compound IL-4

### 7-1. Cell culture

For differentiation of primary cultures of human nasal epithelial cells, cells were cultured in Transwell (Transwell, Costar Co., Cambridge, MA) with 0.45 µm pores at a density of 2 × 10⁵ cells/cm². Cells were cultured in a 1:1 mixed culture medium of Dulbecco's modified Eagle's medium (Lonza) and bronchial epithelial growth medium (Lonza) supplemented with the following growth factors, while changing the medium every 2 to 3 days. The cells were cultured in a precipitated state in the culture medium for the first 7 days, and during the subsequent culture period, and cultured by exposing the upper part of transwell to air. After establishment of the air-liquid interface (ALI), cells were cultured and differentiated for 21 days. At all stages of culture, the cell culture medium was maintained at 5% CO₂ and 37°C.

### 7-2. Assay of the volume of airway surface liquid (ASL)

Vehicle, IL-4 (10 ng/ml), and YPI-H05 (30 µM) were added to the basal fluid of transwell in which differentiated NHE cells expressing normal pendrin were growing, and cultured at 37°C under 5% CO₂ in an incubator for 48 hours. After cultured, a round filter paper with a diameter of 10 mm was placed in the lumen side of the transwell and left for 10 seconds to absorb moisture from the airway surface liquid. Changes in the volume of airway surface liquid were measured through increased filter paper weight using a precision electronic balance.

As a result, as can be seen in Figure 5, the novel pendrin inhibitor **YPI-H05** significantly increased the volume of airway surface liquid (ASL), which was greatly reduced by pendrin, whose expression increased by IL-4 treatment in HNE cells which are differentiated respiratory epithelial cells. Whereby, it was confirmed that **YPI-H05** is a pendrin inhibitor that can act on human respiratory epithelial cells and exhibit pharmacological effects.

### [Example 8]

### Confirmation of the effect of the compound in the LPS-induced acute lung injury model

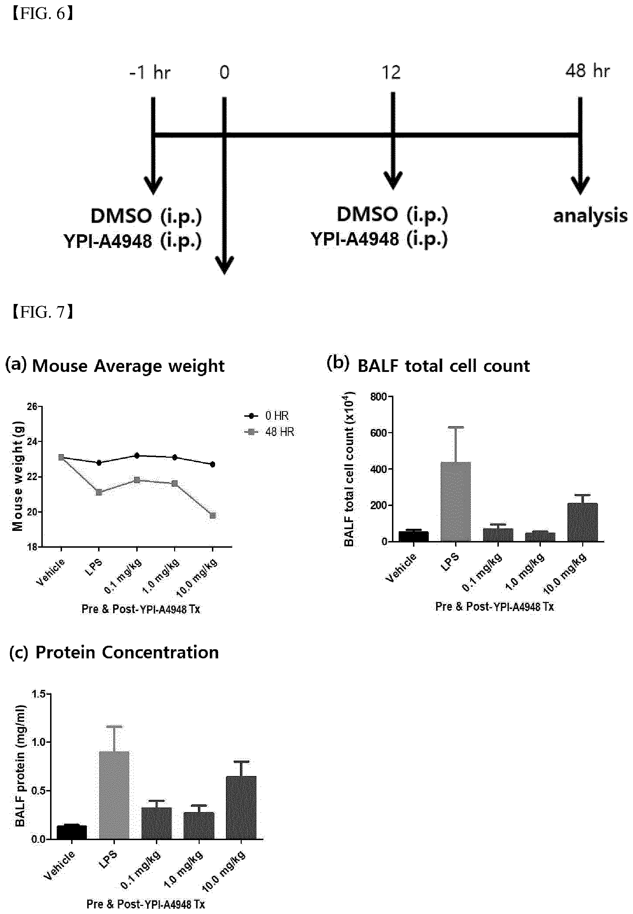

Wild-type male C57BL/6N mice aged 8 to 10 weeks and weighing 20-24 g were purchased from Orient Bio (Seongnam, South Korea). As shown in Table 2 below, mice were divided into 5 groups and samples were treated according to the administration schedule of FIG. 6.

**[Table 2]**

| Group | Number of individuals (head) | Administration |
|---|---|---|
| Vehicle control | 9 | Intraperitoneal injection of DMSO before and after intranasal inhalation administration of PBS |
| LPS-treated group | 11 | Inhalation administration of LPS |
| LPS+YPI-A4948-treated group | 9 | Intraperitoneal injection of YPI-A4948 by concentration before and after inhalation administration of LPS |
| | 10 | |
| | 9 | |

Specifically, LPS (Escherichia coli, O111: B4, Sigma) (10 µg/g) dissolved in 50 µL PBS was administered by intranasal (i.n.) inhalation. The control group received 50 µL of sterile PBS through intranasal administration. For pretreatment, **YPI-A4948** dissolved in 50 µL DMSO was administered intraperitoneally (i.p) at 0.1 mg/kg, 1.0 mg/kg and 10 mg/kg, respectively, 1 hour before LPS inhalation, and for post-treatment, **YPI-A4948** was administered at 0.1 mg/kg, 1.0 mg/kg, and 10 mg/kg, respectively, 12 hours after LPS inhalation. The vehicle control group was intraperitoneally administered with 50 µL DMSO at the same time point as pretreatment and post-treatment. Mice were euthanized 48 hours after LPS inhalation to remove the lungs.

As a result, as confirmed in FIG. 7, the mice in the group treated with **YPI-A4948** before and after LPS inhalation showed significantly reduced levels of BALF total cell counts and protein concentration compared to mice in the group treated with vehicle before and after LPS inhalation.

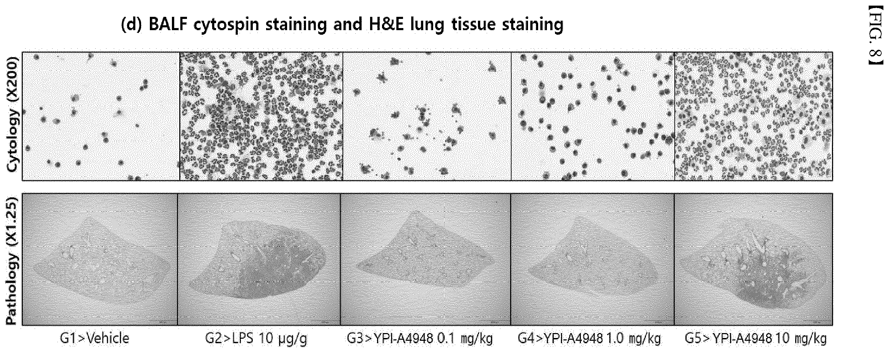

In addition, BALF cytospin staining and H&E lung tissue staining were performed to observe the degree of lung injury. As confirmed in FIG. 8, the mice in the group treated with **YPI-A4948** before and after inhalation of LPS showed significantly reduced lung injury compared to the mice in the group treated with vehicle before and after inhalation of LPS.

### [Example 9]

### Confirmation of diuretic effect of novel compound

Wild-type male C57BL/6N mice aged 8 weeks and weighing 20-24 g were purchased from Orient Bio (Seongnam, South Korea). Before the experiment, water and diet were restricted, and the bladder was stimulated to induce urine. A 100 mg/ml stock concentration of **YPI-A4948** dissolved in DMSO was prepared to have final concentrations of 0.001 mg/kg/100ul, 0.005 mg/kg/100ul and 0.001 mg/kg/100ul, 0.05 mg/kg/100ul, 0.1 mg/kg/100ul, 0.5 mg/kg/100ul and 1 mg/kg/100ul (1% DMSO + DW), respectively, and injected intraperitoneally twice with an interval of 3 hours. After 6 hours, the urine volume of each group of mice was measured.

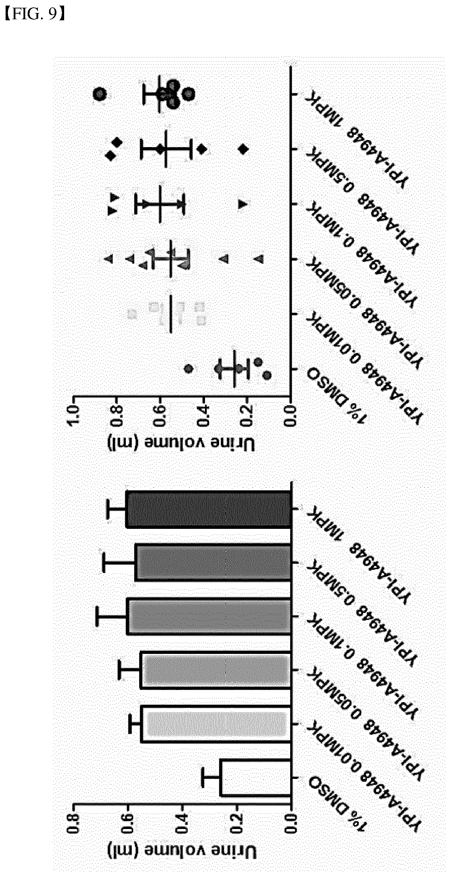

As a result, as shown in Figure 9, when **YPI-A4948** was administered alone at different concentrations, it exhibited a diuretic effect starting from 0.01 mg/kg, confirming the potential of the pendrin inhibitor as a diuretic.

### [Example 10]

### Confirmation of the diuretic effect of novel compound in a heart failure model

### 10-1. Establishment of heart failure mouse model

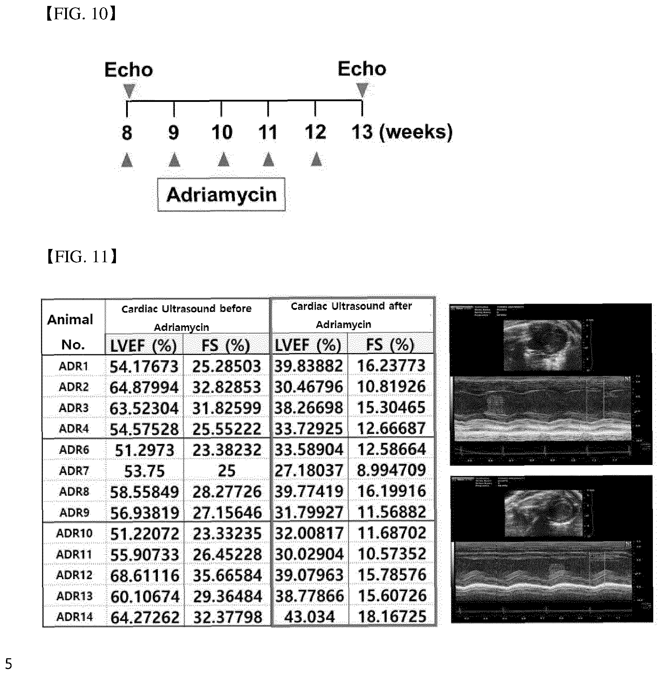

Wild-type male C57BL/6N mice aged 8 weeks and weighing 20~24 g were purchased from Orient Bio (Seongnam, South Korea). Cardiac ultrasound imaging was performed before administration of Adriamycin. Then, as shown in Figure 10, 5 mg/kg of Adriamycin was administered intraperitoneally once a week for a total of 5 weeks, and the body weight before administration was measured. Five weeks later, cardiac ultrasound imaging was performed to confirm whether heart failure had been induced.

As can be seen in Figure 11, left ventricular ejection fraction (LVEF) and fractional shortening (FS) were significantly reduced after administration of Adriamycin, confirming that a heart failure model was established.

### 10-2. Confirmation of diuretic effect following treatment with novel compounds

A 100 mg/ml stock concentration of **YPI-A4948** dissolved in DMSO was prepared to have final concentrations of 0.1 mg/kg/100ul, 0.5 mg/kg/100ul and 1 mg/kg/100ul (1% DMSO + DW), respectively. Heart failure mice were placed in mouse metabolic cages, and water and food were restricted. Before administering **YPI-A4948,** the bladder of the mouse was stimulated, and **YPI-A4948** was injected intraperitoneally alone for two times in total, once every 3 hours for 6 hours. In other groups, it was administered with furosemide at 0.5 mg/kg and 1 mg/kg, respectively, in order to confirm the co-administration effect with other diuretics. Urine was collected from each group of mice after 6 hours.

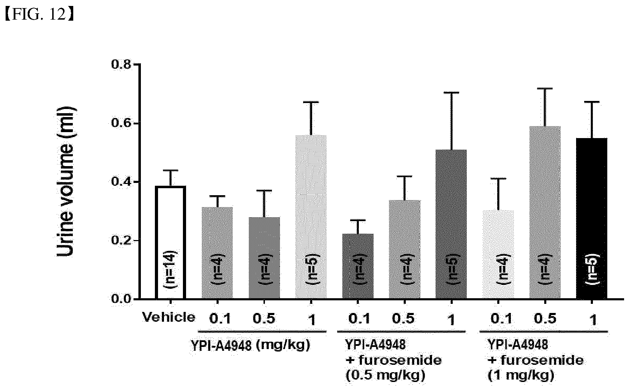

As a result, as confirmed in Figure 12, when YPI-A4948 was administered alone at different concentrations, it showed a diuretic effect at 1 mg/kg, and when 0.5mg/kg of **YPI-A4948** and 1.0 mg/kgmg/kg of furosemide were co-administered, an additive effect was observed. Whereby, the potential of pendrin inhibitors as diuretics in a heart failure mouse model was confirmed.

### [Example 11]

### Effect of YPI-A4948 on pendrin activity in human alveolar epithelial cells (hAEC)

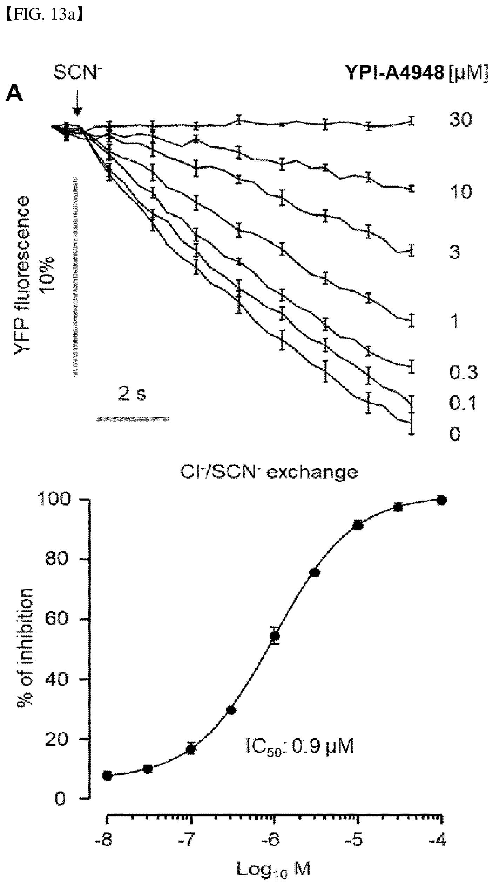

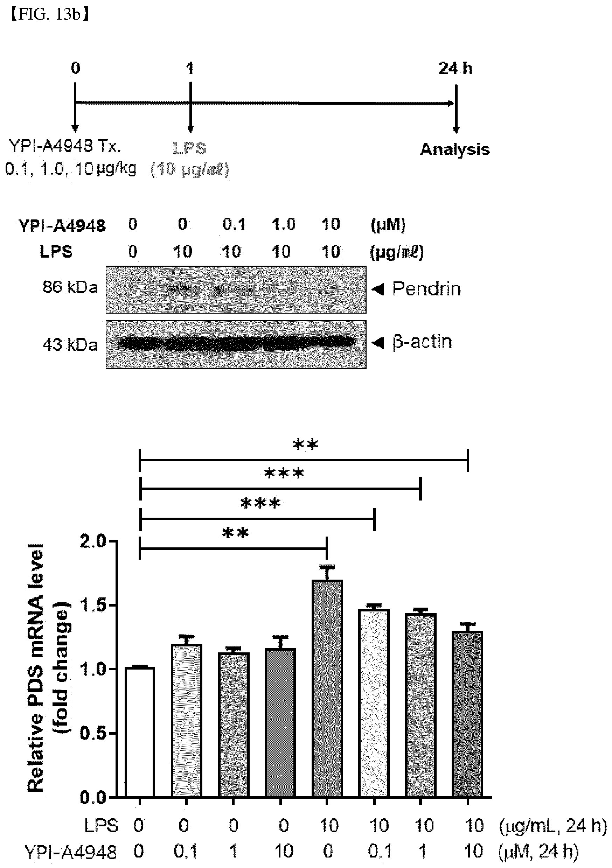

In Figure 13a, human alveolar epithelial cells (hAEC) overexpressing pendrin were treated with **YPI-A4948** as a novel pendrin inhibitor from 0.3 µM to 30 µM, and the Cl⁻/SCN⁻ exchange ability of SLC26A4 was measured by the change in YFP fluorescence when compared to before **YPI-A4948** treatment. The IC₅₀ of human wild-type pendrin-mediated Cl⁻/SCN⁻ exchange activity was confirmed to be 0.9 µM. Moreover, as can be confirmed in Figure 13b, the protein expression level of SLC26A4 (pendrin), which was induced by overexpression by LPS treatment after **YPI-A4948** pretreatment in vitro, was inhibited by YPI-A4948 in a dose-dependent manner. However, the mRNA expression level of pendrin increased by LPS in vitro was not statistically significantly reduced by **YPI-A4948.**

### [Example 12]

### Pendrin inhibitory effect on LPS-induced lung injury model in mice

Lipid polysaccharide (LPS, 200 µg/head) dissolved in 50 µL PBS was injected intranasally into C57BL/6N mice. **YPI-A4948** (1, 10 *µ*g/kg) was administered intravenously twice in total at 6 hours and 12 hours after LPS injection, and the mice were euthanized at 24 hours after LPS inhalation to remove the lungs.

Immune cell recruitment was confirmed through bronchoalveolar lavage fluid (BALF) obtained by injecting 1 mL of PBS into the removed lung and then re-extracting the PBS. Lung injury parameters were measured by ELISA after lysing lung tissue. The expression level of pendrin protein was confirmed by Western blot, and this was digitalized and measured using a shade measuring instrument (mean ± SEM, n = 3 per group). Pendrin mRNA levels were determined by real-time quantitative PCR in extracted lung tissue (mean ± SEM, n = 3 per group). IL-1β, MIP-2, IL-6, and TNF-α levels were measured by ELISA in lung tissue lysates (mean ± SEM, n = 4-7 mice per group).

As a result, as can be confirmed in Figure 14a, mice in the group treated with YPI-A4948 after LPS inhalation showed significantly reduced the levels of pendrin mRNA and protein, BALF total cell counts, and BALF protein concentration compared to mice in the group treated only with LPS. In addition, it showed the same level of efficacy at a dose that was 300 to 3,000 times lower than that of the positive control group, dexamethasone (3mg/kg).

Furthermore, as shown in Figure 14b, it was confirmed that lung inflammatory cytokines IL-1β, IL-6, MIP-2, and TNF-α were statistically significantly reduced after treating mice with **YPI-A4948.**

### [Example 13]

### Role of anions in LPS-induced lung injury model administered with YPI-A4948

Changes in the efficacy of YPI-A4948 by anion supply in an LPS-induced lung injury mouse model were confirmed.

Specifically, lipid polysaccharide (LPS, 200 µg/head) dissolved in 50 µL PBS was injected intranasally, and 6 hours after LPS inhalation, **YPI-A4948** was intravenously administered once at 5 µg/kg, and at the same time, OH⁻, HCO₃⁻, and SCN⁻ anions were administered intranasally, respectively. The mice were euthanized 24 hours after LPS inhalation to remove the lungs. Then, lung tissue H&E staining was performed to observe the degree of lung injury, and bronchoalveolar lavage fluid (BALF) was extracted in the same manner as in Example 12, and cytopathology staining using BALF total cell count, BALF protein concentration level, and Diff-Quik Stain was performed. Data provided are mean ± SEM (n = 3-5 per group), scale bar is 50 µm for H&E staining and cytopathology staining imaged at 400×.

As a result, as seen in Figures 15a and 15b, it was confirmed that of the three types of anions administered, the lung injury improvement effect caused by **YPI-A4948** was weakened only by SCN⁻. This confirmed that during LPS-induced lung injury in the mechanism, SCN⁻ anions induce lung injury via pendrin, and its sub-mechanism also plays an important role. It was confirmed lung injury is reduced through **YPI-A4948** administration, demonstrating the association with SCN⁻ anion.

### [Example 14]

### Confirmation of the effects of new compounds on SLC26A3, SLC26A6, CFTR, and ANO1 activities

In the case of SLC26A3 and SLC26A6, each transporter protein and halide sensitive YFP were expressed in LN-215 cells to measure the activity of the transporter protein.

In the case of CFTR, WT-CFTR and halide sensitive YFP were expressed in CHO-K1 to measure CFTR activity.

In the case of ANO1, ANO1 and halide sensitive YFP were expressed in FRT to measure CFTR activity.

In the case of CFTR, forskolin as an adenylyl cyclase activator was treated at 10 µM for 10 minutes to increase intracellular cyclic AMP and activate CFTR, and then the assay was proceeded.

In the case of ANO1, it was immediately treated with 100 µM of ATP to increase and activate intracellular Ca²⁺, and then the assay was proceeded.

Other details were carried out in the same manner as Example 5.

The results are shown in Figure 16. As a result of determining the effect of a novel pendrin inhibitor YPI-A4948 on SLC26A4 (Pendrin) of the SLC26A family, similar proteins SLC26A3 and SLC26A6 (A6), and typical anion transporters CFTR and ANO1 anion channels, it was confirmed that **YPI-A4948** did not inhibit the activity of SLC26A6 at all, and the IC₅₀ for SLC26A3 was 10.6µM, as can be seen in Figure 16. This can be said to show a large difference compared to the IC50 of Cl⁻/I⁻exchange of SLC26A4 (Pendrin) of 0.55µM. In the case of CFTR, **YPI-A4948** had almost no effect on CFTR activity at 100 µM. In the case of ANO1 activity, **YPI-A4948** weakly inhibited ANO1 activity at 30 µM. Whereby, it was confirmed that **YPI-A4948** is a pendrin-specific inhibitor with excellent selectivity.

## Claims

1. A compound represented by Chemical Formula 1 below, an E- or Z-isomer thereof, an optical isomer thereof, a precursor thereof, a pharmaceutically acceptable salt thereof, a solvate thereof or a mixture of two isomers thereof: 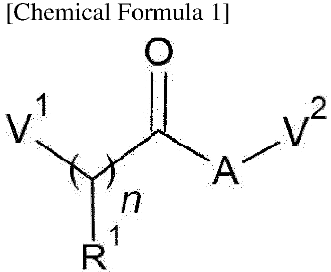
wherein, in Chemical Formula 1,
V¹ is a C₅∼C₁₀ heteroaryl containing 1 to 3 nitrogen atoms, which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂~C₁₀ alkenyl, C₂~C₁₀ alkynyl, C(O)OR², C(O)R², OR², and OCR²F₂,
A is a C₅~C₇ heteroaryl containing 1 to 3 nitrogen atoms or a C₅∼C₇ heterocycloalkyl containing 1 to 3 heteroatoms, which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁~C₁₀ alkyl, C₂~C₁₀ alkenyl, C₂~C₁₀ alkynyl, C(O)OR², C(O)R², OR², OCR²F₂ and OCOR²,
V² is an aryl, a C₁~C₁₀ alkyl, a C₅∼C₇ cycloalkyl, or a C(O)OR², which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂∼C₁₀ alkenyl, C₂∼C₁₀ alkynyl, C(O)OR², C(O)R², OR², OCR²F₂ and OCOR²,
R¹ is one or more independently selected from the group consisting of hydrogen, halogen, C₁∼C₁₀ alkyl, C₂~C₆ alkenyl, and C₂~C₆ alkynyl,
R² is independently selected from the group consisting of hydrogen, trifluoromethyl, trifluoromethoxy, aryl, aryl (C₁~C₁₀ alkyl), C₁~C₁₀ alkylaryl, heteroaryl, heteroaryl (C₁~C₁₀ alkyl), C₁~C₁₀ alkylheteroaryl, C₁∼C₁₀ alkyl, C₂~C₆ alkenyl, C₂~C₆ alkynyl, C₃~C₆ cycloalkyl and heterocycloalkyl, and
n is an integer from 1 to 5.

2. The compound, an E- or Z-isomer thereof, an optical isomer thereof, a precursor thereof, a pharmaceutically acceptable salt thereof, a solvate thereof or a mixture of two isomers thereof as claimed in claim 1, wherein:
V¹ is indolyl, isoindolyl, 3H-indolyl, 1H-indolyl, quinolyl, isoquinolyl, 5,6,7,8-tetrahydroquinolyl, 5,6,7,8-tetrahydroisoquinolyl, quinolizinyl, quinazolinyl, phthalazinyl, cinnolinyl or naphthyridinyl, which is substituted with one or more groups independently selected from the group consisting of hydrogen, halogen, trifluoromethyl, C₃∼C₇ cycloalkyl, C₁∼C₁₀ alkyl, C₂~C₁₀ alkenyl, C₂~C₁₀ alkynyl, C(O)OR², C(O)R², OR², OCR²F₂ and OCOR²,
A is pyrrolidinyl, piperidinyl, piperazinyl or decahydroisoquinolinyl, which is substituted with one or more groups independently selected from the group consisting of hydrogen, halogen, trifluoromethyl, C₃∼C₇ cycloalkyl, C₁∼C₁₀ alkyl, C₂∼C₁₀ alkenyl and C₂~C₁₀ alkynyl,
V² is a C₅∼C₇ aryl, a C₁∼C₁₀ alkyl, a C₅∼C₇ cycloalkyl or a C(O)OR², which is substituted with one or more groups independently selected from the group consisting of hydrogen, halogen, trifluoromethyl, trifluoromethoxy, aryl, C₁∼C₁₀ alkylaryl, C₃∼C₇ cycloalkyl, heteroaryl, heterocycloalkyl, C₁∼C₁₀ alkyl, C₂∼C₁₀ alkenyl, C₂∼C₁₀ alkynyl, C₃~C₆ cycloalkyl, C(O)OR², C(O)R², OR² and OCR²F₂,
R¹ is one or more independently selected from the group consisting of hydrogen, halogen, C₁∼C₁₀ alkyl, C₂~C₆ alkenyl, and C₂~C₆ alkynyl,
R² is independently selected from the group consisting of hydrogen, trifluoromethyl, aryl, aryl (C₁~C₁₀ alkyl), C₁~C₁₀ alkylaryl, C₁~C₁₀ alkyl, C₂~C₆ alkenyl, C₂~C₆ alkynyl, and C₃~C₆ cycloalkyl, and
n is an integer from 1 to 5.

3. The compound, an E- or Z-isomer thereof, an optical isomer thereof, a precursor thereof, a pharmaceutically acceptable salt thereof, a solvate thereof or a mixture of two isomers thereof as claimed in claim 1, wherein:
the compound is represented by Chemical Formula 1a below: 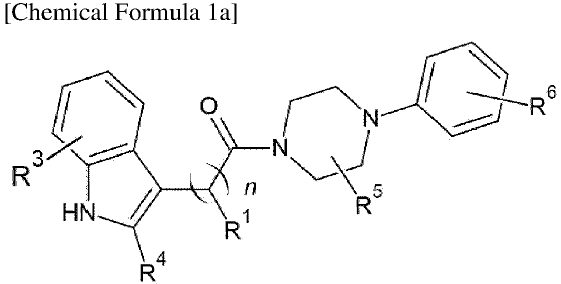
wherein, in Chemical Formula 1a,
R¹ is one or more independently selected from the group consisting of hydrogen, halogen, C₁∼C₁₀ alkyl, C₂~C₆ alkenyl, and C₂~C₆ alkynyl,
R³ is one or more groups independently selected from the group consisting of hydrogen, halogen, trifluoromethyl, C₃∼C₇ cycloalkyl, C₁∼C₁₀ alkyl, C₂∼C₁₀ alkenyl, C₂~C₁₀ alkynyl, C(O)OR², C(O)R², OR² and OCR²F₂,
R⁴ is a group independently selected from the group consisting of C(O)OR², C(O)R², OR², OCR²F₂ and OCOR²,
R⁵ is one or more groups independently selected from the group consisting of hydrogen, C₃∼C₇ cycloalkyl, C₁∼C₁₀ alkyl, C₂~C₁₀ alkenyl and C₂~C₁₀ alkynyl,
R⁶ is one or more groups independently selected from the group consisting of hydrogen, halogen, trifluoromethyl, C₃∼C₇ cycloalkyl, C₁∼C₁₀ alkyl, C₂~C₁₀ alkenyl, C₂~C₁₀ alkynyl, C(O)OR², C(O)R², OR² and OCR²F₂,
R² is independently selected from the group consisting of hydrogen, trifluoromethyl, aryl, aryl (C₁~C₁₀ alkyl), C₁~C₁₀ alkylaryl, C₁~C₁₀ alkyl, C₂~C₆ alkenyl, C₂~C₆ alkynyl, and C₃~C₆ cycloalkyl, and
n is an integer from 1 to 5.

4. The compound, an E- or Z-isomer thereof, an optical isomer thereof, a precursor thereof, a pharmaceutically acceptable salt thereof, a solvate thereof or a mixture of two isomers thereof as claimed in claim 1, wherein:
the compound is represented by Chemical Formula 1b below: 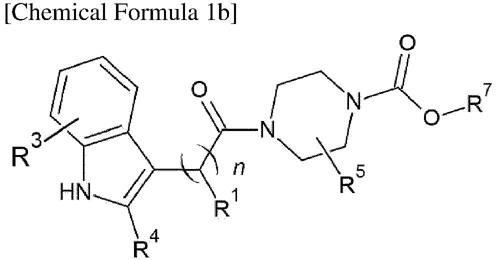
wherein, in Chemical Formula 1b,
R¹ is one or more independently selected from the group consisting of hydrogen, halogen, C₁∼C₁₀ alkyl, C₂~C₆ alkenyl, and C₂~C₆ alkynyl,
R³ is one or more groups independently selected from the group consisting of hydrogen, halogen, trifluoromethyl, C₃∼C₇ cycloalkyl, C₁∼C₁₀ alkyl, C₂~C₁₀ alkenyl, C₂~C₁₀ alkynyl, C(O)OR², C(O)R², OR² and OCR²F₂,
R⁴ is a group independently selected from the group consisting of C(O)OR², C(O)R², OR², OCR²F₂ and OCOR²,
R⁵ is one or more groups independently selected from the group consisting of hydrogen, C₃∼C₇ cycloalkyl, C₁∼C₁₀ alkyl, C₂~C₁₀ alkenyl and C₂~C₁₀ alkynyl,
R⁷ is one or more groups independently selected from the group consisting of hydrogen, halogen, trifluoromethyl, C₃∼C₇ cycloalkyl, C₁∼C₁₀ alkyl, C₂~C₁₀ alkenyl, and C₂~C₁₀ alkynyl,
R² is independently selected from the group consisting of hydrogen, trifluoromethyl, aryl, aryl (C₁~C₁₀ alkyl), C₁~C₁₀ alkylaryl, C₁~C₁₀ alkyl, C₂~C₆ alkenyl, C₂~C₆ alkynyl, and C₃~C₆ cycloalkyl, and
n is an integer from 1 to 5.

5. The compound, an E- or Z-isomer thereof, an optical isomer thereof, a precursor thereof, a pharmaceutically acceptable salt thereof, a solvate thereof or a mixture of two isomers thereof as claimed in claim 1, wherein the compound is:
(1) 5-chloro-3-(2-oxo-2-(4-(4-(trifluoromethyl)phenyl)piperazin-1-yl)ethyl)-1H-indole-2-carboxylic acid;
(2) 5-chloro-3-(2-oxo-2-(4-(2-(trifluoromethyl)phenyl)piperazin-1-yl)ethyl)-1H-indole-2-carboxylic acid;
(3) 5-chloro-3-(2-(4-(2,6-dimethylphenyl)piperazin-1-yl)-2-oxoethyl)-1H-indole-2-carboxylic acid;
(4) 5-chloro-3-(2-(4-(2,4-dimethylphenyl)piperazin-1-yl)-2-oxoethyl)-1H-indole-2-carboxylic acid;
(5) 5-chloro-3-(2-(4-(3-methoxyphenyl)piperazin-1-yl)-2-oxoethyl)-1H-indole-2-carboxylic acid;
(6) 5-chloro-3-(2-(4-(2-chlorophenyl)piperazin-1-yl)-2-oxoethyl)-1H-indole-2-carboxylic acid;
(7) 5-chloro-3-(2-(4-(4-chlorophenyl)piperazin-1-yl)-2-oxoethyl)-1H-indole-2-carboxylic acid;
(8) 3-(2-(4-(2-bromophenyl)piperazin-1-yl)-2-oxoethyl)-5-chloro-1H-indole-2-carboxylic acid;
(9) 3-(2-(4-(3-bromophenyl)piperazin-1-yl)-2-oxoethyl)-5-chloro-1H-indole-2-carboxylic acid;
(10) 3-(2-(4-(4-bromophenyl)piperazin-1-yl)-2-oxoethyl)-5-chloro-1H-indole-2-carboxylic acid;
(11) 5-chloro-3-(2-oxo-2-(4-(o-tolyl)piperazin-1-yl)ethyl)-1H-indole-2-carboxylic acid;
(12) 5-chloro-3-(2-oxo-2-(4-(m-tolyl)piperazin-1-yl)ethyl)-1H-indole-2-carboxylic acid;
(13) 5-chloro-3-(2-oxo-2-(4-(*p*-tolyl)piperazin-1-yl)ethyl)-1H-indole-2-carboxylic acid;
(14) 3-(2-(4-(*tert*-butoxycarbonyl)piperazin-1-yl)-2-oxoethyl)-5-chloro-1H-indole-2-carboxylic acid;
(15) 5-chloro-3-(2-(4-(2-chloro-6-methylphenyl)piperazin-1-yl)-2-oxoethyl)-1H-indole-2-carboxylic acid;
(16) 5-chloro-3-(2-(4-(2-ethoxyphenyl)piperazin-1-yl)-2-oxoethyl)-1H-indole-2-carboxylic acid;
(17) 5-chloro-3-(2-(3-methyl-4-phenylpiperazin-1-yl)-2-oxoethyl)-1H-indole-2-carboxylic acid;
(18) 5-chloro-3-(2-(4-(4-chlorophenyl)-2,2-dimethylpiperazin-1-yl)-2-oxoethyl)-1H-indole-2-carboxylic acid;
(19) 5-chloro-3-(2-(4-(2-chloro-4-fluorophenyl)piperazin-1-yl)-2-oxoethyl)-1H-indole-2-carboxylic acid;
(20) 5-chloro-3-(2-(4-(2-chloro-5-methoxyphenyl)piperazin-1-yl)-2-oxoethyl)-1H-indole-2-carboxylic acid;
(21) 5-chloro-3-(2-(4-(2,4-dichlorophenyl)piperazin-1-yl)-2-oxoethyl)-1H-indole-2-carboxylic acid;
(22) (R)-5-chloro-3-(2-(2-methyl-4-phenylpiperazin-1-yl)-2-oxoethyl)-1H-indole-2-carboxylic acid;
(23) 5-chloro-3-(2-(4-(2,4-difluorophenyl)piperazin-1-yl)-2-oxoethyl)-1H-indole-2-carboxylic acid;
(24) 5-chloro-3-(2-oxo-2-(4-(3,4,5-trichlorophenyl)piperazin-1-yl)ethyl)-1H-indole-2-carboxylic acid;
(25) 5-chloro-3-(2-(4-(2-fluorophenyl)piperazin-1-yl)-2-oxoethyl)-1H-indole-2-carboxylic acid;
(26) 5-chloro-3-(2-(4-(3-chloro-2-methylphenyl)piperazin-1-yl)-2-oxoethyl)-1H-indole-2-carboxylic acid;
(27) 3-(2-(4-(4-carboxyphenyl)piperazin-1-yl)-2-oxoethyl)-5-chloro-1H-indole-2-carboxylic acid;
(28) 3-(2-(4-(4-acetylphenyl)piperazin-1-yl)-2-oxoethyl)-5-chloro-1H-indole-2-carboxylic acid;
(29) 5-chloro-3-(2-(4-(4-ethoxyphenyl)piperazin-1-yl)-2-oxoethyl)-1H-indole-2-carboxylic acid;
(30) 3-(2-(4-(4-(*tert*-butyl)phenyl)piperazin-1-yl)-2-oxoethyl)-5-chloro-1H-indole-2-carboxylic acid;
(31) 3-(2-(4-(4-(*tert*-butyl)phenyl)piperazin-1-yl)-2-oxoethyl)-5-methyl-1H-indole-2-carboxylic acid;
(32) 3-(2-(4-(4-(*tert*-butyl)phenyl)piperazin-1-yl-2-oxoethyl)-5,7-dimethyl-1H-indole-2-carboxylic acid;
(33) 3-(2-(4-(2,6-dimethylphenyl)piperazin-1-yl)-2-oxoethyl)-5-methyl-1H-indole-2-carboxylic acid;
(34) 3-(2-(4-(2,6-dimethylphenyl)piperazin-1-yl)-2-oxoethyl)-5,7-dimethyl-1H-indole-2-carboxylic acid;
(35) 3-(2-(4-(2-chloro-6-methylphenyl)piperazin-1-yl)-2-oxoethyl)-5-methyl-1H-indole-2-carboxylic acid;
(36) 3-(2-(4-(2-chloro-6-methylphenyl)piperazin-1-yl)-2-oxoethyl)-5,7-dimethyl-1H-indole-2-carboxylic acid;
(37) 3-(2-(4-(2-chloro-6-methylphenyl)piperazin-1-yl)-2-oxoethyl)-5-fluoro-1H-indole-2-carboxylic acid;
(38) 3-(2-(4-(2,6-dimethylphenyl)piperazin-1-yl)-2-oxoethyl)-5-fluoro-1H-indole-2-carboxylic acid;
(39) 3-(2-(4-(4-(tert-butyl)phenyl)piperazin-1-yl)-2-oxoethyl)-5-fluoro-1H-indole-2-carboxylic acid;
(40) 3-(2-(4-([1,1'-biphenyl]-4-yl)piperazin-1-yl)-2-oxoethyl)-5-chloro-1H-indole-2- carboxylic acid; or
(41) 5-chloro-3-(2-(4-(2,5-dimethylphenyl)piperazin-1-yl-)-2-oxoethyl)-1H-indole-2-carboxylic acid.

6. A pharmaceutical composition for preventing or treating respiratory diseases, comprising, as an active ingredient, the compound as claimed in any one of claims 1 to 5, an E- or Z-isomer thereof, an optical isomer thereof, a precursor thereof, a pharmaceutically acceptable salt thereof, a solvate thereof or a mixture of two isomers thereof.

7. The pharmaceutical composition as claimed in claim 6, wherein the respiratory diseases are inflammatory airway diseases.

8. The pharmaceutical composition as claimed in claim 7, wherein the inflammatory airway diseases is one or more selected from the group consisting of asthma, acute or chronic bronchitis, allergic rhinitis, acute respiratory tract infection, acute upper respiratory tract infection, cystic fibrosis, acute respiratory distress syndrome (ARDS), acute lung injury (ALI) and chronic obstructive pulmonary disease (COPD).

9. The pharmaceutical composition as claimed in claim 6, wherein the active ingredient acts as a pendrin inhibitor.

10. The pharmaceutical composition as claimed in claim 6, wherein the active ingredient preserves or increases the volume of airway surface liquid (ASL).

11. The pharmaceutical composition as claimed in claim 6, further comprising other pharmaceutical ingredients.

12. A health functional food for preventing or ameliorating respiratory diseases, comprising, as an active ingredient, the compound as claimed in any one of claims 1 to 5, an E- or Z-isomer thereof, an optical isomer thereof, a precursor thereof, a pharmaceutically acceptable salt thereof, a solvate thereof or a mixture of two isomers thereof.

13. A diuretic pharmaceutical composition, comprising, as an active ingredient, the compound as claimed in any one of claims 1 to 5, an E- or Z-isomer thereof, an optical isomer thereof, a precursor thereof, a pharmaceutically acceptable salt thereof, a solvate thereof or a mixture of two isomers thereof.

14. The pharmaceutical composition as claimed in claim 13, which is co-administered with other diuretics.

15. The pharmaceutical composition as claimed in claim 14, wherein
the other diuretic is furosemide, and the pharmaceutical composition and furosemide are administered in a weight ratio of 1:10.

16. A diuretic health functional food composition, comprising, as an active ingredient, the compound as claimed in any one of claims 1 to 5, an E- or Z-isomer thereof, an optical isomer thereof, a precursor thereof, a pharmaceutically acceptable salt thereof, a solvate thereof or a mixture of two isomers thereof.

17. A method for preventing or ameliorating respiratory diseases, comprising administering the compound as claimed in any one of claims 1 to 5, an E- or Z-isomer thereof, an optical isomer thereof, a precursor thereof, a pharmaceutically acceptable salt thereof, a solvate thereof or a mixture of two isomers thereof.

18. A diuretic method, comprising administering the compound as claimed in any one of claims 1 to 5, an E- or Z-isomer thereof, an optical isomer thereof, a precursor thereof, a pharmaceutically acceptable salt thereof, a solvate thereof or a mixture of two isomers thereof.
